(19) Europäisches Patentamt
European Patent Office
Office européen des brevets

(11) EP 4 361 238 A1

(12) EUROPÄISCHE PATENTANMELDUNG

(43) Veröffentlichungstag:
**01.05.2024 Patentblatt 2024/18**

(21) Anmeldenummer: **23020436.4**

(22) Anmeldetag: **20.09.2023**

(51) Internationale Patentklassifikation (IPC):
***C11D 1/66*** *(2006.01)* ***A61K 8/60*** *(2006.01)*

(52) Gemeinsame Patentklassifikation (CPC):
**C11D 1/662; A61K 8/44; A61Q 19/10**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(30) Priorität: **26.10.2022 CH 12642022**

(71) Anmelder: **ChemTEK UG (haftungsbeschränkt)**
**79618 Rheinfelden (DE)**

(72) Erfinder:
- **Der Erfinder hat auf sein Recht verzichtet, als solcher bekannt gemacht zu werden.**

(54) **ZUSAMMENSETZUNGEN MIT GLYCAMINEN**

(57) Die Erfindung betrifft Zusammensetzungen mit einem oder mehreren N-Acylglycaminen und/oder Biotensiden, insbesondere als kosmetische Reinigungs- oder Pflegemittel oder Reinigungs-oder Pflegemittel für Haushalt oder den Gewerbesektor umfassend diese Zusammensetzung. Als Reinigungs- oder Pflegemittel sind Mittel für harte Oberflächen, flexible Oberflächen, textile Oberflächen und natürliche Oberflächen wie z.B. Zähne, Haut und Haare zu verstehen.

EP 4 361 238 A1

## Beschreibung

Die Erfindung betrifft Zusammensetzungen mit einem oder mehreren N-Acylglycaminen und/oder Biotensiden, insbesondere als kosmetische Reinigungs- oder Pflegemittel oder Reinigungs-oder Pflegemittel für Haushalt oder den Gewerbesektor umfassend diese Zusammensetzung. Als Reinigungs- oder Pflegemittel sind Mittel für harte Oberflächen, flexible Oberflächen, textile Oberflächen und natürliche Oberflächen wie z.B. Zähne, Haut und Haare zu verstehen.

## Background

Reinigungsmittel sind Wasch- oder Reinigungsmittel zur Entfernung unerwünschter Verschmutzungen oder Belägen wie beispielweise Flecken, Rückstände, Verunreinigungen, Stoffwechselprodukte von Substraten ausgewählt aus natürlichen oder biologischen Oberflächen, z.B. Haut, Zähne und Haaren u.a., flexiblen Oberflächen, harten Oberflächen umfassend z.B. Geschirr, Boden, Glas, Plastik, Fenster, Spiegel, Keramik, Kunststoff, Metall oder Holz - lackiert sowie unlackiert ,u.a., sowie textile Oberflächen, umfassend z.B. Kleidung, Tücher, Teppiche, Leder, Wolle, synthetische Fasern, Mischfasern oder Naturfasern. Ein Pflegemittel ist ein Mittel, das die Attraktivität einer Person erhöhen, die Gesundheit von Zähnen, Haut, Schleimhaut und Haar erhalten kann, inklusive der Rasur, verschönern und pflegen kann. Das Pflegemittel umfasst auch die Behandlung von Materialien zur Verbesserung deren Eigenschaften oder Verlängerung der Lebensdauer, z.B. Weichspüler für Textilien. Eingeschlossen sind Kombinationen aus Reinigungs- und Pflegemittel, sowie die Tierpflege und -reinigung.

Wichtige Zusammensetzung sind die Reinigungs- oder Pflegemittel. Eine gute Benetzung der zu reinigenden oder pflegenden Oberfläche oder die Fähigkeit Fett zu emulgieren spielen hierbei eine wesentliche Rolle. Um eine ausreichende Benetzung zu erreichen, müssen oft hohe Konzentrationen oder bedenkliche Inhaltstoffe eingesetzt werden. Die Fähigkeiten zu benetzen und zu emulgieren, können in der Regel aus technischen Gründen nicht von ein und derselben Tensidzusammensetzung befriedigend erfüllt werden.

Zudem ist wichtig, dass die Substrate nach der Behandlung rückstandsarm sind. Besonders in Anwesenheit von Elektrolyten, z.B. mit hartem Wasser oder Gebrauchswasser ist dies eine Herausforderung, da viele Tenside selber Rückstände bilden mit Bestandteilen, mit denen sie während der Verwendung in Berührung kommen.

Rückstände können aus dem Mittel, z.B. Pflegerückständen (z.B. Haarfestiger) oder aus dem Schmutz stammen. Ein besonderes Problem sind Rückstände, die sich im Laufe der Behandlung bilden. Solche intrinsischen Rückstände sind z.B. Konglomerate aus Tensiden mit Schmutz oder Salzen aus dem Wasser, wie beispielsweise die Kalkseife, und bilden schlecht lösliche oder haftende Verbindungen.

Rückstände sind optisch störend, insbesondere bei Geschirrspülmitteln, Fensterreinigern, oder Textilbehandlungsmittel und können - selbst wenn anfangs nicht optisch wahrnehmbar- als Schmutzmagnet wirken und zu Grauschleiern oder Wiederanschmutzung führen. Als zusätzlicher Reinigungsschritt wird daher üblicherweise mit Wasser gespült um Rückstände aus der Anwendung vollständig zu entfernen. Rückstände beeinträchtigen ausserdem die Sensorik und den Weichgriff, z.B. in Anwendungen der Kosmetik und der Textilwäsche. Geschmackliche oder oleofaktorische Einflüsse, oder die Aufnahme von Rückständen und Schmutz oral oder durch die Haut, sollen vermieden werden, z.B. in der Verwendung im Bereich Geschirr, Zahnpflege oder Haarpflege. Eine Vermeidung intrinsischer Rückstände ist daher wesentlich.

Manche Produkte werden vom Anwender aufgrund deren Schäumungsverhalten beurteilt. Ein gutes Schaumbildungvermögen und Schaumstabilität wird beispielsweise in manuellen Geschirrspülmitteln oder Shampoo vorausgesetzt.

Elektrolyte setzen üblicherweise die Schaumleistung von Tensiden herab, so auch die Schaumleistung von Glycolipid-Biotensiden. In herkömmlichen Mitteln mit vornehmlich anionischen Tensiden, z.B. den Sulfattensiden, wird dies durch eine höhere Tensidkonzentration oder Zugabe von aufwändig synthetisch hergestellten Alkylpolyglykosiden (APG) oder mittelkettige Alkylbetaine, z.B. Cocoamidopropylbetain (CAPB) kompensiert, beides irritierende Tenside. Hartes Wasser, Gebrauchswasser oder Elektrolyte wirken bei Tensiden und deren Mischungen oft nachteilig auf Eigenschaften wie Schaum, Benetzungsvermögen, Reinigungsvermögen, Emulgiervermögen, Rückstandsbildung und Stabilität aus. Üblicherweise müssen Wasch- und Reinigungsmittel daher bereits bei mittlerer Wasserhärte (1,5 mmol/l Calciumcarbonat) höher dosiert werden um eine vergleichbare Leistung wie bei weichem Wasser zu erreichen. Viele Zusammensetzungen enthalten daher Enthärter, die die Umwelt belasten können. Bei Wasser Die Dosierung der Produkte bei der Anwendung. Ziel ist daher, auch in Anwesenheit von Elektrolyten, in hartem Wasser oder Gebrauchswasser (Wasser geringerer Reinheit) eine gute Leistung zu erzielen, möglichst ohne die Tensidkonzentration erhöhen zu müssen.

Weiterhin besteht der Wunsch, über möglichst ökologische, pflanzenbasierte und wenig augen- oder hautreizende Zusammensetzungen zu verfügen. Die Verwendung milder Tenside führt jedoch oft zu einem Verlust an Reinigungsleistung und / oder Schaumvermögen, was der Anwender durch höhere Dosierungen oder einem grösseren mechanischer Aufwand auszugleichen versucht.

Für die Ökologie, spielt nicht nur die biologische Abbaubarkeit eine Rolle, vielmehr ist die Herkunft und Art der

Gewinnung von Rohstoffen zunehmend von Bedeutung - insbesondere in einer holistischen Betrachtung im Sinne des Klimaschutzes, Erhalt der Biodiversität und Schonung erschöpfender Rohstoffe. Viele Tenside werden aus Erdöl hergestellt, beispielsweise kann der hydrophobe Teil oder der hydrophile Teil der Tenside aus Erdöl stammen (petrochemisch), bspw. Alkoxylate. Der petrochemische Anteil (z.B: Alkoxylate) soll aus Nachhaltigkeitsbetrachtungen in dieser Erfindung möglichst minimiert werden.

In den letzten Jahrzehnten wurden zunehmend Tenside mit einem hydrophoben Teil basierend auf Palmkernöl eingesetzt. Da deren Einsatz aufgrund von langen Transportwegen und Monokulturen nicht unproblematisch ist, ist es zugunsten eines nachhaltigen Formulierungskonzepts wünschenswert, neben Erdöl auch Pflanzenöle aus den tropischen Zonen, d.h. z.B. insbesondere Palmöle zu reduzieren oder ganz zu vermeiden. Durch die bevorzugte Verwendung von Pflanzenölen der gemässigten Klimazone (C-18-Pflanzenöle) oder fermentativ hergestellte Tenside (Biotenside) können weite Transportwege verhindert und der tropische Regenwald als Biosphäre geschützt werden.

Die technische Herausforderung besteht daher darin, Erdöl, tierische Fette und Öle, sowie Palmöle (d.h. Palmöl, Palmkernöl) als Fettsäurequelle in der Zusammensetzung zu reduzieren und stattdessen in einem grösstmöglichen Mass Inhaltsstoffe, insbesondere Tenside aus weniger problematischen Quellen einzusetzen. Idealerweise soll auch die Herstellung der Tenside möglichst umweltverträglich sein, das heisst zum Beispiel kurze Synthesewege, möglichst wenig Reinigungsschritte und möglichst wenig Abfall. Die erfindungsgemässen Zusammensetzungen sollen zu einem grösstmöglichen Umfang auf natürlichen Rohstoffen basieren und biologisch gut abbaubar sein, sowie einen möglichst hohen Anteil an regenerativem Kohlenstoff in der Zubereitung aufweisen.

Weiterhin sollen die Tensid- Zusammensetzungen eine möglichst grosse Formulierungsflexibilität aufweisen, z.B. für die Wahl der weiteren Inhaltststoffe. So sollen idealerweise auch variable Ausführungsformen möglich sein, bei denen wahlweise auf üblicherweise eingesetzte Tenside verzichtet werden kann, welche z.B. aufgrund deren Irritationspotential (z.B. Sulfate), Kundenwahrnehmung (z.B. Alkoxylate) oder zugrundeliegenden Rohstoffe (z.B. Palmkerntenside) weniger bevorzugt sind.

Weiterhin bestand die Aufgabe neben fliessfähigen Ausführungsformen auch feste Formulierungen zu ermöglichen. Feste Formulierungen haben gegenüber fliessfähigen Formulierungen den Vorteil, dass sie plastikarm verpackt werden können. Viele heutige Produkte sind nur in flüssiger Form verfügbar. Andere Produkte in fester Ausführung zeigen Nachteile in der Anwendung, wie z.B. geringere Leistung oder erschwertes Handling, z.B. durch Bildung einer schlecht löslichen Oberfläche mit hartem Wasser.

Es war daher die Aufgabe, Zusammensetzungen, sowie Reinigungs- oder Pflegemittel umfassend diese Zusammensetzungen zu entwickeln, welche eine oder mehrere der oben genannten Eigenschaften erfüllen. Darüber hinaus wurde angestrebt, Zusammensetzungen zu entwickeln, deren Inhaltsstoffe zu einem grösstmöglichen Mass pflanzlichen Ursprungs sind, bevorzugt sollen hierbei Palmkerntenside reduziert oder ganz vermieden werden.

**State of the art**

N-Acylglycamine, bevorzugt N-Alkyl-N-Acylglucamine, mit hydrohilen Resten aus Kohlenhydraten und lipophilen Resten bevorzugt von Fettsäuren von Pflanzenölen, besonders bevorzugt aus C-18-Pflanzenölen, erfüllen die gewünschten Anforderungen an Nachhaltigkeit und Toxikologie. N-Alkyl-N-Acylglycamine sind bekannt als milde Co-Tenside. Meist liegen sie in geringen Mengen zusammen Tensiden aus tropischen Pflanzenölen als Haupttensiden vor, z.B. linearen Alkylsulfaten oder linearen Alkylethersulfaten (v.a. Sodium lauryl sulfat, Sodium laureth sulfat). Hauptsächlich werden N-Methyl-N-Acylglucamine basierend auf destillierten Fettsäuren von Palmkernöl mit Fettsäureacylresten zwischen 8 und 14 Kohlenstoffatomen in kosmetischen Reinigungslösungen mit Sulfaten oder Sulfonaten eingesetzt. N-Acylglycamine mit ungesättigten Fettsäureacylreste mit 18 und mehr Kohlenstoffatomen treten, wenn überhaupt, meist als untergeordnete Nebenkomponente in Erscheinung.

Seit den letzten Jahren werden auch langkettige, vorwiegend ungesättigte N-Alkyl-N-acylglucaminen aus C-18-Pflanzenölen in Kombination mit dem Schaumboostern Cocoamidopropylbetaine (CAPB) und gegebenfalls Alkyl(poly)glycosiden (APG) eingesetzt, insbesondere wenn eine starke Schaumbildung erwünscht ist, wie z.B. bei Shampoos oder manuellen Geschirrspülmitteln. Dies ist insbesondere bei Handgeschirrspülmitteln von Bedeutung, da nach Industriestandard deren Reinigungsleistung über die Schaummenge definiert wird. Des Weiteren werden diese N-Alkyl-N-acylglucaminen in Tensidmischungen für die Konditionierung z.B. zur Haarkonditionierung (WO2014/206555), Rückfettung, z.B. als hautpflegende Komponente (US2016/0136072) oder als Verdicker (WO 2013/178697) einsetzt.

Kurzkettige cyclische N-Acylglycamine sind aus WO 2018002100 als biozide Wirkstoffe bekannt in unterschiedlichen kosmetischen Mitteln bekannt. Die Kombination von N-Acylglycaminen, insbesondere von langkettigen, vorwiegend ungesättigten N-Alkyl-N-acylglucaminen oder cyclischen N-Acylglycaminen mit Glycolipid-Biotensiden ist in keinem der Prior Art Dokumente nahegelegt.

Glycolipid-Biotenside werden über Fermentation mit Mikroorganismen aus unterschiedlichsten Substraten hergestellt. Sie erfüllen die Anforderungen an Nachhaltigkeit und zeichnen sich zudem durch eine geringe Toxizität aus. Im Gegensatz zu synthetischen Glycolipiden, wie z.B. Alkyl(poly)glycosiden oder Alkyl(poly)glucosiden, den APGs,

werden Biotensid-Glycolipide biotechnologisch mithilfe von Mikroorganismen wie Bakterien, Hefen oder Pilzen hergestellt. Die Mikroorganismen können aus natürlich vorkommenden Quellen stammen oder für Fermentationszwecke optimiert worden sein, z.B. gentechnisch. Weiterhin ist eine chemische Modifizierung der Ausgangsstoffe für die Fermentation oder der fermentativ erhaltenen Biotenside möglich wie z.B. Acetylierungen, Sulfatierungen, Carboxlierungen, Ethoxylierungen u.a.

Bekannt sind Mittel mit Biotensiden für eine verbesserte Fettlösekraft. Eingesetzt werden sie vor allem in Kombination mit vorwiegend nicht-glycolipischen, gesättigten, mittelkettigen Tensiden auf Basis von tropischen oder petrochemischen Ölen, z.B. offenbart in EP 0 499434 A1 (Textilwaschmittel mit einer erhöhte Öllösekraft) oder EP 1 445 302 A1 (erhöhte Reinigungsleistung auf Standardschmutz oder Mineralöl bei geringer Schaumbildung). WO 2016050439 offenbart biotensidhaltige Formulierungen mit verbessertem Schaumbildungs- und Fettlösevermögen enthaltend mindestens ein Tensid ausgewählt aus der Gruppe der Betaine, alkoxylierte Fettalkoholsulfate und Alkylaminoxid. Es werden auch Kombinationen offenbart, in denen neben nicht-glykolipidischen Tensiden zusätzlich Alkylpolyglycoside als Co-Tenside eingesetzt werden. Aufgrund der geringen Schaumstabilität von Biotensiden, insbesondere in hartem Wasser, ist die technische Wirkung der bekannten Zubereitungen, welche sich in erster Linie im Schaumvermögen manifestiert, der Kombination der Biotenside mit nicht-glykolipidischen Tensiden und/oder mit Tensiden mit überwiegend kurz- oder mittelkettigen (< C18), gesättigten Kohlenwasserstoffketten als hydrophobem Teil der Tenside, v.a. Kokos oder Lauryl, geschuldet.

Um die gewünschten Schaum- und Reinigungseigenschaften zu erzielen, werden in der Literatur sowohl N-Alkyl-N-Acylglycaminen als auch Glycolipid-Biotensiden jeweils als Co-Tenside, also als unterstützende Tenside in untergeordneten Mengenanteilen, in Kombination mit primären Tensiden eingesetzt. Die stark entfettende Wirkung der primären Tenside auf die Haut wird durch die N-Alkyl-N-Acylglycamine oder durch Glycolipid-Biotenside abgemildert, durch die Zugabe der primären Tenside werden die wesentlichen Aspekte der Reinigungsaufgabe gelöst (d.h. Benetzen, Emulgieren, Dispergieren). Das Problem ist daher noch nicht gelöst, einen möglichst hohen Anteil an nachhaltigen Tensiden, und bevorzugt gleichzeitig einen möglichst geringen Anteil oder gar keine Erdöl-Tenside einzusetzen, um rückstandsarme, gegebenenfalls schaumstabile Mittel mit gutem Emulgier- oder Fettlöseverhalten und Benetzungsvermögen zu erhalten.

Es galt also Zusammensetzungen bzw. Mittel zu finden, die möglichst viele der Anforderungen an Milde, Sensorik, Reinigungsleistung, Emulgierfähigkeit, Fettlösevermögen, Rückstandsarmut, Schaum und Nachhaltigkeit erfüllen und die idealerweise diese Eigenschaften auch in Anwesenheit von Elektrolyten, Gebrauchswasser oder hartem Wasser aufweisen. Die Herausforderung ist unter anderem, dass die Reinigungswirkung und Schaumeigenschaften von Zusammensetzungen nicht vorausgesagt werden können. Tenside treten mit anderen Stoffen und insbesondere auch untereinander in Wechselwirkung, die die Eigenschaften wie Schaum, Reinigungsleistung, Rückstand, etc. bestimmen. Eine Voraussage über die Leistung einer Tensidkombination kann nicht a priori getroffen werden.

Überraschend gelöst werden diese Aufgaben durch Zusammensetzungen enthaltend mindestens ein N-Acylglycamin, bevorzugt zusätzlich enthaltend ein oder mehrere Glycolipid-Biotenside umfassend Rhamnolipide, Sophorolipide, Mannosylerythritollipide, Cellobioselipide und Trehaloselipide, sowie deren Kombinationen. Mit Hilfe dieser Erfindung konnten Reinigungs-und Pflegemittel geschaffen werden, welche ein gutes Reinigungsvermögen, geringe Rückstandsbildung, vorteilhafte Schaumeigenschaften und ein gutes Benetzungsvermögen, Eignung für hartes Wasser oder hohe Elektrolytkonzentrationen, und eine gute Sensorik aufweisen.

Die Lösung der Aufgabe war insbesondere deshalb für die Fachperson nicht naheliegend, weil sie davon ausgehen musste, dass Mittel mit N-Acylglycaminen, welche bekanntermassen eine mässige Wasserlöslichkeit aufweisen, nicht rückstandsarm sind. Dies gilt insbesondere für die bevorzugte Ausführungsform mit N-Acylglycaminen aus C-18-Fettsäuren, die einen hohen Anteil an langkettigen, ungesättigten Fettsäureresten aufweisen und zudem bevorzugt als Tensidgemisch mit Acylresten unterschiedlicher Kettenlängen und Sättigungsgrade vorliegen. Hier würde die Fachperson aufgrund der langen Kettenlänge und der erhöhten Reaktivität durch die Doppelbindung, Schlieren- oder Rückstandsbildung erwarten. Auch die gute Benetzbarkeit selbst bei langer Kettenlängen (ab C-18) ist überraschend. In keiner Weise vorhersehbar sind für die Fachperson Synergien mit Biotensiden, welche für die erfindungsgemässen Zusammensetzungen gefunden wurden, zum Beispiel in Anwesenheit von Elektrolyten.

Für die Fachperson war es zudem in keiner Weise naheliegend, zwei scheinbar gleichgerichtete Tensidklassen, wie hier zwei Glycolipide zu kombinieren. Überraschenderweise konnten neuartige flüssige und feste Zusammensetzungen enthaltend mindestens ein N-Acylglycamin, bevorzugt zusätzlich enthaltend ein oder mehrere Glycolipid-Biotenside, mit vorteilhaften Eigenschaften und einem hervorragenden ökologischen und toxikologischen Profil erhalten werden. Insbesondere ist überraschend, dass die Tensidkombination von N-Acylglycaminen mit Biotensiden zudem synergistisch wirkt.

Vorteil der Erfindung ist, dass die erfinderischen Mittel enthaltend N-Acylglycamine, und bevorzugt zusätzlich enthaltend mindestens ein Biotensid-Glycolipid, überraschenderweise keine oder nur geringe, inbesondere intrinsische, Rückstände bilden, d.h. gleiche oder weniger Rückstandsbildung als alternative Zusammensetzungen ohne N-Acylglycamin, bzw. ohne die Kombination von N-Acylglycaminen mit Glycolipid-Biotensiden. Das Substrat wird bzw. bleibt

infolge der geringeren intrinsischen Rückstandsbildung nach Anwendung des Mittels rückstandsarm. Es werden glatte und saubere Oberflächen mit einer angenehmen Haptik erhalten. Insbesondere in der Haut- und Haarpflege wirken sich Rückstände negativ auf die Sensorik und Pflegeleistung aus. Anders als Zusammensetzungen, die Schmutz in der Waschflotte halten und so als Antiredepositionsmittel wirken, haben die erfinderischen Zusammensetzungen den Vorteil, dass sie auch ohne Nachspülen - oder allenfalls mit nur einer geringen Menge Wasser- rückstandsarm sind oder auf glatten und glänzenden Oberflächen oder Haut, Haar und Zähnen angewendet werden können. In anderen Anwendungen kann hierdurch auf einen Polierschritt verzichtet werden. Dies erlaubt eine resourcenschonende Anwendung der Mittel unter Verzicht eines zusätzlichen Spül- oder Polierschritts.

Ein weiterer Vorteil ist der verbesserte Glanz der Substrate nach Anwendung dieser erfinderischen Zusammensetzung, insbesondere der synergistischen Tensidmischungen mit Biotensiden.

Besonders geeignet sind die Mittel daher für wassersparende Kurz- oder Ecoprogramme in Wasch- oder Spülmaschinen, Anwendungen ohne Spülen, z.B. Triggeranwendungen, Wischtücher oder auf no-rinse Anwendungen z.B. Gesichtwasser, Mundwasser.

Ein weiterer Vorteil ist, dass die Zusammensetzungen eine hohe Schaumstabilität aufweisen. Während der Schaum von Biotensid-Glycolipiden, insbesondere von Sophorolipiden in Verwendung mit hartem Wasser, schnell wieder zusammenfällt, kann der Schaum in Kombination mit N-Acylglycaminen stabilisiert werden. Anders als die bisherige Anwendung von Biotensiden, insbesondere Sophorolipiden und Rhamnolipiden, die bei Wasserhärten unter 8,43° dH und Formulierungen mit geringer Ionenstärken, oder Zugaben von Enthärtern, verwendet werden, können die neuartigen Zusammensetzungen mit N-Acylglycaminen überraschenderweise auch in hartem Wasser oder in Anwesenheit von Elektrolyten mit gutem Anschäumverhalten und Schaumstabilität angewendet werden.

Die N-Acylglycamine gemäss der Erfindung, bevorzugt in Kombination mit mindestens einem Biotensid, können auch als Additive für eine gute Schaumbildung und -stabilität in hartem Wasser verwendet oder bei hohen Ionenstärken verwendet werden. Dies erhöht die Formulierungsflexibilität. Die erfinderischen Zusammensetzungen können, z.B. in Shampoos oder Geschirrspülmittel, sparsam verwendet werden.

Die erfinderischen Zusammensetzungen sind für Wasser ohne Enthärterzugaben mit einem Calciumcarbonatgehalt von > 0,89 mmol/l (5° dH) bevorzugt von ≥ 1,5 mmol/l (8,43°dH), besonders bevorzugt von > 3,2 mmol/l (18°dH). (≥ 15°F, bevorzugt > 16°d) oder Salzwasseranwendungen geeignet, zeigen weiterhin auch unter diesen Bedingungen ein hohes Emulgierverhalten bei Fett, eine hohe Reinigungsleistung und bilden einen stabilen Schaum, insbesondere auch ohne APGs auf Basis von tropischen Pflanzenölen und/oder mittelkettigen Alkylbetainen, insbesondere ohne CAPB.

Insbesondere feste Formulierungen bilden an der Oberfläche mit hartem Wasser eine schlecht lösliche Schicht und machen damit die Produkte nahezu unbrauchbar. Wesentlicher Vorteil ist daher für feste Formulierungen mit mindestens einem Biotensid oder mindestens einem N-Acylglycamin oder deren Kombination, dass bei der Anwendung, z.B. Reiben in den Handflächen, eine ausreichende Menge an Produkt freigesetzt wird.

Die erfindungsgemässe Zusammensetzung, enthaltend ein oder mehrere N-Acylglycamine, und bevorzugt zusätzlich enthaltend mindestens ein Biotensid-Glycolipid, weist zudem eine angenehme Sensorik auf. Sie weisen gleichzeitig eine geringe Haut-, Schleimhaut- und Augenreizung auf. Dies ermöglicht die Anwendung auf empfindliche Körperteile, z.B. in der Mundhygiene oder Intimpflege. Trotz hoher Reinigungsleistung wird die Zusammensetzung als nicht austrocknend und damit hautpflegend empfunden. Rückfettende Substanzen können minimiert oder ausgelassen werden. Insbesondere die festen Formulierungen mit mindestens einem Biotensid oder mindestens einem N-Acylglycamin oder deren Kombination, weisen eine gute Sensorik und gefühlte Rückfettung auf.

Ein Vorteil ist weiterhin, dass in den fliessfähigen Zusammensetzungen enthaltend ein oder mehrere Biotensid-Glycolipide das mässig wasserlösliche N-Acylglycamin in Lösung gehalten oder gebracht wird. Die Konzentration der N-Acylglycamine kann gegenüber Zusammensetzungen ohne Glycolipid-Biotenside erhöht werden, Lösungsvermittler können reduziert oder ganz ausgelassen werden und es werden stabile Zusammensetzungen erhalten, z.B. kann so die Formulierung von klaren Produkten ermöglicht werden. In den festen Formulierungen wurde überraschend festgestellt, dass die Zusammensetzungen enthaltend mit mindestens einem Biotensid oder mindestens einem N-Acylglycamin oder deren Kombination sich positiv auf die Stabilität auswirken. Es wird vermutet, dass die Emulgierkraft der Glycolipid-Biotenside und/oder der N-Acylglycamine, insbesondere bei deren Kombination, zur Stabilität beiträgt.

Die erfinderischen Zusammensetzungen enthaltend ein oder mehrere N-Acylglycamine, und bevorzugt zusätzlich enthaltend mindestens ein Biotensid-Glycolipid, zeigen ein überraschend gutes Emulgierverhalten von öl- oder fetthaltigen Verbindungen. Dies erhöht zum einen die Formulierungsflexibilität für lipophile Inhaltsstoffe, zum Beispiel in Pflegeprodukten, zum anderen zeigen die erfinderischen Zusammensetzungen damit ein gutes Reinigungsvermögen von lipophilem Schmutz. Durch das Emulgiervermögen der N-Acylglycamine, inbesondere in Kombination mit Biotensiden, können so W/O oder O/W-Emulsionen gebildet werden, bevorzugt die O/W-Emulsion. Die festen Formulierungen mit mindestens einem Biotensid oder mindestens einem N-Acylglycamin oder deren Kombination erlauben dank ihres überraschend guten Emulgierverhaltens eine gute Homogenisierung der Komponenten und eine stabile feste Produktform.

Die erfinderischen Zusammensetzungen weisen ein hervorragendes Nachhaltigkeitsprofil auf. Die Zusammen-

setzungen haben einen geringen $CO_2$-Fussabdruck, schonen natürliche Habitate (z.B. Regenwald) und fördern die Biodiversität. Sie sind gut biologischer abbaubar. Gegenüber herkömmlichen Zusammensetzungen auf petrochemischer oder rein oleochemischer Basis tropischer Pflanzenöle stellt dies einen markanten Vorteil dar, der zunehmend in der Öffentlichkeit wahrgenommen wird und das Kaufverhalten beeinflusst. Die erfinderischen Zusammensetzungen sind a priori frei von Alkoxylaten, z.B. PEG-frei (PEG = Polyethylenglykol). Die als problematisch empfundene Inhaltsstoffe, wie bspw. Alkoxylate oder PEG-Verbindungen können reduziert werden oder gar auf deren Einsatz verzichtet werden. Feste Formulierungen können verpackungssparend, z.B. plastikarm konfektioniert werden.

Die erfinderischen Zusammensetzungen enthaltend ein oder mehrere N-Acylglycamine, und bevorzugt zusätzlich enthaltend mindestens ein Biotensid-Glycolipid, zeigen einen überraschenden synergistischen Effekt in ihrem Benetzungsverhalten und unterstützen damit den Reinigungs- und Pflegeprozess.

Die erfinderischen Zusammensetzungen enthaltend ein oder mehrere N-Acylglycamine, und bevorzugt zusätzlich enthaltend mindestens ein Biotensid-Glycolipid, weisen eine erstaunliche Formulierungsflexibilität auf. Dies erlaubt dem Formulierer, schnell auf Rohstoffverfügbarkeiten, Preisänderungen und geänderte Regulatorien zu reagieren. Insbesondere sind folgenden Ausführungsformen der Zusammensetzungen offenbart:
Die Ausführungsformen ohne Sulfate (z.B. Sodium laureth sulfate, sodium lauryl sulfate) haben eine gute Reinigungswirkung und Emulgierwirkung (z.B. auf Fette). Anionische Sulfat-Tenside gelten als irritierend und werden von bestimmten Konsumentengruppe als nachteilig angesehen. Bevorzugt sind Laureth sulfat-freie Ausführungsformen, bevorzugter Alkylethersulfat-freie, ganz besonders bevorzugt vollständig sulfat-freie Ausführungsformen, insbesondere gilt dies für die Zusammensetzungen in der kosmetischen Anwendung.

Die erfinderischen Zusammensetzungen sind a priori PEG-frei (PEG = Polyethylenglykol). Die Besonders bevorzugt werden Ausführungsformen, die einen geringen Gehalt an PEG-Tensiden aufweisen und Ausführungsformen, die keine PEG-Tenside, besonders bevorzugt keine alkoxylierten Tenside enthalten. Neben der Reduktion bzw. Vermeidung von Ethylenoxid als petrochemischen Rohstoff oder der aufwändigen Synthesen über pflanzliche Rohstoffe, weist die Zusammensetzung nicht den typischen, als unangenehm empfundenen Glykoleigengeruch auf. Mindestens eine der Ausführungsformen ist parfümfrei und wird als angenehm emfunden. Besonders vorteilhaft ist, dass PEG-Verbindungen bei mindestens gleich bleibender Leistung in der erfinderischen Zusammensetzung und insbesondere bei der Kombination mit Biotensiden vollständig oder teilweise ersetzt werden können.

Die erfinderischen Zusammensetzungen zeigen überraschenderweise auch ohne CAPB oder APGs hervorragende Eigenschaften betreffend Schaum, Reinigungskraft, Emulgierverhalten, Rückstandsbildung und Sensorik.

**Definitionen**

"Enthaltend" im Sinne der Erfindung schliesst ein, dass Komponenten der Erfindungen erst bei der Verwendung miteinander in Kontakt kommen, z.B. wenn die Ausführungsform kompartimentiert dargeboten wird, z.B. N-Acylglycamin in einer Phase/ Kompartiment und das Biotensid in einer anderen Phase/ Kompartiment. Beispiele hierfür sind Tabs, Pods, Sachets u.ä.

Verhältnisse sind soweit nicht anders angegeben als Mengenverhältnisse zu verstehen. Ein Verhältnis > 1 : 5 bedeutet z.B. 1 : 4, 1: 3, 1: 2, u.s.w.; Ein Verhältnis < 1 : 5 bedeutet z.B. 1 : 6 1: 7, 1: 8, u.s.w.

Tenside sind amphiphile organische Substanzen mit jeweils mindestens einem hydrophilen und mindestens einem hydrophoben Rest. In Lösung tendieren Tenside zur Selbstaggregation und bilden Mikrostrukturen wie bspw. Mizellen, lamellare Strukturen u.a. Diese Strukturen bilden sich abhängig von der Art der Tenside und deren Mengen-Verhältnissen und beeinflussen so die Eigenschaften dieser Lösung. Im Zusammenhang mit dieser Erfindung werden als Tenside bevorzugt Verbindungen bezeichnet, die die Fähigkeit besitzen, die Oberflächenspannung von Wasser bei 20°C und bei einer Konzentration von 0.5 Gew.-% bezogen auf die Gesamtmenge der Zubereitung auf unter 45 mN/m zu verringern.

Unter einer festen Formulierung im Sinne der Erfindung, werden Ausführungsformen verstanden, die bei Raumtemperatur fest sind. Feste Formulierungen sind nicht fliessfähig.

Die feste Produktform kann sich z.B. in Blättern ("sheets"), Stücken ("Bars"), Flocken, Nudeln ("Noodles"), Pulver, Granulaten, Pearls, Pastillen, Pucks, Sticks, Kapseln, Extrudaten, Komprimate, Tabletten, gehärtete Schmelzen oder Kombinationen derselben manifestieren. Sie können z.B. durch Härtung, z.B. von Schmelzen, Copolymerisation, Extrudieren, Kompression (Pressung, Tablettierung), oder Trocknung, gegebenenfalls auf einer Trägersubstanz entstehen.

Je nach fester Formulierungen können diese bis zu 25 Gew.-% Lösungsmittel enthalten, bevorzugter < 15 Gew.-%, noch bevorzugter < 9 Gew.-%, besonders bevorzugt < 8 Gew.-%, ganz besonders bevorzugt < 2 Gew.-%. In einer äussert besonders bevorzugten Form enthält die feste Form nur noch geringe Mengen an Lösungsmittel, d.h. < 1 Gew.-%, besonders bevorzugt < 0,5 Gew.-%, jeweils bezogen auf das Gewicht der Zusammensetzung.

Feste Formulierungen in Form von Stücken ("Bars"), Pucks, Sticks, Kapseln, insbesondere für die Kosmetik oder Weichspüler, weisen einen bevorzugten Shore AO-Härtewert von 15 bis 35 auf, insbesondere von 20 bis 30.

" Biotensid": Im Sinne dieser Anmeldung wird, soweit nicht explizit anders genannt, unter Biotensid ein Glycolipid-Biotensid verstanden umfassend Rhamnolipide, insbesondere Mono-, Di- oder Polyrhamnolipide oder deren Gemische, Sophorolipide, insbesondere in ihrer Säure- oder Laktonform oder in beiden Formen, diacetyliert, acetyliert oder nicht-acetyliert, oder jeweils deren Gemische, Mannosylerythritollipide, Cellobioselipide und Trehaloselipide, sowie Kombinationen der einzelnen Glycolipid-Biotensid-Klassen. Mitumfasst werden unter dem Begriff "Biotensid" auch deren protonierten Formen sowie deren Salze. Bevorzugt liegen diese Glycolipid-Biotenside als Tensidgemische vor, je nach Biotensid z.B. unterschiedlicher Substituierungsgrade, unterschiedliche Kettenlängen der Kohlenwasserstoffreste, unterschiedliche Sättigungsgrade der Kohlenwasserstoffreste, Lakton-/Ester-Gemisch, u.a.

Die Herstellung der Biotenside umfasst mindestens einen fermentativen Schritt. Chemische Syntheseschritte können zusätzlich zum fermentativen Schritt erfolgen.

"C-18-Pflanzenöle": Technisch unterscheiden sich die Pflanzenöle aus Ölpalmen, Babassu, Palmkernen, oder Kokosnüssen deutlich in der Fettsäurezusammensetzung von den C-18-Pflanzenölen, welche in der klimatischen Zone der gemässigten Breiten heimisch sind und dort gedeihen können. Bei den C-18-Pflanzenölen handelt es sich um natürliche Triglyceride. In dieser Erfindung werden solche Pflanzenöle als C-18-Pflanzenöl verstanden, deren Fettsäuren als ein Gemisch an gesättigten und ungesättigten Fettsäuren vorliegen, wobei die Fettsäureverteilung von Fettsäuren mit 18 und mehr Kohlenstoffatomen über 60 Gew.-%, besonders bevorzugt über 72 Gew.-% und ganz besonders bevorzugt von über 77 Gew.-% liegt und wobei der Anteil an ungesättigten Fettsäuren über 55 Gew.-%, vorzugsweise über 65 Gew.-% und besonders bevorzugt über 72 Gew.-% liegt. Bevorzugt liegt zudem der Anteil an Fettsäuren mit 16 und weniger Kohlenstoffatomen unter 30 Gew.-%, bevorzugt unter 25 Gew.-% und besonders bevorzugt unter 17 Gew.-%. Bevorzugt enthalten die C-18-Pflanzenöle einen Anteil von < 0.5%, besonders bevorzugt > 0.05% Fettsäuren mit 6 Kohlenstoffatomen. Bevorzugt enthalten die C-18-Pflanzenöle einen Anteil von < 75 Gew-% Hydroxyfettsäuren, bevorzugt < 25 Gew.-%, besonders bevorzugt < 5 Gew.-%. Bevorzugt enthalten C-18-Pflanzenöle gesättigte oder ungesättigte Fettsäuren mit 20 und mehr Kohlenstoffatomen, wobei deren Gehalt bis zu 96 Gew.-% betragen kann. Bevorzugt enthalten C-18-Pflanzenöle einen Anteil von gesättigten oder ungesättigten Fettsäuren mit 20 und mehr Kohlenstoffatomen von > 0.01 Gew.-% und besonders bevorzugt > 0.05 Gew.-% und ganz besonders bevorzugt > 0.1 Gew-% und äusserst bevorzugt >= 0.2 Gew.-% . Bevorzugt enthalten die C-18-Pflanzenöle einen Anteil von weniger als 95 Gew.-% Ölsäure, besonders bevorzugt unter 85 Gew.-% Ölsäure.

Hierbei sind jeweils auch die Kombinationen von mindestens zwei dieser Anteile an Fettsäuren bevorzugt, insbesondere beliebige Kombinationen von zwei oder mehreren dieser Anteile an Fettsäuren. Gew.-% hier jeweils bezogen auf den Gesamtgehalt an Fettsäuren im Pflanzenöl.

C-18-Pflanzenöle werden aus Pflanzen ("C-18-Pflanzen") gewonnen, die dem Brassica-, Carthamus-, Arachis- und Linumtyp zugeteilt sind. Aus folgenden bevorzugten Pflanzen bzw. Pflanzenteilen, wie Samen, Kerne, Früchte, Blätter, Wurzeln und andere, im folgenden C-18-Pflanzen genannt, werden C-18 Pflanzenöle gewonnen, welche die technischen Merkmale betreffend Fettsäurezusammensetzungen für die erfindungsgemässen Mittel erfüllen und definiert sind wie folgt: Amarant, Anis, Apfel, Aprikose, Argan, Arnika, Avocado, Baumwolle, Borretsch, Brennessel, Brokkoli, Canola, Chia, Hanf, Haselnuss, Buche, Buchsbaum, Distel, Dinkel, Erdnuss, Erdmandel, Flieder, Gartenkresse, Gerste, Granatapfel, Hafer, Hanf, Haselnuss, Heidelbeere, Holunder, Jasmin, Johannisbeere, Johanniskraut, Jojoba, Kamelie, Kamille, Kümmel, Karotte, Kirsche, Koriander, Königskerze, Krambe, Kreuzblättrige Wolfsmilch, Kreuzblütengewächse, Kürbis, Iberischer Drachenkopf, Lavendel, Leindotter, Leinsamen, Liguster, Lupine, Luzerne, Macademia, Mais, Mandel, Marula, Mirabelle, Melone, Mohn, Mongongo, Nachtkerze, Olive, Ölrettich, Ölrauke, Passionsblume, Pekannuss, Pfirsich, Pflaume, Pistazie, Preiselbeere, Purgiernuss (Jatropha), Raps, Reis, Ringelblume, Rübsen, Saflor, Salbei, Sanddorn, Schwarzkümmel, Sesam, Sesamblatt, Senf, Sonnenblume, Soja, Tabak, Walnuss, Weintraube, Weizen, Wiesenschaumkraut und Wildrose; sowie deren Kombinationen.

" Abgeleitet von Pflanzenölen": Es ist ökonomisch und ökologisch vorteilhaft, Mischungen von Fettsäuren unterschiedlicher Kettenlängen und Sättigungsgrade in Reaktionen der Oleochemie einzusetzen. Im Rahmen der vorliegenden Erfindung steht - soweit nicht anders angegeben- "auf Basis von" oder "abgeleitet von Pflanzenölen" oder "stammend von Pflanzenölen" oder "aus Pflanzenölen" stellvertretend für Derivate aus Fettsäuren - gereinigt oder als Gemisch - und/oder deren Reaktionsprodukte, wie beispielsweise Reaktionen an die Doppelbindung, Reaktionen an der Fettsäurefunktion, wie z.B. Fettalkohole und deren Ether und/ oder Carboxylether, Amine oder Fettsäureamide, Fettsäureester, sowie Imine. Bevorzugt liegen diese Fettsäurederivate als Mischung gemäss der Fettsäureverteilung im nativen Öl vor oder wie sie bei der Umsetzung von natürlich vorkommenden Pflanzenöle oder Fetten anfallen.

"Abgeleitet von C-18-Pflanzenölen" bedeutet, dass mindestens einer, bevorzugt mehrere Verbindungen mit Fettresten vorliegen, d.h. Fettsäureacyl- (R(O)O-) oder Fettalkoholresten (RO-) die sich in Länge und/oder Sättigungsgraden der Fettresten unterscheiden vorliegen, mit R einem aliphatischen gesättigten oder ein- oder mehrfach ungesättigten Rest mit 6 bis 24 Kohlenstoffatomen; bevorzugt liegt eine Verteilung an Resten R vor (entsprechend der Fettsäureverteilung gemäss obigen Definition für C-18-Pflanzenöle bezogen auf die Reste R, wobei ein Fettsäurerest R(O)O- darstellt) , d.h. z.B. eine Verteilung von R mit 18 und mehr Kohlenstoffatomen über 60 Gew.-%, besonders bevorzugt über 72 Gew.-% und ganz besonders bevorzugt von über 77 Gew.-% liegt und einem Anteil an ungesättigten

Resten R über 55 Gew.-%, vorzugsweise über 65 Gew.-% und besonders bevorzugt über 72 Gew.-% liegt, bezogen auf die Reste R in der entsprechenden Verbindung u.s.w.

Unter Fettrest wird ein Fettsäureacyl- oder ein Fettalkoholrest verstanden. Diese werden von Fettsäuren abgeleitet. Wird der Fettrest bspw. von Ölsäure $C_{17}H_{33}COOH$ abgeleitet, wird unter dem Fettsäureacylrest abgeleitet von Ölsäure der Rest $C_{17}H_{33}CO$- (Oleoyl-) verstanden; unter dem Fettalkoholrest abgeleitet von Ölsäure wird der Oleylrest $C_{18}H_{35}O$ verstanden. Falls explizit angegeben, können die ungesättigten Fettsäuren gegebenenfalls hydrogeniert oder teilweise hydrogeniert werden.

Im Rahmen dieser Erfindung ist unter dem Gemisch an N-Acylglycaminen das N-Acylglycamin-Produktgemisch, welches aus der Umsetzung eines entsprechenden Gemischs aus Fettsäurederivaten eines Pflanzenöls (z.B. Fettsäurehalogeniden, Fettsäureester wie Fettsäurealkylester oder Fettsäureglyceride) oder aus einem Pflanzenöl als Edukte erhalten wird. Die Reaktion kann mehrstufig verlaufen. Das von einem Pflanzenöl abgeleitete Gemisch besteht aus N-Acylglycamine unterschiedlicher Kettenlängen und/oder Sättigungsgraden des Fettsäureacylrestes wie aus der Reaktion erhalten. Dieses "Gemisch" weist im Falle von C-18-Pflanzenölen, sofern nicht anders genannt, eine Verteilung der Fettsäureacylreste wie unter "C-18-Pflanzenöl" definiert auf. Es entstehen analoge Produktgemische mit Verteilungen der Fettsäurereste wie bei den Edukten. Bevorzugt liegt in dem Gemisch an N-Acylglycaminen die gleiche Fettsäureverteilung wie im ursprünglichen Pflanzenöl vor.

Im Rahmen der vorliegenden Erfindung stehen Fettsäuren bzw. Fettalkohole bzw. deren Derivate soweit nicht anders angegeben - stellvertretend für verzweigte oder unverzweigte, lineare oder substituierte, insbesondere hydroxysubstituierte, gesättigte, einfach oder mehrfach ungesättigte Carbonsäuren bzw. Alkohole bzw. deren Derivaten mit vorzugsweise 6 bis 24 Kohlenstoffatomen. Bevorzugt handelt es sich um lineare, unverzweigte Fettsäuren.

Unter "Tensid" werden im Zusammenhang dieser Erfindung amphiphile organische Substanzen mit jeweils mindestens einem hydrophilen und mindestens einem hydrophoben Rest mit grenzflächenaktiven Eigenschaften verstanden, die sich an die Grenzfläche zwischen zwei Flüssigkeiten, wie beispielsweise Öl und Wasser adsorbieren und die Fähigkeit besitzen, die Oberflächenspannung von Wasser zu verringern. In Lösung tendieren Tenside zur Selbstaggregation und bilden Strukturen wie bspw. Mizellen, lamellare Strukturen u.a. Im Zusammenhang mit dieser Erfindung werden als Tenside Verbindungen bezeichnet, die die Fähigkeit besitzen, die Oberflächenspannung von Wasser bei 20°C und bei einer Konzentration von 0.5 Gew.-% bezogen auf die Gesamtmenge der Zubereitung auf unter 45 mN/m zu verringern.

"Mindestens ein" wie hierin verwendet, bezieht sich auf 1 oder mehr, beispielsweise 1, 2, 3, 4, 5, 6, 7, 8, 9 oder mehr. Mindestens zwei bezieht sich auf 2 oder mehr, z.B. 3, 4, 5, 6, 7, 8, 9 oder mehr.

Unter Alkoxylaten werden Verbindungen mit Alkylenoxid-Monomeren verstanden, insbesondere mit den Monomeren EO = Ethylenoxid, PO = Propylenoxid oder BO (Butylenoxid) vorliegen und welche zu PEG- (Polyethylenglykol-), zu PPG-(Polypropylen- bzw. Polypropylen(glykol)-) oder PBG- (Polybutylen, bzw. Polybuylen(glykol))- haltigen Verbindungen führen. "PEG-haltige" Tenside/ Verbindungen sind Tenside/ Verbindungen die Polyethylenglykol enthalten, bzw. ethoxyliert vorliegen. Polyethylenglykol ist ein Oligomer oder Polymer (EO)n mit EO = Ethylenoxideinheiten und n der Anzahl der Ethylenoxideinheiten, mit n = 1 bis 500. Alkoxylate und deren Polymere aus pflanzlichen, regenerativen Quellen sind bevorzugt.

" Frei von" einer bestimmten Komponente im Rahmen dieser Erfindung bedeutet, dass keine Mengen der jeweiligen Komponente willentlich zu der Tensidzusammensetzung oder dem Mittel zugesetzt werden, besonders bevorzugt bedeutet dies, dass die Komponente jeweils in Mengen von kleiner 0.1 Gew.-%, ganz besonders bevorzugt von kleiner 0.01 Gew.-% jeweils bezogen auf das gesamte Mittel vorliegen. Am meisten bevorzugt ist unter "frei von" zu verstehen, dass die in Frage kommende Komponente in der Tensidzusammensetzung oder dem Mittel in nicht nachweisbaren Mengen enthalten ist.

Unter "Pflegemittel" wird im Rahmen der Erfindung ein Mittel verstanden, das die Attraktivität einer Person erhöhen kann, die Gesundheit von Zähnen, Haut und Haar erhalten kann, inklusive der Rasur, verschönern und pflegen kann. Das Pflegemittel umfasst auch die Behandlung von Materialien, z.B. Textilien. In den Begriff eingeschlossen ist die Tierpflege. Bevorzugte Ausführungsformen von Pflegeprodukten sind Konditionierungsmittel für Haare (z.B. Haarspülungen) oder Textilien (z.B. Weichspüler). Weitere bevorzugte Ausführungsformen sind Zahnpflegeprodukte.

Reinigungsmittel" beinhalten Wasch- und reinigungsmittel für die manuelle oder maschinelle Anwendung. In mindestens einer Ausführungsformen sind die Reinigungsmittle kosmetische Haut- und Haarreiniger. In mindestens einer weiteren Ausführungsformen sind die Reinigungsmittel Geschirrreiniger und Textilwaschmittel. In mindestens einer weiteren Ausführungsform ist die Kombination von Wasch- und Reinigungsmitteln mit Pflegemitteln, z.B. hautpflegende Reiniger, konditionierende Haut- oder Haar-Shampoos, weichspülende Waschmittel.

Die Mittel können durch einreiben, zudosieren, sprayen, schäumen und andere Methoden (z.B. salben, auflegen, etc.) direkt oder über ein Hilfsmittel wie beispielsweise ein Tuch, verdünnt oder unverdünnt, auf das zu reinigende Material, Haut, Haar etc. aufgebracht werden.

Unter Synergie wird verstanden, wenn die Kombination der Einzelkomponenten eine stärkere Wirkung betreffend eines Merkmals (Wirkung $(A+B)_c$ ) bei einer bestimmten Konzentration c zeigt als man auf Basis der Einzelkomponenten (Wirkung $(A)_c$ bzw. Wirkung $(B)_c$) durch deren Kombination erwartet könnte, d.h. der experimentelle Wert der Kombination A + B (Wirkung $(A+B)_c$) bei einer bestimmten Gesamtkonzentration c ist grösser als der erwartete, rechnerische Wert der Einzelkomponenten (hier Wirkung $(A)_c$ bzw. Wirkung $(B)_c$) bei jeweils der gleichen Gesamtkonzentration c, mit einem Gewichtsverhältnis von A: B = a : b. Im Rahmen dieser Erfindung wird Synergie verstanden als:

$$\text{Wirkung }(A+B)_c > [a^* \text{ Wirkung }(A)_c + b^* \text{ Wirkung }(B)_c]/(a+b)$$

Stoffe, die auch als Inhaltsstoffe von kosmetischen Mitteln dienen, werden nachfolgend gegebenenfalls gemäss der International Nomenclature Cosmetic Ingredient- (INCI-) Nomenklatur bezeichnet. Die INCI-Bezeichnungen sind dem "International Cosmetic Ingredient Dictionary and Handbook, 13th Edition (2010)" zu entnehmen. Herausgeber: The Personal Care Products Council.

Soweit nicht explizit anders angegeben, beziehen sich die angegeben Mengen in Gewichtsprozent (Gew.-%) auf das gesamte Mittel. Dabei beziehen sich die prozentualen Mengenangaben auf Aktivgehalte.

## Gegenstand

Gegenstand der Erfindung sind Zusammensetzungen enthaltend mindestens ein N-Acyl-glycamin der Formel (I) und bevorzugt zusätzlich enthaltend mindestens ein Glycolipid-Biotensid umfassend Rhamnolipide, Sophorolipide, Mannosylerythritollipide (MEL), Cellobioselipide und Trehaloselipide, sowie deren Kombinationen.

Weitere Gegenstände der Erfindung sind Verfahren zu der Herstellung der Zusammensetzungen sowie deren Verwendung als Reinigungs- oder Pflegemittel.

## N-Acylglycamine

N-Acylglycamine folgen der Formel (I):

(I) 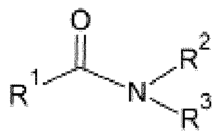

mit $R^1CO$ einem linearen oder verzweigten, gesättigten oder ungesättigten, Acylrest mit 6 bis 24 Kohlenstoffatomen, gegebenenfalls -OH oder - $OR^s$ substituiert mit $R^S$=-$SO_3H$ oder ein Salz desselben, bevorzugt ist $R^1CO$ einem Fettsäureacylrest,

mit $R^2$ einem linearen, verzweigten oder heterozyklischen Polyhydroxyalkylrest, bevorzugt ist in mindestens einer Ausführungsform $R^2$ = $-CH_2-(CHOH)_n-CH_2OH$ mit n= 3 oder bevorzugter n = 4 und in mindestens einer alternativen Ausführungsform ein heterozyklisches Anhydrid derselben, besonders bevorzugt stammt $R^2$ von Glucose, und mit $R^3$ einem Wasserstoffatom, einem Hydroxyalkylrest mit 1 bis 4 Kohlenstoffatomen oder Alkenylrest, bevorzugter einem Alkylrest ausgewählt aus $-(CH_2)_mCH_3$ mit m = 0,1, 2 oder 3, $-(CH_2)_oCH(CH_3)_2$- mit o = 0 oder 1, oder-$C(CH_3)_3$-, besonders bevorzugt ist $R^3$ = $-CH_3$, wobei die Varianten der unterschiedlichen Reste $R^1CO$, $R^2$ und $R^3$ beliebig und unabhängig voneinander kombiniert werden können. Besonders bevorzugt sind die Ausführungsformen enthaltend N-Methyl-N-Acyl-Glucamine der Formel (Ia) oder (Ib) oder deren Kombinationen, ganz besonders bevorzugt ist eine Zusammensetzung enthaltend Ausführungsform (Ia).

Der Polyhydroxyalkylrest $R^2$ kann sich ableiten von einem Monosaccharid wie Allose, Altrose, Arabinose, Fructose, Erythrose, Galactose, Glucose, Gulose, Idose, Lyxose, Mannose, Ribose, Talose, Threose, oder Xylose, oder deren Derivaten wie Desoxymonosaccharide, z.B. Desoxyribose oder Rhamnose, Carboxyzucker, wie z.B. Glucuronsäure oder Aminozucker z.B. Glucosamin oder Oligosacchariden wie Saccharose, Lactose, Trehalose, Maltose, Cellobiose, Gentiobiose, Raffinose oder technischen Abbauprodukten aus Polysacchariden z.B. Dextrine oder Stärkeabbauprodukten, z.B. Glucosesirup, abgeleitet werden. Die Saccharideinheiten liegen bevorzugt in den D-Konfigurationen vor. Aus den Sacchariden entstehen nach reduktiver Aminierung, die entsprechenden Aminoalditole, welche dann acyliert und gegebenenfalls anhydriert werden können.

N-Acylglycamine mit linearem Polyhydroxylrest

In mindestens einer erfinderisch bevorzugten Ausführungsform folgt $R^2$ der Formel $-CH_2-(CHOH)_n-CH_2OH$, abgeleitet von einer Aldopentose mit n= 3 oder noch bevorzugter von einer Aldohexose mit n = 4. Ganz besonders bevorzugt stammt dieser Polyhydroxyalkylrest $R^2$ von der Glucose, insbesondere von der D-Glucose. Ein erfinderisch bevorzugter Vertreter der resultierenden N-Alkyl-N-Acylglucamine ist in Formel (Ia) dargestellt mit Ra= $R^1$, insbesondere bevorzugt Ra = Fettsäurereste abgeleitet von einem C-18-Pflanzenöl vorzugsweise gemäss den als bevorzugt beschriebenen Verteilungen der Fettsäureacylreste; und $R^3$ = -CHs.

(Ia) 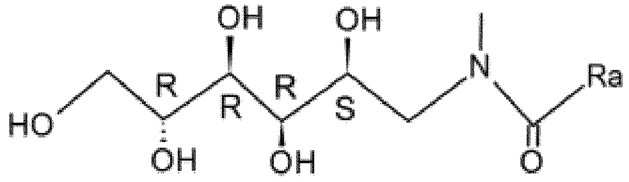

N-Acylglycamine mit hetercyclischen Polyhydroxylrest

Die N-Acylglycamine, bevorzugt das N-Methyl-N-acylglucamin der Formel (Ia) mit Ra= $R^1$, können duch Wasserabspaltung im Polyhydroxylrest in heterocyclische N-Acylglycamine der Formeln (Ib- Id) überführt werden. Der Rest $R^2$ der N-Acylglycamine der Formel (I) wird durch die Wasserabspaltung zum heterocyclisches Anhydrid des Hexanpentolrests. In mindestens einer Ausführungsform der Erfindung enthalten die Zusammensetzungen mindestens einen Vertreter dieser N-Acyl-N-methyl cyclic glucamide ausgewählt aus den Formeln (Ib), (Ic) und (Id) und insbesondere deren Kombinationen; ganz besonders bevorzugt ist mindestens eine Verbindung der Formel (Ib) gegebenenfalls in Kombination mit (Ic) und/ oder (Id) in der Zusammensetzung enthalten. In Formeln (Ib) bis (Id) ist Rb = R1, gemäss obiger Definition, in mindestens einer bevorzugten Ausführungsform sind N-Acylglycamine mit Rb= $-(CH_2)_8CH_3$ oder Rb=$-(CH_2)_6CH_3$ oder beide N-Acylglycamine enthalten.

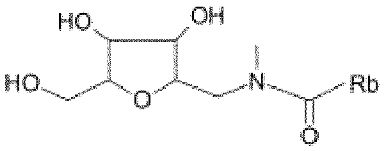

(Ib)

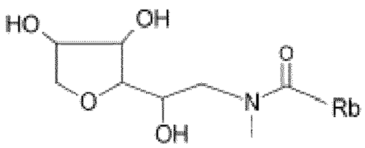

(Ic)

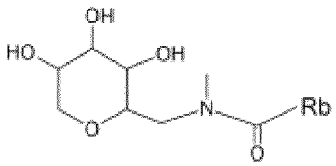

(Id)

In einer weiteren Ausführungsform sind sowohl N-Acylglycamine der Formel (Ia), als auch N-Acylglycamine der Formel (Ib), (Ic), und (Id) enthalten, insbesondere die Kombination der Vertreter der Formeln (Ia) und (Ib).

Der Rest $R^3$ umfasst insbesondere Wasserstoff, Methyl-, Ethyl-, n-Propyl, iso-Propyl, n-Butyl, iso-Butyl, sec.-Butyl, tert.-Butyl-Gruppen. Bevorzugt sind Methyl-, Ethyl-, N-Propyl und n-Butyl als teilkristalline Verbindungen, ganz besonders bevorzugt ist die Methylgruppe, insbesondere in der Form der N-Methyl-N-Acyl-Glucaminen.

Fettsäurereste:

$R^1CO$ stellt in mindestens einer bevorzugte Ausführungsform einen Acylrest dar mit R1 einer gesättigten oder ungesättigten Kohlenwasserstoffkette mit 7 oder 9 Kohlenstoffatomen, oder eine Mischung derselben. Ganz besonders bevorzugt folgend der der Struktur (Ib)

$R^1CO$ stellt in mindestens einer besonders bevorzugten Ausführungsform einen Fettsäureacylrest dar, wobei R1 eine gesättigte oder ungesättigte Kohlenwasserstoffketten mit 5 bis 23 Kohlenstoffen darstellt, gegebenenfalls hydroxysubstituiert, bevorzugt unverzweigt. Beispielhafte, nicht abschliessende Vertreter, hier am Beispiel $R^3$= Methyl, mit gesättigten Fettsäureacylresten sind Caproyl Methyl Glycamid, Capryloyl Methyl Glycamid, Lauroyl Methyl Glycamid, Myristoyl Methyl Glycamid, Palmitoyl Methyl Glycamid, Stearoyl Methyl Glycamid, Arachinoyl Methyl Glycamid, Behenoyl Methyl Glycamid, Vertreter mit ungesättigten Fettsäureacylresten sind Gadoleinoyl Methyl Glycamid,

Erucoyl Methyl Glycamid, Oleoyl Methyl Glycamid, Linoleoyl Methyl Glycamid, Linolenoyl Methyl Glycamid und deren Mischungen, bevorzugt ist in der Zusammensetzung mindestens ein N-Acylamin enthalten mit $R^1$ einem ein- oder mehrfach ungesättigte Kohlenwasserstoffatome mit 17 C-Atomen, d.h. Oleoyl Methyl Glycamid, Linoleoyl Methyl Glycamid, Linolenoyl Methyl Glycamid, besonders bevorzugt ist mindestens ein N-Acylglycamin, insbesondere mindestens ein N-Alkyl-N-Acylglycamin, jeweils mit $R^1CO$ = Oleoyl in der Zubereitung enthalten.

Bevorzugt werden mehrere N-Acylglyamine der Formel (I) in den Zusammensetzungen eingesetzt, die sich durch die Länge und/oder dem Sättigungsgrad der Fettsäureacylreste $R^1CO$ unterscheiden. In mindestens einer äusserst bevorzugten Ausführungsform, entspricht die Verteilung der Fettsäureacylreste $R^1CO$ in dem Gemisch von N-Acylglycaminen der Formel (Ia) der Verteilung der Fettsäureacylreste des ursprünglichen C-18-Pflanzenöls.Die Gemische sind aus ökologischen, ökonomischen sowie technischen, wie z.B. Löslichkeit, Gründen bevorzugt.

Ein "Gemisch" unterschiedlicher N-Acylglycamine der Formel (I), oder bevorzugt (Ia), wird als Produktgemisch von N-Acylglycaminen mit unterschiedlichen Acylresten erhalten.

Das bevorzugte Produktgemisch ("Gemisch") abgeleitet von einem C-18-Pflanzenöl aus N-Acylglycaminen mit unterschiedlichen Fettsäureresten resultiert aus der Umsetzung von Fettsäurederivaten eines C-18-Pflanzenöls (z.B. Fettsäureester wie Mono-, Di-, Triglyceride, Fettsäurealkylester, Fettsäuren, Fettsäurehalogenide, etc.) oder einem C-18-Pflanzenöl mit bevorzugt einem Glycamin oder besonders bevorzugt mit einem N-Alkylglycamin, wobei die Verteilung von Fettsäureacylresten in den Fettsäurederivaten oder dem Pflanzenöl mit 18 und mehr Kohlenstoffatomen über 60 Gew.-%, besonders bevorzugt über 72 Gew.-% und ganz besonders bevorzugt von über 77 Gew.-% liegt und wobei der Anteil an ungesättigten Fettsäureacylresten über 55 Gew.-%, vorzugsweise über 65 Gew.-% und besonders bevorzugt über 72 Gew.-% liegt, Gew.-% jeweils bezogen auf die Fettsäureacylreste der eingesetzten Fettsäurederivate oder des C-18-Pflanzenöls. Erfindungsgemäss bevorzugt ist das Gemisch aus N-Acylglycaminen resultierend aus der Umsetzung von Sonnenblumenöl, bzw. den Fettsäurederivaten des Sonnenblumenöls, inbesondere folgend der Formel (Ia) und benannt als Sunfloweroyl methylglucamid. Aus einem C-18-Pflanzenöl entsteht ein Gemisch; in der Zusammensetzung können mehrere Gemische enthalten sein, bspw. Sunfloweroyl methylglucamid als Gemisch 1 und Coco methylglucamid als Gemisch 2.

Bevorzugt resultiert in dem Produktgemisch ("Gemisch") abgeleitet von C-18-Pflanzenölen aus den N-Acylglycaminen eine Verteilung von Fettsäureacylresten mit 18 und mehr Kohlenstoffatomen über 60 Gew.-%, besonders bevorzugt über 72 Gew.-% und ganz besonders bevorzugt von über 77 Gew.-% liegt und einem Anteil an ungesättigten Fettsäureacylresten über 55 Gew.- %, vorzugsweise über 65 Gew.-% und besonders bevorzugt über 72 Gew.-% liegt, Gew.-% jeweils bezogen auf die Fettsäureacylreste des jeweiligen Gemischs an N-Acylglycaminen abgeleitet von C-18-Pflanzenölen.

Vorzugsweise betragen weiterhin, jeweils unabhängig voneinander, die Anteile der weiteren Fettsäureacylreste - in Bezug auf Kettenlängen und Sättigungsgraden- in dem N-Acylglycamin-Gemisch abgeleitet von C-18-Pflanzenölen denjenigen Anteilen für die entsprechenden Fettsäuren von C-18-Pflanzenölen, die vorstehend in der Erfindung als bevorzugt bezeichnet werden. Zum Beispiel, beträgt der Anteil an Fettsäuren im C-18-Pflanzenöl von 16 und weniger Kohlenstoffatomen unter 30 Gew.-%, bevorzugt unter 25 Gew.-% und besonders bevorzugt unter 17 Gew.-%; Gew.-% bezogen auf das C-18-Pflanzenöl. Analog ist bevorzugt, dass die Anteile der Fettsäureacylreste in dem N-Acylglycamin-Gemisch abgeleitet von C-18-Pflanzenölen von 16 und weniger Kohlenstoffatomen unter 30 Gew.-%, bevorzugt unter 25 Gew.-% und besonders bevorzugt unter 17 Gew.-%; Gew.-% jeweils bezogen auf die Fettsäureacylreste des jeweiligen Gemisch an N-Acylglycaminen abgeleitet von C-18-Pflanzenölen. Die Anteile der Fettsäureacylreste im N-Acylglycamin-Gemisch abgeleitet von C-18-Pflanzenölen aus entsprechen in Bezug auf Kohlenstoffkettenlängen und Sättigungsgraden besonders bevorzugt dem Fettsäureprofil des C-18-Pflanzenöls, wie vorstehend in der Erfindung als bevorzugt bezeichnet.

Die Herstellung von N-Alkyl-N-Acylglycaminen ist beispielsweise in EP 3071546 A1 und den zitierten Druckschriften beschrieben.Werden z.B. in ein und derselben Reaktion zwei Pflanzenöle oder Fettsäuren aus zwei verschiedenen Pflanzenölquellen eingesetzt, werden im Rahmen dieser Erfindung zwei Gemische an N-Acylglycaminen als Produktgemische erhalten.

Gemische aus destillierten Fettsäurederivate werden häufig mit den chemischen Namen der Fettsäureacylreste benannt, z.B. Lauroyl/Myristoyl Methyl Glucamide. Erfinderisch bevorzugt sind die Gemische, benannt nach dem Pflanzenöl, von dem sie abgeleitet sind, bspw. Cocoyl methylglucamid aus Kokosöl oder insbesondere Sunfloweroyl methylglucamid aus dem erfinderisch bevorzugten Sonnenblumenöl.

Anteile der Fettsäurereste in der Zusammensetzung:

Bevorzugt enthält die Zusammensetzung mehrere N-Acylglycaminen aus mindestens einem Gemisch, wobei sich mindestens 2 oder mehrere der N-Acylglycamine durch deren Fettsäureacylreste $R^1CO$ unterscheiden. Besonders bevorzugt betragen die Fettsäureacylanteile der N-Acylglycamine in der gesamten Zusammensetzung wie folgt.

Besonders bevorzugt enthält die Zusammensetzung ein oder mehrere N-Acylglycamine mit einfach und/oder mehrfach ungesättigten Fettsäureacylreste mit mindestens 18 Kohlenstoffatomen, wie sie z.B. aus Ölsäure, Linolsäure oder Linolensäure im Falle von $R^1$ mit 17 Kohlenstoffatomen erhalten werden.

Bevorzugt beträgt in der Zusammensetzung der Anteil an ein- oder mehrfach ungesättigten Fettsäureacylresten der N-Acylglycamine mit mindestens 18 Kohlenstoffatomen dabei > 8 Gew.-%, bevorzugter über 20 Gew.-%, besonders bevorzugt > 60 Gew.-% und äusserst bevorzugt über 72 Gew.-%, jeweils bezogen auf das Gesamtgewicht der Fettsäureacylreste $R^1CO$ der N-Acylglycamine in der Zusammensetzung.

Besonders bevorzugt enthalten die Zusammensetzungen N-Alkyl-N-Oleoylglycamin. Ganz besonders bevorzugt sind Zusammensetzungen bei denen zusätzlich zu N-Alkyl-oleoylglycamid mindestens ein N-Alkyl-Acylglycamin enthalten ist, das einen Fettsäureacylrest mit einer mehrfach ungesättigte Kohlenwasserstoffkette mit 18 C-Atomen oder mehr enthält (z.B. Linoleoyl Methyl Glycamid, Linolenoyl Methyl Glycamid). Hierbei sind jeweils die cis- und transständigen Formen möglich, erfindungsgemäss bevorzugt sind die cis-Konfigurationen der Fettsäurereste.

Hierbei gilt insbesondere bevorzugt, dass die Zusammensetzung einen Anteil an mehrfach ungesättigten Fettsäureacylresten mit 18 oder mehr Kohlenstoffatomen > 8 Gew.-% beträgt, Gew.-% jeweils bezogen auf die Fettsäureacylreste $R^1CO$ in dem jeweiligen Gemisch von N-Acyl-N-Methylglucaminen (la).

Bevorzugt enthält die Zusammensetzung einen oder mehreren N-Acylglycamine mit einen Anteil an gesättigten oder ungesättigten Fettsäureacylresten mit 20 oder mehr Kohlenstoffatomen, wobei deren Anteil bis zu 96 Gew.-% betragen kann, > 0.01 Gew.-% und bevorzugter > 0.05 Gew.-% und besonders bevorzugt > 0.1 Gew-% und ganz bevorzugt >= 0.2 Gew.-% und äusserst bevorzugt >= 0.5 Gew.-%, jeweils bezogen auf das Gesamtgewicht der Fettsäureacylreste $R^1CO$ der N-Acylglycamine in der Zusammensetzung.

Der Anteil an gesättigten Fettsäureacylreste, insbesondere mit 16 oder 18 Kohlenstoffatomen muss gering gehalten werden um die gewünschten Eigenschaften z.B. in Bezug auf Löslichkeit, Schmelzpunkt, Emulgierfähigkeit, Gelbildung u.s.w. zu erreichen. Die bevorzugten Zusammensetzungen enthalten neben den N-Acylglycamine mit ein- oder mehrfach ungesättigten Fettsäureacylreste mit mindestens 18 Kohlenstoffatomen keine oder nur einen geringeren Gewichtsanteil an N-Acylglycaminen mit gesättigten Fettsäureacylresten mit 16 oder 18 Kohlenstoffatomen. Bevorzugt enthält die Zusammensetzung einen oder mehreren N-Acylglycamine der Formel (I) mit gesättigten Fettsäureacylresten mit 16 oder 18 Kohlenstoffatomen ist bevorzugt < 40 Gew.-%, bevorzugter < 30 Gew.-%, besonders bevorzugt < 25 Gew.-%, ganz besonders bevorzugt $\leq$ 20 Gew.-%, und äusserst bevorzugt $\leq$ 15 Gew.-% jeweils bezogen auf das Gesamtgewicht der Fettsäureacylreste $R^1CO$ der N-Acylglycamine in der Zusammensetzung.

Bevorzugt sind weiterhin auch Ausführungsformen der Zusammensetzung, bei denen der Anteil der ein oder mehreren N-Acylglycamine mit gesättigten Fettsäureacylresten mit 16 Kohlenstoffatomenoder weniger < 30 Gew.-% ist, bevorzugt unter 25 Gew.-% und besonders bevorzugt unter 17 Gew.-%; Gew.-% jeweils bezogen auf das Gesamtgewicht der Fettsäureacylreste $R^1CO$ der N-Acylglycamine in der Zusammensetzung.

Bevorzugt sind weiterhin auch Ausführungsformen der Zusammensetzung, bei denen der Anteil der ein oder mehreren N-Acylglycamine mit gesättigten Fettsäureacylresten mit 12 Kohlenstoffatomenoder weniger < 30 Gew.-% ist, bevorzugt unter 27 Gew.-% und besonders bevorzugt unter 17 Gew.-%; Gew.-% jeweils bezogen auf das Gesamtgewicht der Fettsäureacylreste $R^1CO$ der N-Acylglycamine in der Zusammensetzung.

Besonders bevorzugt ist der Anteil an dem ein oder mehreren N-Acylglycaminen mit Fettsäureacylresten mit 10 Kohlenstoffatomen oder weniger < 20 Gew.-%, besonderes bevorzugt < 10, insbesondere bevorzugt < 5 Gew.-%, jeweils bezogen auf das Gesamtgewicht der Fettsäureacylreste $R^1CO$ der N-Acylglycamine in der Zusammensetzung.

In mindestens einer besonders bevorzugten Ausführungsformen der Zusammensetzung enthält N-Acylglycamine, bei denen der Anteil der ein- oder mehrfach ungesättigten Fettsäureacylreste mit mindestens 18 Kohlenstoffatomen > 60 Gew.-%, und äusserst bevorzugt über 72 Gew.-% beträgt und gleichzeitig der Anteil von 16 Kohlenstoffatomen und weniger, < 30 Gew.-%, bevorzugt unter 25 Gew.-% und besonders bevorzugt unter 17 Gew.-% liegt; Gew.-% jeweils bezogen auf das Gesamtgewicht der Fettsäureacylreste $R^1CO$ der N-Acylglycamine in der Zusammensetzung.

Besonders bevorzugt sind Ausführungsformen der Zusammensetzung, bei denen der Anteil der ein- oder mehrfach ungesättigten Fettsäureacylreste mit mindestens 18 Kohlenstoffatomen der ein oder mehreren N-Acylglycamine > 60 Gew.-%, und äusserst bevorzugt über 72 Gew.-% beträgt und gleichzeitig der Anteil von 12 Kohlenstoffatomen und weniger < 30 Gew.- %, bevorzugt unter 27 Gew.-% und besonders bevorzugt unter 17 Gew.-% liegt; Gew.-% jeweils bezogen auf das Gesamtgewicht der Fettsäureacylreste $R^1CO$ der N-Acylglycamine in der Zusammensetzung.

Erfindungsgemäss bevorzugt ist eine Zusammensetzung, die ein oder mehrere N-Acylglycamin-Gemische abgeleitet von C-18-Pflanzenölen enthält. Dieses mindestens eine Gemisch abgeleitet von einem C-18-Pflanzenöl kann erfindungsgemäss mit mindestens einem weiteren Gemisch aus N-Acylglycaminen in der Zusammensetzung vorliegen, beispielsweise mit einem Gemisch an N-Acylglycaminen mit Fettsäureacylresten aus Kokosöl.

Der Anteil an N-Acylglycaminen mit Fettsäureacylresten stammend aus einem oder mehreren C-18-Pflanzenölen kann bis 100 Gew.-% bezogen auf den Gesamtanteil an N-Acylglycaminen in der Zusammensetzung betragen. Bevorzugt beträgt dieser Anteil $\geq$ 10 Gew.-%, bevorzugter $\geq$ 50 Gew.-%, besonders bevorzugt $\geq$ 70 Gew.-% und ganz besonders bevorzugt $\geq$ 85 Gew.- %, jeweils bezogen auf das Gesamtgewicht der N-Acylglycamine in der Zubereitung.

In der ganz besonders bevorzugten Ausführungsvariante dieser Erfindung beträgt dieser Anteil 100 Gew.-%, d.h. die Fettsäureacylreste der in der Zubereitung enthaltenen N-Acylglycamine sind vollständig von einem oder mehreren C-18-Pflanzenölen abgeleitet. Eine ganz besonders bevorzugte Ausführungsform der Erfindung ist, wenn in der Zusammensetzung ein oder mehrere "C-18-Pflanze"-oyl Alkyl Glycamide, z.B. Sunfloweroyl Methyl Glucamide, als alleinige N-Acylglycamine vorliegen.

In mindestens einer Ausführungsform sind erfindungsgemäss äusserst bevorzugt die N-Alkyl-N-Methylglycamine der Glucose, insbesondere D-Glucose, resultierend in den entsprechenden N-Acyl-N-Methylglucaminen gemäss der Formel (Ia) mit Ra = R1.

(Ia) 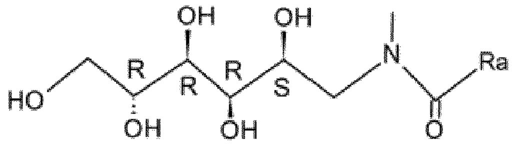

Sowohl aus Nachhaltigkeitsbetrachtungen als auch für die vorteilhaften Eigenschaften der Zusammensetzung ist der Anteil an gesättigten Verbindungen mit maximal 18 Kohlenstoffatomen von max. 40 Gew.-% wesentlich.

In mindestens einer äusserst bevorzugten Ausführungsform, entspricht die Verteilung der Fettsäureacylreste RaCO in dem Gemisch von N-Acylglycaminen der Formel (Ia) der Verteilung der Fettsäureacylreste des C-18-Pflanzenöls.

Bevorzugt ist das Gemisch an N-Methyl-N-Acylglucaminen der Formel (Ia) mit unterschiedlichen Fettsäureacylresten RaCO aus mindestens einem C-18-Pflanzenöl in der Zusammensetzung enthalten, wobei die Verteilung der Fettsäureacylresten RaCO mit 18 und mehr Kohlenstoffatomen über 60 Gew.-%, besonders bevorzugt über 72 Gew.-% und ganz besonders bevorzugt von über 77 Gew.-% liegt und der Anteil an ein- oder mehrfach ungesättigten Fettsäureacylresten über 55 Gew.-%, vorzugsweise über 65 Gew.-% und besonders bevorzugt über 72 Gew.-% und der Anteil an gesättigten Fettsäureacylresten mit 16 oder 18 Kohlenstoffatomen < 40 Gew.-%, bevorzugter < 25 Gew.-%, besonders bevorzugt ≤ 15 Gew.-%, und ganz besonders bevorzugt zwischen 6 und 15 Gew.-% liegt; Gew.-% bezogen auf die Fettsäureacylreste RaCO des Gemischs an N-Methyl-N-Acylglucaminen der Formel (Ia);
Besonders bevorzugt enthält eine Ausführungsform der Zusammensetzung mindestens ein Gemisch an N-Acylglycaminen der Formel (Ia) abgeleitet von mindestens einem C-18-Pflanzenöl mit Anteilen an ein- und mehrfach ungesättigten Fettsäureacylresten mit 18 Kohlenstoffatomen von ≥. 80 Gew.-% und mit Anteilen von gesättigten Fettsäureacylresten mit 18 oder weniger Kohlenstoffatomen ≤ 20 Gew.-%, bevorzugt ≤ 15 Gew.-% von Palmitoyl- und Stearoylresten; ganz besonders bevorzugt beträgt dabei der Anteil an einfach ungesättigten Fettsäureacylresten mit 18 Kohlenstoffatomen (Oleoylreste) mind. 75 Gew.-%, Gew.-% jeweils bezogen auf die Fettsäureacylreste RaCO des Gemischs an N-Methyl-N-Acylglucaminen der Formel (Ia).

Aussert bevorzugt ist das Gemisch an N-Methyl-N-Acylglucamine mit den Fettsäureacylresten RaCO aus Sonnenblumenöl, Rapsöl oder Olivenöl in der Zusammensetzung enthalten, insbesondere das Gemisch an N-Methyl-N-Acylglucaminen aus Sonnenblumenöl mit der Bezeichnung Sunfloweroyl methylglucamid.

Prozessbedingt kann das bevorzugt eingesetzte Gemisch an N-Methyl-N-acylglucamine der Formel (Ia) untergeordnet weitere Komponenten enthalten, z.B. Glycerin und dessen Derivate, Amine (z.B. N-Methylglycamine,) Meglumine, D-Glucitol, Propan-1,2-diol, Fettsäuren, z.B. ungesättigten C-18-Fettsäuren, N-Methyl-N-acylglucamine mit von Formel (Ia) verschiedenen Kohlenwasserstoffketten, z.B. statt Ra' = C5-C13-Alkyl, C15-Alkenyl, C19-23-Alkyl, C19-23-Alkenyl (Alkenyl einfach oder mehrfach ungesättigt) und Wasser. Typischerweise können diese bevorzugten Gemische an N-Methyl-N-acylglucaminen ≤ 12 Gew.-% Glycerin, ≤ 7 Gew.-% N-Methylglucamine, ≤ 23 Gew.-% Wasser enthalten, bezogen auf den Gesamtgehalt an N-Methyl-N-acylglucaminen. In flüssigen Handelsformen kann desweiteren Propylenglykol enthalten sein.

**Biotenside**

Als Biotenside werden allgemein Tenside biologischen Ursprungs bezeichnet. Die Biotenside werden nicht durch chemische Umsetzung wie synthetische Glycolipide z.B. Alkylpolyglycoside (APG) gebildet. Biotenside können in lebenden Organismen vorkommen, oder entstehen bei der Kultivierung diverser Mikroorganismen wie z.B. Pilze, Hefen, Algen, Viren oder Bakterien oder Enzymen. Biotenside oder deren Ausgangsstoffe können jedoch zusätzlich chemisch modifiziert werden.

Glycolipid- Biotenside können unmittelbar durch die Verwendung von natürlichen Rohstoffen durch mikrobiologische Prozesse biotechnologisch bzw. fermentativ gewonnen werden, während die Herstellung von synthetischen Glycolipide (z.B. Alkylglycoside) durch chemische Schritte, wie bspw. eine katalysierte Glycosylierung, erfolgt. Die Struktur der Glycolipid-Biotenside sowie die Kettenlängen des hydrophoben Teils variiertje nach verwendeten Mikroorganismus bzw. Substrat.

Als Substrate für Glycolipid- Biotenside eignen sich die unterschiedlichsten, literaturbekannten Kohlenstoffquellen, wie z.B. Pflanzenöle und den daraus erhaltenen Glyceriden oder Fettsäuremethylestern, Fettsäuren, Fettalkohole, Fettsäuremethylester oder -ethylester, Kohlenhydrate, z.B. Cellulose, Glucose, Stärke, C4-Quellen wie Succinate, Butan, Buttersäure, C1-Quellen wie $CO_2$, CO oder Methan, öl- und/oder kohlenhydrathaltige Abwässer aus Raffinerien oder der Lebensmittelindustrie und auch Mischungen derselben.

Bevorzugte Mikroorganismen zur Herstellung der Biotenside sind Bacillus, Candida, Pseudomonas, Trichosporan und/oder Pseudozyma Stämme und weitere wie in einschlägiger Literatur beschrieben, insbesondere *Arthrobacter spec.,* Bacillus licheniformis, Bacillus subtilis, Burkholderia, z.B. Burkholderia pseudomallei, Burkholderia plantarii, Candida apicola, Candida antarctica, Candida batistae, Candida bogoriensis, Candida bombicola, Candida sp., Cryptococcus humicola, *Gordonia, Mycobacterium* tuberculosis, *Nocardia* cornynebacterium, Pichia anomala, Pseudomonas, z.B. Pseudomonas aeruginosa, Pseudomonas chloroaphis, Pseudomonas putida, Pseudozyma aphidis (MEL), Pseudomonas sp, Pseudozyma antarctica (MEL), Pseudozyma parantarctica, Pseudozyma fusiformata, *Rhodococcus,* Rhodococcus erythropolis, Rhodotorula bogoriensis, Sphingomonas sp. NM05, Starmerella bombicola, Trichosporon cutaneum, Trichosporan loubieri; Torulopsis magnoliae, Torulopsis bombicola, Tsukamurella spec., Yarrowia alipolytica, Yarrowia lipolytica, Ustilago maydis (MEL), Wickerhamiella domercqiae Y2A.

Die Glycolipid-Biotenside können z. B. wie in EP2735605 (Evonik), US20130130319 (Evonik), US20160272667 (Logos), US20190309248 (Logos), EP 0 499 434, US 7,985 722, WO 03/006146, JP 60-183032, DE 19648439, DE 19600743, JP 01-304034, CN 1337439, JP 2006- 274233, KR 2004033376, JP 2006-083238, JP 2006-070231 , WO 03/002700, FR 2740779, DE 2939519, US 7,556,654, FR 2855752, EP 1445302, JP 2008-062179 und JP 2007-181789, US 2016/0272667, Mannosylerythritol: WO 2004/020647, JP 20042544595, WO 2020006194, WO 2021127339, WO2017220957 oder den dort zitierten Schriften hergestellt bzw. isoliert werden.

**Rhamnolipide**

Unter dem Begriff Rhamnolipid im Zusammenhang mit der vorliegenden Erfindung werden insbesondere Verbindungen der allgemeinen Formel (II) oder deren Salze verstanden

(II) 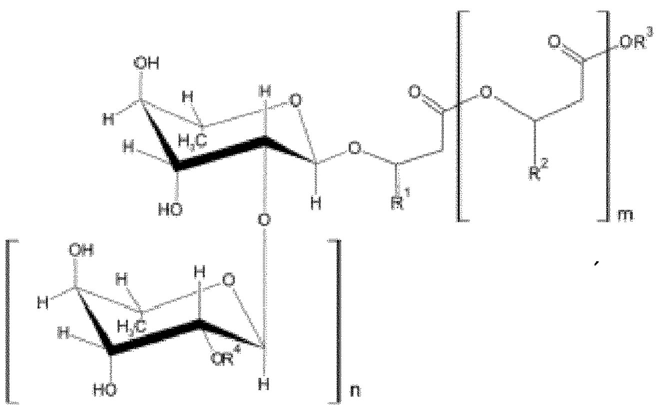 ,

wobei m = 2, 1 oder 0, insbesondere 1 oder 0, n = 1 oder 0, insbesondere 1, $R^{1'}$ und $R^{2'}$ = unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter Kohlenwasserstoffrest, bevorzugt ausgewählt aus der Gruppe bestehend aus Pentenyl, Heptenyl, Nonenyl, Undecenyl und Tridecenyl und $-(CH_2)_o-CH_3$ mit o = 1 bis 23, bevorzugt 4 bis 12, ganz besonders bevorzugt e = 6. $R^{3'}$ = H, $CH_3$, aliphatischer Rest mit 3 bis 32 Kohlenstoffatomen oder Kation, wie zum Beispiel Alkalimetalle Li, Na, oder Kalium oder Erdalkalimetalle wie Mg oder Ca, oder Übergangsmetalle wie Mn, Fe, Cu oder Zn, Ammonium oder organische Ammoniumverbindung, insbesondere Alkalikation, bevorzugt H oder Kation, $R^4$ = H oder die Gruppe $CH_3(CH_2)_aCH=CH-CO$, mit a als Zahlen zwischen 4 und 10, bevorzugt H.

Im Fall von $R^{3'}$ = mehrwertigen Kationen, können mehrere Rhamnolipideinheiten an das Metall assoziert sein.

Unter dem Begriff "di-Rhamnolipid" im Zusammenhang mit der vorliegenden Erfindung werden Verbindungen der allgemeinen Formel (II) oder deren Salze verstanden, bei denen n =1, d.h. mit zwei Rhamnoseresten. Unter dem Begriff "mono-Rhamnolipid" im Zusammenhang mit der vorliegenden Erfindung werden Verbindungen der allgemeinen Formel (II) oder deren Salze verstanden, bei denen n =0, d.h. mit nur einem Rhamnoserest. Je nach Produktionsprozess und verwendetem Bakterienstamm können Variationen auftreten betreffend der Anteile an mono- und di-Rhamnolipiden.

Eine Ausführungsform der Erfindung enthält mono-Rhamnolipide. Diese können wahlweise auch als reine mono-Rhamnolipide, ohne Anteile an di-Rhamnolipiden, eingesetzt werden. Bevorzugt werden die mono-Rhamnolipide Rha-$C_{8-12}$-$C_{8-12}$, der gängigen Bezeichnungsweise für Rhamnolipide folgend, d.h. in diesem Fall beträgt die Anzahl der Kohlenstoffatome der Fragmente - $CHR^{1'}$-$CH_2$-C(O)- bzw. - $CHR^{2'}$-$CH_2$-C(O)- in der Formel (II) 8 bis 12 Kohlenstoffatome. Insbesondere bevorzugt ist Rha-C10-C10 mit jeweils 10 Kohlenstoffatomen in dem Fragment bzw. n=0, $R^{1'}$= $R^{2'}$=linearer Kohlenwasserstoff mit $C_7H_{15}$ und $R^{3'}$=H, IUPAC-Name: (R)-3-(((R)-3-(((2R,3R,4R,5R,6S)-3,4,5-trihydroxy-6-methyltetrahydro-2H-pyran-2-yl)oxy)decanoyl)oxy)decanoic acid.

In einer bevorzugten Ausführungsform werden Di-Rhamnolipide oder deren Gemische mit mono-Rhamnolipiden eingesetzt, besonders bevorzugt mit mind. 50 Gew.-% di-Rhamnolipid, bevorzugter mind. 60 Gew.-% di-Rhamnolipid, noch bevorzugter mind. 70 Gew.-% di-Rhamnolipid und am meisten bevorzugt mind. 80 Gew.-% di-Rhamnolipid.

Bevorzugt sind die di-Rhamnolipide Rha-Rha-$C_{8-12}$-$C_{8-12}$ in dieser Ausführungsform anwesend, der gängigen Bezeichnungsweise für Rhamnolipide folgend, d.h. in diesem Fall beträgt die Anzahl der Kohlenstoffatome der Fragmente - $CHR^{1'}$-$CH_2$-C(O)- bzw. -$CHR^{2'}$-$CH_2$-C(O)- in der Formel (II) 8 bis 12 Kohlenstoffatome, d.h. o = 4-8, n=1. Insbesondere bevorzugt ist Rha-Rha-C10-C10 mit jeweils 10 Kohlenstoffatomen in dem Fragment bzw. n=1, $R^{1'}$= $R^{2'}$=linearer Kohlenwasserstoff mit $C_7H_{15}$ bzw. 0 = 6 und $R^{3'}$=H, IUPAC-Name: (R)-3-{(R)-3-[2-O-($\alpha$-L-rhamnopyranosyl)-a-L-rhamnopyranosyl]oxydecanoyl}oxydecanoate or the synonym name: (R)-3-((R)-3-((2R,3R,4R,5R,6S)-4,5-dihydroxy-6-methyl-3-((2S,3R,4R,5R,6S)-3,4,5-trihydroxy-6-methyltetrahydro-2H-pyran-2-yloxy)tetrahydro-2H-pyran-2-yloxy)decanoyloxy)decanoic acid. Besonders bevorzugt sind Rha-C10-C10 und Rha-Rha-C10-C10 in dem Mittel anwesend.

Zudem können aus dem Produktionsprozess der Rhamnolipide der Formel (II) zu einem geringen Masse Nebenkomponenten z.B. mit unterschiedlichen Kohlenwasserstoffkettenlängen, Prozesshilfsmittel z.B. organische Lösungsmittel, oder Edukte anwesend sein.

In einer speziellen, bevorzugte Ausführungsform ist eine Rhamnolipidmischung folgender Zusammensetzung enthalten: 28-40 Gew.-% Rha-C10-C10, 42-60% Rha-Rha-C10-C10, 0-12 Gew.-% andere Monorhamnolipide und 0-18 Gew.-% andere Dirhamnolipide, wobei diese anderen Rhamnolipide in $R^{1'}$ und/ oder $R^{2'}$ der Formel (II) den Wert von o von 4 oder 8 aufweisen, und im Fall von o= 8 ungesättigt sein können.

Rhamnolipide sind über Pseudomonas und Burkholderia, beides Wildtypen zugänglich. Desweiteren werden Rhamnolipide über rekombinante Zellen hergestellt (z.B. via Gene von P.aeruginosa in P.putida). Rhamnolipide sind Stoffwechselprodukte dieser Mikroorganismen, von denen vor allem Pseudomonas aeruginosa zur Produktion verwendet wird. Es können Lipide (z.B. Pflanzenöle), Glycerin, Kohlenhydrate (z.B. Zucker) oder deren Kombinationen als Substrat eingesetzt werden, aus Nachhaltigkeitsgründen bevorzugt sind jeweils Rohstoffe aus Pflanzen, die in der gemässigten Klimazone wie Zentraleuropa wachsen oder als Abfall-bzw. Nebenkomponenten anfallen, z.B. im Fall von Kohlenhydraten oder Zuckern werde bspw. Mais, Kartoffel, Stroh, Getreide, u.a. bevorzugt oder im Fall von Pflanzenölen oder Glycerin werden C-18-Pflanzen als Rohstoffe bevorzugt oder auch Rohstoffe die einem Upcycling-Prozess unterworfen werden, z.B. Abfallöle oder -kohlenhydrate.

## Sophorolipide

Unter dem Begriff Sophorolipid im Zusammenhang mit der vorliegenden Erfindung werden insbesondere Verbindungen der offenen Form oder Säureform, der Ring- oder Laktonform, oder deren Gemische verstanden, vorzugsweise Formel (III) folgend

(III)

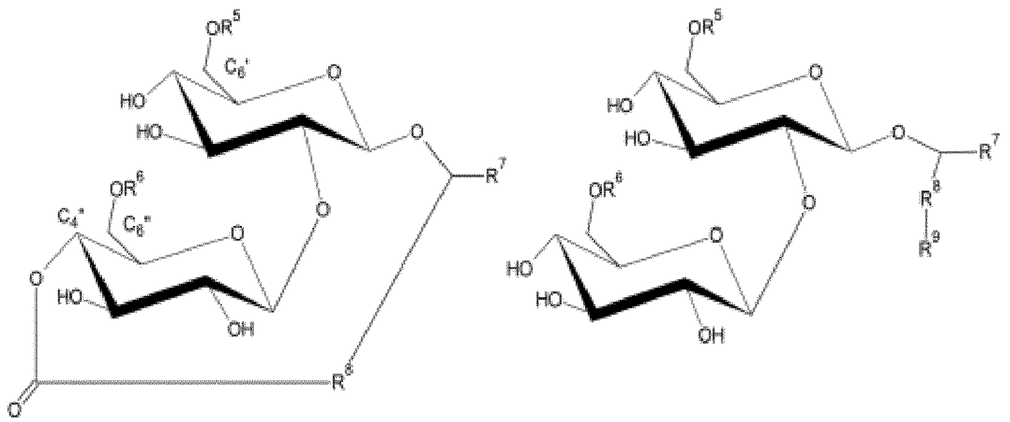

Ringform        Offene Form

Wobei

- $R^5$ und $R^6$ unabhängig voneinander H oder Acetylgruppen sind
- $R^7$ H oder eine reine gesättigte oder ungesättigte, hydroxylierte oder nicht-hydroxylierte Kohlenwasserstoffkette mit 1-9 Kohlenstoffatomen ist, bevorzugt $CH_3$
- $R^8$ eine gesättigte oder ungesättigte, hydroxylierte oder nicht-hydroxylierte, lineare oder verzweigte Kohlenwasserstoffkette mit 1-22 Kohlenstoffatomen ist, bevorzugt besteht $R^8$ aus einer gesättigten Kohlenwasserstoffkette aus 11bis 22 Kohlenstoffatomen oder aus einer einfach oder zweifach ungesättigten Kohlenwasserstoffkette aus 13 bis 22 Kohlenstoffatomen, besonders bevorzugt aus einer einfachen oder zweifach ungesättigten Kohlenwasserstoffkette aus 15 oder 16 Kohlenstoffatomen stammend aus Rapsöl als Substrat.
- $R^9$ ist COOH oder ein kationisches Salz desselben, oder $COO(CH_2)_mCH_3$ mit m zwischen 0 und 3,

In der Laktonkonfiguration kann die Laktonbildung der Carboxylgruppe mit Hydroxylgruppe an C4", wie hier exemplarisch in Formel (V) dargestellt, oder alternativ an C6" oder C6' erfolgen, an der C4" Position befindet sich dann eine Hydroxylgruppe.

Lakton- oder Säureform können jeweils alleine oder als Mischung erfindungsgemäss eingesetzt werden. Jede Form wiederum kann aus einer Mischung mit unterschiedlichem Acetylierungsgrad, und unterschiedlichen Kohlenstoffketten mit unterschiedlichem Sättigungsgrad bestehen. Bevorzugt werden Mischungen beider Formen eingesetzt, besonders bevorzugt liegt der Anteil der Laktonform ≤ 67 Gew.-%, noch bevorzugter ≤ 55 Gew.-%, äusserst bevorzugt ≤ 37 Gew.-% und am meisten bevorzugt ≤ 27 Gew.-%, bezogen auf das Trockengewicht der eingesetzten Sophorolipidmischung. Zusätzlich können die Sophorolipide Verunreinigungen enthalten, wie Fettalkohole, Fettsäureester, Fettsäuren, Triglyceride oder Öle, Zucker, besonders Glucose, Sophorose, oder organische Säuren, die typischerweise ≤ 20 Gew.-%, bevorzugt ≤ 8 Gew.-% und besonders bevorzugt ≤ 5 Gew.-% liegen, bezogen auf das Trockengewicht der eingesetzten Sophorolipidmischung

Durch Verwendung unterschiedlicher Substrate kann das Biotensid strukturell modifiziert werden, z.B. kann das Verhältnis Laktonform zu freier Säure durch das Substrat gesteuert werden. Erfindungsgemäss können unterschiedlichste Substrate verwendet werden, wie in C. Mulligan, Biosurfactants: Research Trends and Application, CRC Press 2014, S. 112. Beispiele für bevorzugten Substrate für Sophorolipide aus der Literatur sind: Maisöl (EP 0282942), Öl von C22 Fettsäuren (KR20100022289), Olivenöl (ES 2018637, CN 1431312), Rapsöl oder -ester (CN 102250790, US2008032383), Sonnenblumen- oder Rapsfettsäureester (DE4319540, FR 2692593) oder Abfallöle bzw. -fette (JP 2004254595, JP 2007252279, CN 101845468, CN 101948786).

Sophorolipide sind durch eine Vielzahl unterschiedlicher dem Fachmann bekannten Organismen wie Candida bombicola, Starmerella, e.g. S. bombicola, oder Wickerhamiella zugänglich.

**Mannosylerythritollipide**

Unter dem Begriff Mannosylerythritollipide (MEL) im Zusammenhang mit der vorliegenden Erfindung werden insbesondere Verbindungen der allgemeinen Formel (IV) oder deren Salze verstanden,

(IV)

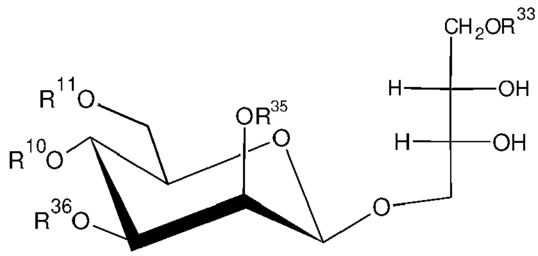

wobei

$R^{10}$ und $R^{11}$ unabhängig voneinander -H oder $-COCH_3$
$R^{33}$ -H oder $-COR^{34}$, bevorzugt H
$R^{35}$ -H oder $-COR^{12}$, bevorzugt $-COR^{12}$
$R^{36}$ -H oder $-COR^{13}$, bevorzugt $-COR^{13}$
$R^{12}$, $R^{13}$ und $R^{34}$ unabhängig voneinander, lineare oder verzweigte $C_1$ bis $C_{23}$, vorzugsweise $C_1$ bis $C_{17}$, und besonders bevorzugt $C_7$ bis $C_{17}$Alkylgruppen; oder lineare oder verzweigte $C_2$ bis $C_{23}$, bevorzugt $C_2$ bis $C_{17}$, und besonders bevorzugt $C_7$ bis $C_{17}$ Alkenylgruppen; oder lineare oder verzweigte $C_5$ bis $C_{23}$, vorzugsweise $C_5$ bis $C_{17}$, und besonders bevorzugt $C_7$ bis $C_{15}$ Alkadienylgruppen; oder lineare oder verzweigte $C_8$ bis $C_{19}$, bevorzugt $C_8$ bis $C_{17}$, und besonders bevorzugt $C_8$ bis $C_{15}$ Alkatrienylgruppen.

$R^{12}CO$-, $-COR^{34}$ bzw. $R^{13}CO$- stellen Acylgruppen dar, bevorzugt sind die diacylierten MEL-Varianten mit $R^{12}CO$-, $R^{13}CO$- und $R^{33}$ =-H (Vlb), Triacyliertes MEL mit den Acylgruppen $R^{12}CO$-, $-COR^{34}$ und $R^{13}CO$- und monoacyliertes MEL z.B. mit $R^{36}$ = - $COR^{13}$, $R^{35}$ und $R^{11}$ =-H.

Die hydrophilen Teile sind bevorzugt β-D-mannopyranosyl-(1->4)-D-meso-erythritole als Zuckerreste.

Bevorzugt liegt ein Gemisch an Mannosylerythritollipiden mit unterschiedlichen Kettenlängen und gegebenenfalls Sättigungsgraden der Acylreste vor, insbesondere von $R^{12}CO$- bzw. $R^{13}CO$-,

Weiterhin sind die beiden Diastereomere S-MEL (4-O-β-D-mannopyranosyl-(2S,3R)-erythritol), und R-MEL (4-O-β-D-mannopyranosyl-(2R,3S)-erythritol) möglich, bevorzugt ist die meso-Erythritol-Form S-MEL (vgl VI b).

Besonders bevorzugte Strukturen folgen der Formel IV b

(IV b)

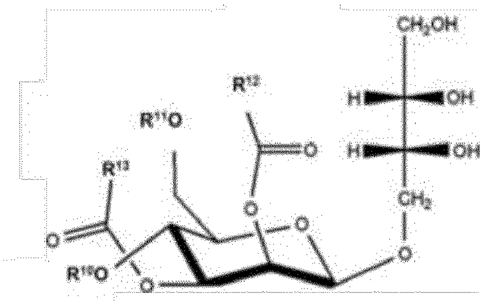

Strukturelle Varianten der Formel (IVb) sind Variationen in den Positionen C4'und C6' des Mannosylrestes: MEL-A ($R^{10}$ = $R^{11}$ = Acetyl $-COCH_3$), MEL-B ($R^{11}$ = $-COCH_3$, $R^{10}$ = H), MEL-C ($R^{10}$ = $-COCH_3$, $R^{11}$ = H), MEL-D ($R^{10}$ = $R^{11}$ = H), Variationen in Kettenlänge und Sättigungsgraden der Fettsäureacylreste in C2'und C3'des Mannosylrestes, z.B. MEL-C10-C10 ($R^{12}$ = $R^{13}$= $C_9H_{19}$), MEL-C16:1-C4.

Mannosylerythritollipide können z.B. über Pseudomonas, Candida oder Ustilago, z.B. Pseudozyma Antarctica NBRC 10736, Pseudozyma tsukubaensis oder Ustilago maydis erhalten werden.

**Cellobiose Lipid**

Unter dem Begriff Cellobioselipid im Zusammenhang mit der vorliegenden Erfindung werden insbesondere Verbindungen der allgemeinen Formel (V) oder deren Salze verstanden

(V)

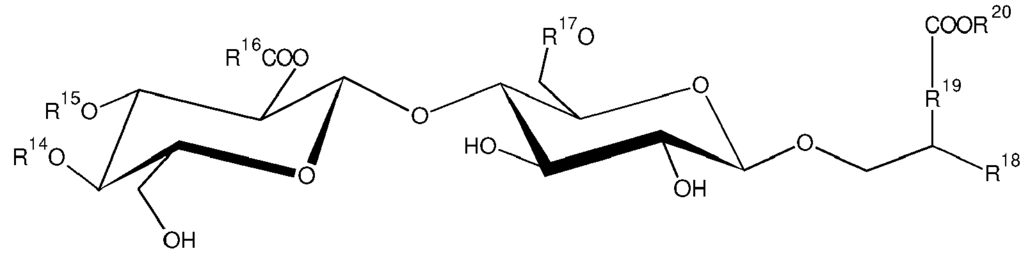

$R^{14}$, $R^{15}$ und $R^{17}$= unabhängig voneinander H oder $-COCH_3$,

$R^{16}$ = gesättigter oder ungesättigter, hydroxylierter oder nicht-hydroxylierter Kohlenwasserstoff mit 5 bis 23 Kohlenstoffatomen, oder $-CH_3$, bevorzugt - $CH_3$ oder - $CH_2$-CH(OH)-$(CH_2)_n$-$CH_3$ mit n= 2-4,

$R^{18}$= H oder -OH,

$R^{19}$= gesättigter oder ungesättigter, hydroxylierter oder nicht-hydroxylierter Kohlenwasserstoff mit 9 bis 21 Kohlenstoffatomen, bevorzugt 13 Kohlenstoffatome, besonders bevorzugt - $(CH_2)_n$-CH(OH)- mit n= 12,

$R^{20}$= H, oder ein Kation.

Cellobioselipide können durch literaturbeschrieben Verfahren durch Crypotcoccus humicola, Pseudozyma fusiformata oder durch den Brandpilz Ustilago maydis erhalten werden.

**Trehaloselipide**

Unter dem Begriff Trehaloselipide im Zusammenhang mit der vorliegenden Erfindung werden insbesondere Verbindungen der allgemeinen Formel (VI) oder deren Salze verstanden.

(VI)

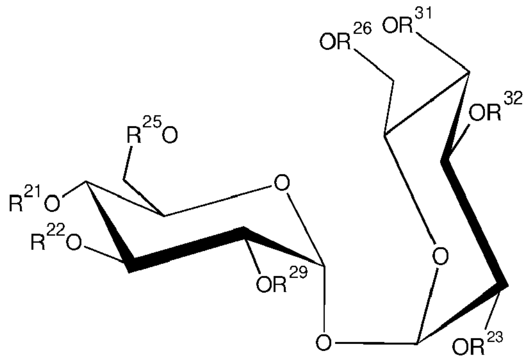

$R^{21}$, $R^{22}$, $R^{31}$ und $R^{32}$= jeweils unabhängig voneinander Wasserstoff oder $COR^{27}$ mit $R^{27}$ gesättigtem oder ungesättigtem, hydroxyliertem oder nicht-, hydroxyliertem Kohlenwasserstoff mit 5 bis 23 Kohlenstoffatomen;
$R^{25}$ und $R^{26}$ jeweils unabhängig voneinander Wasserstoff oder $COR^{28}$ mit $R^{28}$ gesättigtem oder ungesättigtem, verzweigtem oder nicht-verzweigtem, hydroxyliertem oder nicht-, hydroxyliertem Kohlenwasserstoff mit 5 bis 23 Kohlenstoffatomen oder $R^{28}$ = $CH[(CH_2)_cCH_{3]}CHOH(CH_2)_dCH_3$ mit c+d = 27;$R^{29}$ und $R^{23}$ jeweils unabhängig voneinander Wasserstoff, $COR^{30}$ mit $R^{30}$ gesättigtem oder ungesättigtem, verzweigt oder nicht-verzweigtem, hydroxyliertem oder nicht-, hydroxyliertem Kohlenwasserstoff mit 5 bis 23 Kohlenstoffatomen oder $R^{30}$ = $(CH)_2COOR^{24}$ mit $R^{24}$= H oder Kation.

Der Zuckerteil der Trehaloselipide besteht aus zwei α,α'-1,1-glycosidisch verknüpften Glucose-Molekülen. Die lipophilen Teile stellen unterschiedlichste Acylreste dar, acyliert mit Fettsäuren oder organischen Säuren, z.B. Succinyl Trehalose Lipide (STL) zugänglich durch Rhodococcus sp oder Mykolsäure, Corynomykolsäure oder Norcadomykolsäure (Corynebacterium bzw. Nocardia). Erfindungsgemäss eignen sich vorzugsweise Tetralose Dimycolate, Tetralose Monomycolate, Succinyl Trehalose Lipide und Trehalose Tetraester oder Gemische derselben.

Beispiele besonders bevorzugter Trehalosen sind STL-1 (3,4-di-O-alkanoyl-2-O-succinoyl-α-d-glucopyranosyl-2'-O-succinoyl-α-d-glucopyranoside) oder 3,4-di-O-alkanoyl-2-O-succinoyl-α-d-glucopyranosyl-2'-O-alkanoyl-α-d-glucopyranoside, z.B. gesättigten, linearen C8-C16 Alkanoylresten. Trehaloselipide können durch literaturbeschriebene Verfahren durch Rhodococcus erythropolis, Rhodococcus sp., Arthobacter spec., Gordonia, Mycobacterium tuberculosis, *Nocardia* Cornynebacterium spec. oder Tsukamurella spec. erhalten werden.

Die Zubereitungen enthalten bevorzugt 0.1 bis 50 Gew.-% eines oder mehrerer Glycolipid-Biotenside, bevorzugter 0.1 bis 25 Gew.-%, besonders bevorzugt 0.1 bis 15 Gew.-% und ganz besonders bevorzugt 0.3 bis 10 Gew.-%, bezogen auf die Gesamtmenge der Zubereitung.

**Zusammensetzungen**

Verhältnisse und Mengen

In mindestens einer Ausführungsform liegt das mindestens eine N-Acylglycamin der Formel (I), vorzugsweise wie vorhergehend als bevorzugt beschrieben und insbesondere das Gemisch von N-Acylglycaminen abgeleitet von C-18-Pflanzenölen, als alleinige Tenside eingesetzt werden.

In mindestens einer Ausführungsform liegt das mindestens eine N-Acylglycamin der Formel (I), vorzugsweise wie vorhergehend als bevorzugt beschrieben und insbesondere das Gemisch von N-Acylglycaminen abgeleitet von C-18-Pflanzenölen, als alleinige Tenside mit dem mindestens einen Biotensid in der Zusammensetzung vor.

In mindestens einer Ausführungsform liegen die erfindungsgemässen Zusammensetzungen als fliessfähige Ausführungsformen bei 25°C und Normaldruck vor, dies sind insbesondere flüssige, gelförmige oder pastöse Ausführungsformen.

Die Zusammensetzungen, enthaltend mindestens ein N-Acylglycamin und bevorzugt mindestens ein Biotensid,

weisen in der fliessfähige Ausführungsform bevorzugt einem Lösungsmittelgehalt > 10 Gew.-%, einen N-Acylglycamin-Gehalt bis zu 40 Gew.- %, bevorzugt < 10 Gew.-%, und besonders bevorzugt zwischen 0,2 und 5 Gew.-% auf, Gew.-% bezogen auf die Zusammensetzung.

Die fliessfähige Ausführungsform weist zudem bevorzugt einen Gehalt an anionischen Tensiden unter 30 Gew.-% auf. Anionische Tenside umfassen dabei die gegebenenfalls anionischen Biotensid-Glykolipide und zusätzlichen anionischen Tenside in der Zusammensetzung, besonders bevorzugt liegt der Gehalt des N-Acylglycamins gleichzeitig unter 10 Gew.-% liegt, Gew.-% bezogen auf das gesamte Zusammensetzung.

In mindestens einer Ausführungsform liegen die Zusammensetzungen als feste Formulierungen vor. Die Zusammensetzungen enthaltend mindestens ein N-Acylglycamin, und bevorzugt enthaltend mindestens ein Biotentensid enthalten in der festen Formulierung bis zu 75 Gew.-% N-Acylglycamin, bevorzugt 0,1 bis 20 Gew.-%, bevorzugter zwischen 0,2 und 12 Gew.-%, und besonders bevorzugt zwischen 0,2 und 5 Gew.-%.

Die feste Formulierung weist zudem bevorzugt einen Gehalt an Tensiden, ausgewählt aus den Aniontensiden und den bevorzugten Glycolipid-Biotensiden, bis 65 Gew.-% auf, besonders bevorzugt liegt der Gehalt des N-Acylglycamins gleichzeitig unter 20 Gew.-% liegt, und ganz besonder bevorzugt liegt zusätzlich der Wassergehalt < 10 Gew-%; Gew.-% bezogen auf das gesamte Zusammensetzung.

In den bevorzugten Ausführungsformen mit Glycolipid- Biotensiden, enthalten die Zusammensetzungen mindestens ein Glycolipid-Biotensid mit einem bevorzugten Gesamtgehalt von mindestens 0,3 Gew.-%, bevorzugt grösser als 0,3 Gew.-%, bevorzugter > 1 Gew.-%, besonders bevorzugt ≥ 5 Gew.-% ganz besonders bevorzugt > 5 Gew.-%, bezogen auf die Zusammensetzung

Insbesonders sind für die alternativen Ausführungsformen die folgenden Konzentrationsbereiche der Glycolipid-Biotenside bevorzugt:

- Feste Formulierungen für Wasch- und Reinigungsprodukte in Haushalt oder Gewerbe:
  Gesamtgehalt des mindestens einen Biotensids mindestens 0,05 Gew.-%, bevorzugt mindestens 0,3 Gew.-%, bevorzugt grösser als 0,3 Gew.-%, besonders bevorzugt > 1 Gew.-%
- Feste Formulierungen für Pflegeprodukte in Haushalt oder Gewerbe
  Gesamtgehalt des mindestens einen Biotensids mindestens 0,05 Gew.-%, bevorzugt mindestens 0,3 Gew.-%, bevorzugt grösser als 0,3 Gew.-%, besonders bevorzugt > 1 Gew.-%
- Feste Formulierungen für Reinigung- und Pflege in der Kosmetik
  Gesamtgehalt des mindestens einen Biotensids mindestens 0,05 Gew.-%, bevorzugt mindestens 0,3 Gew.-%, bevorzugt grösser als 0,3 Gew.-%, besonders bevorzugt > 1 Gew.-% .
- Fliessförmige Wasch- und Reinigungsmittel in Haushalt und Gewerbe:
  Gesamtgehalt des mindestens einen Biotensids mindestens 0,3 Gew.-%, bevorzugt grösser als 0,3 Gew.-%, bevorzugter > 1 Gew.-%, besonders bevorzugt ≥ 5 Gew.-% ganz besonders bevorzugt > 5 Gew.-%.
- Fliessförmige Pflegeprodukte in Haushalt oder Gewerbe
  Gesamtgehalt des mindestens einen Biotensids mindestens 0,05 Gew.-%, bevorzugt mindestens 0,3 Gew.-%, bevorzugt grösser als 0,3 Gew.-%, besonders bevorzugt > 1 Gew.-% .
- Fliessfähige Formulierungen für Reinigung- und Pflege in der Kosmetik
  Gesamtgehalt des mindestens einen Biotensids mindestens 0,3 Gew.-%, bevorzugt grösser als 0,3 Gew.-%, bevorzugter > 1 Gew.-%, besonders bevorzugt ≥ 5 Gew.-% ganz besonders bevorzugt > 5 Gew.-%.

In den bevorzugten Ausführungsformen mit Glycolipid- Biotensiden, enthalten die Zusammensetzungen mindestens ein Glycolipid-Biotensid, wobei das Verhältnis von Biotensid : N-Acylglycamin bevorzugt > 1:1, bevorzugter > 5: 2,9 (18er Beispiel), besonders bevorzugt ≥ 2 : 1 und äusserst bevorzugt ≥ 3 : 1 beträgt (bezogen auf Gew.-%).

Im Sinne dieser Erfindung umfassen die Zusammensetzungen, insbesondere die Reinigungs-und Pflegemittel, bevorzugt neben dem mindestens einen N-Acylglycamin, und bevorzugt dem mindestens einen zusätzlichen Biotensid, zusätzlich mindestens 4, bevorzugter 5, weitere Inhaltsstoffe umfassend die Gruppen: Lösungsmittel; weitere (von N-Acylglycamin und Glycolipid-Biotensiden) unterschiedliche Tenside; Emulgatoren, Enthärter und Komplexbildner; Viskositätsregulatoren; Konsistenzgeber, pH-Stellmittel, Puffersubstanzen, Säuren, Basen; Konditionierungsmittel, Gerüststoffe; Lösungsvermittler; Abrasiva; Antioxidantien; Vitamine; UV-Filter; physiologische Wirkstoffe ("actives"), z.B. antiageing Wirkstoffe, Trübungsmittel; Polymere, Antikorrosionsmittel; Konservierungsmittel; Fette und Öle, Emollients, Duftstoffe; Farbstoffe; Bleichmittel; anorganische Alkali- oder Erdalkalisalze; gegebenenfalls Enyzme.

Unter diesen weiteren Inhaltsstoffen werden Komponenten verstanden, die zur Herstellung der Zusammensetzung gezielt zugegeben werden - also keine prozessbedingten Komponenten, wie Produktionshilfsmittel, Nebenprodukte oder Edukte. Werden mehrere Komponenten einer Kategorie, z.B. zwei unterschiedliche Enzyme zugegeben, werden diese als 2 Inhaltsstoffe gezählt. Gemische, welche unter einer chemischer Bezeichnung, einer CAS-Nummer bzw. einem INCI-Namen zusammengefasst werden, werden als einzelner Inhaltsstoff gezählt, z.B. Produktgemische mit unterschiedlichen Kettenlängen, z.B. "Kokos", PEG-7, etc.

In einer bevorzugten Ausführungsform der Emulsion (fliessfähig) beträgt der Anteil an nicht-wassermischbaren, organischen Verbindungen, die bei 20-25°C in flüssiger Form vorliegen, z.B. verzweigte oder lineare Kohlenwasserstoffe, Ether oder Ester sowie fette Öle, in den erfindungsgemässen Zusammensetzungen bevorzugt < 10 Gew.-%, bezogen auf die Gesamtzusammensetzung). Der pH-Wert der erfinderischen Zusammensetzung liegt bevorzugt zwischen zwischen 3 und 11,5.

Anteile PEG-18-Tenside

Mindestens eine Ausführungsform der Erfindung stellt die Zusammensetzung dar, enthaltend mindestens ein N-Acylglycamin und bevorzugt enthaltend mindestens ein Biotensid, und zusätzlich enthaltend mindestens ein weiteres Tensid. Dieses kann in Hinsicht auf den lipophilen Tensid petrochemischen Ursprungs, z.B. Erdöl, oleochemischen, z.B. C-18-Pflanzenöl, Kokosöl, Palmkernöl, Algenöl, etc., oder tierischen Ursprungs, z.B. Talg sein.

Bevorzugt betragen in den Zusammensetzungen, enthaltend mindestens ein N-Acylglycamin und bevorzugt mindestens ein Biotensid der Gehalt an nichtionischen PEG-Tensiden abgeleitet von C-18-Pflanzenölen ≤ 66 ,6 Gew.-%, bevorzugt ≤ 54,5 Gew.-%, und besonders bevorzugt ≤ 18 Gew.-% liegt, bezogen auf den Gesamtgehalt an Tensiden; in mindestens einer besonders bevorzugten Ausführungsvariante beträgt der Gehalt der nichtionischen PEG-Tensiden abgeleitet von C-18-Pflanzenölen < 1 Gew.-%, bevorzugter < 0,1 Gew.-%, noch bevorzugter < 0,01 Gew.-%, und ganz bevorzugt ist die Ausführungsvariante frei von nichtionischen PEG-Tensiden abgeleitet von C-18-Pflanzenölen, bezogen auf die Gesamtzusammensetzung.

Insbesondere beträgt das bevorzugte Mengenverhältnis der N-Acylglycamine zu den optionalen nichtionischen PEG-Tensiden abgeleitet von C-18-Pflanzenölen bevorzugt > 1 : 2.

Bevorzugt betragen in den Zusammensetzungen, enthaltend mindestens ein N-Acylglycamin und bevorzugt mindestens ein Biotensid der Gehalt an anionischen PEG-Tensiden abgeleitet von C-18-Pflanzenölen ≤ 66 ,6 Gew.-%, bevorzugt ≤ 54,5 Gew.- %, und besonders bevorzugt ≤ 18 Gew.-% liegt, bezogen auf den Gesamtgehalt an Tensiden; Bevorzugt beträgt der Gehalt an anionischen PEG-Tensiden abgeleitet von C-18-Pflanzenölen in den Zusammensetzungen, enthaltend mindestens ein N-Acylglycamin und bevorzugt mindestens ein Biotensid in mindestens einer besonders bevorzugten Ausführungsvariante < 0,5 Gew-% bevorzugter < 0,1 Gew.-%, noch bevorzugter < 0,01 Gew.-%, und ganz bevorzugt ist die Ausführungsvariante frei von anionischen PEG-Tensiden abgeleitet von C-18-Pflanzenölen, bezogen auf die Gesamtzusammensetzung. Insbesondere beträgt das bevorzugte Mengenverhältnis der N-Acylglycamine zu den optionalen anionischen PEG-Tensiden abgeleitet von C-18-Pflanzenölen > 1 : 3,4.

Weitere PEG-Reduzierte Formen

Für manche Anwendungen mag der Wunsch bestehen, PEG-haltige Tenside gesamthaft zu reduzieren.

Für mindestens eine bevorzugte Ausführungsform beträgt der Gesamtgehalt an PEG-Tensiden mit Fettresten von 18 oder mehr C-Atomen in Summe ≤ 66 ,6 Gew.-%, bevorzugter ≤ 33 Gew.-%, besonders bevorzugt ≤ 22 Gew.-%, ganz besonders bevorzugt ≤ 11 Gew.-%, bezogen auf den Gesamtgehalt an Tensiden; eine äusserst bevorzugte Ausführungsform ist PEG-frei , d.h. der Gesamtgehalt an PEG-Tensiden in der Zusammensetzung ist < 0,1 Gew.-%, bevorzugter < 0.01 Gew.-%, Gew.-% bezogen auf den Tensidgehalt in der Zusammensetzung.

Für mindestens eine bevorzugte Ausführungsform beträgt der Gesamtgehalt an PEG-Tensiden mit Fettresten von C8-C22 in Summe ≤ 66 ,6 Gew.-%, bevorzugter ≤ 33 Gew.-%, besonders bevorzugt ≤ 22 Gew.-%, ganz besonders bevorzugt ≤ 11 Gew.- %, bezogen auf den Gesamtgehalt an Tensiden; eine äusserst bevorzugte Ausführungsform ist PEG-frei.

In mindestens einer ganz besonders bevorzugt beträgt in PEG-haltigen Ausführungsformen der Gesamtgehalt an alkoxylierten Tensiden in der Zusammensetzung, in Summe ≤ 66 ,6 Gew.-%, bevorzugter ≤ 33 Gew.-%, besonders bevorzugt ≤ 22 Gew.-%, ganz besonders bevorzugt ≤ 11 Gew.-%, bezogen auf den Gesamtgehalt an Tensiden; eine äusserst bevorzugte Ausführungsform ist PEG-frei. ,

Desweiteren beträgt in der PEG-haltigen Ausführungsform das Verhältnis Biotensid zu PEG-Tensiden vorzugsweise > 1: 2, bevorzugter > 1 : 1, besonders bevorzugt > 2 : 1.

**Anionische Tenside**

Mindestens eine Ausführungsform der Erfindung stellt die Zusammensetzung dar, enthaltend ein oder mehrere N-Acylglycamine und bevorzugt enthaltend ein oder mehrere Biotenside, und zusätzlich enthaltend mindestens ein weiteres anionisches Tensid.

Alle anionischen Tenside können jeweils auch in deren alkoxylierten Formen vorliegen, z.B. Alkylsulfat, Alkylethersulfat oder Sulfosuccinate, ethoxylierte Sulfosuccinate, etc. Die anionischen Tenside können jeweils in Form ihrer Alkali-, Erdalkali- oder Ammoniumsalze, sowie als lösliche Salze organischer Basen, wie Mono-, Di- oder Tri- bzw.

Tetraalkyl- oder - alkylenammonium, wobei gegebenenfalls die Alkylreste oder Alkylenreste ein oder mehrere Heteroatome enthalten oder in der Säureform vorliegen.

Eine Ausführungsform der Erfindung stellt die Zusammensetzung dar, enthaltend ein oder mehrere N-Acylglycamine und bevorzugt enthaltend ein oder mehrere Biotenside, welche zusätzlich weitere anionische Tenside enthalten kann. Bevorzugte anionische Tenside sind ausgewählt aus den Gruppen der Seifen (Salze von Fettsäuren), Alkylsulfate, Alkylethersulfaten, Alkenylsulfate, Alkenylethersulfate, Sulfonate, Fettalkoholcarboxylate, Alkylethercarboxylate, Alkenylethercarboxylate, Amidethercarboxylate, anionische N-Acylamide, N-Acylaminosäuretenside, acylierte Polypeptide, Alkylisethionate, Alkenylisethionate Acylisethionate, Acyl methyl isethionate, Acyltaurine, Acyl Methyl taurate, Acyl sulfate, Sulfatierte Amide, sulfatierte Fettsäuren oder sulfatierte Öle, Carbonsäureamidethersulfate, Acyllactylate und Lactyl Lactate, anionische Glycolipide, z.B. Polyhydroxyalkylether oder Polyhydroxyalkenylether oder Polyhydroxyalkylamide oder Polyhydroxyalkenylamide, jeweils derivatisiert mit anionischen Gruppen, wie bspw. Citrat, Tartrat, Carboxylat, Sulfat, Sulfonat oder Phosphate; Sulfosuccinate, Sulfoacetate; Alkyletherphosphate, Alkylenetherphosphate, Phosphor- und Polyphosphorsäureester, Alkyl- und Alkenyltartrate, insbesondere ausgewählt aus Alkylsulfate, Alkylethersulfaten, Alkenylsulfate, Alkenylethersulfate, Sulfonate, Fettalkoholcarboxylate, Alkylethercarboxylate, Alkenylethercarboxylate, Amidethercarboxylate, anionische N-Acylamide, N-Acylaminosäuretenside ausgewählt aus Acylglycinat, Acylaspartat, Acylsarcosinat, acylierten Polypeptide, Alkylisethionate, Alkenylisethionate Acylisethionate, Acyl methyl isethionate, Acyltaurine, Acyl Methyl taurate, Acyl sulfate, Sulfatierte Amide, sulfatierte Fettsäuren oder sulfatierte Öle, Carbonsäureamidethersulfate, Acyllactylate und Lactyl Lactate, anionische Glycolipide, z.B. Polyhydroxyalkylether oder Polyhydroxyalkenylether oder Polyhydroxyalkylamide oder Polyhydroxyalkenylamide, jeweils derivatisiert mit anionischen Gruppen, wie bspw. Citrat, Tartrat, Carboxylat, Sulfat, Sulfonat oder Phosphate; Sulfosuccinate, Sulfoacetate; Alkyletherphosphate, Alkylenetherphosphate, Phosphor- und Polyphosphorsäureester, Alkyl- und Alkenyltartrate

In mindestens einer bevorzugten Ausführungsform, enthält die Zusammensetzung mindestens ein N-Acylglycamid, bevorzugt ein Sunfloweroyl methyl glucamid, und bevorzugt mindestens ein Biotensid und weitere anionische Tenside, wobei die Tenside gesättigte Fettreste, also Alkanol- oder Acylgruppen, mit Kettenlängen zwischen 6-24 Kohlenstoffatomen, oder Gemische aus > 60 Gew.-% gesättigten Alkanol- oder Acylgruppen mit 8 bis18 Kohlenstoffatomen und < 40 Gew.-% ungesättigten C-18-Alkenol-oder Acylgruppen enthalten, wie sie aus Kokosöl, Palmkernöl oder Babassuöl, tierischem oder petrochemischen Usprung erhalten werden, bevorzugt ausgewählt aus den Gruppen der Seifen (Salze von Fettsäuren), Alkylsulfate, Alkylethersulfaten, Alkenylsulfate, Alkenylethersulfate, Sulfonate, Fettalkoholcarboxylate, Alkylethercarboxylate, Alkenylethercarboxylate, Amidethercarboxylate, anionische N-Acylamide, N-Acylaminosäuretenside, acylierte Polypeptide, Alkylisethionate, Alkenylisethionate Acylisethionate, Acyl methyl isethionate, Acyltaurine, Acyl Methyl taurate, Acyl sulfate, Sulfatierte Amide, sulfatierte Fettsäuren oder sulfatierte Öle, Carbonsäureamidethersulfate, Acyllactylate und Lactyl Lactate, anionische Glycolipide, z.B. Polyhydroxyalkylether oder Polyhydroxyalkenylether oder Polyhydroxyalkylamide oder Polyhydroxyalkenylamide, jeweils derivatisiert mit anionischen Gruppen, wie bspw. Citrat, Tartrat, Carboxylat, Sulfat, Sulfonat oder Phosphate; Sulfosuccinate, Sulfoacetate; Alkyletherphosphate, Alkylenetherphosphate, Phosphor- und Polyphosphorsäureester, Alkyl- und Alkenyltartrate.

Exemplarische Vertreter, aber nicht limitierend, aus den jeweiligen Tensidgruppen sind

Seifen: $C_{12}$-$_{18}$- Fettsäure(natriumsalz), Palmkernölfettsäure, Natriumcocoat, Kaliumoleate, POTASSIUM COCOATE, Alkylsulfate und Alkylethersulfate, insbesondere C8-C20 Alkylsulfate, z.B. Sodium Coco Sulfate, Sodium Lauryl Sulfate (SLS), Ammonium Lauryl Sulfate, Cetearylsulfat, $C_{16\text{-}18}$- Fettalkoholsulfat, Sodium octyl sulfat, oder C8-C20 Alkylethersulfate, insbesondere sulfatierte alkoxylierte Fettalkohole, alkoxyliert mit 1 bis 10 Ethylenoxid (EO)-, Propylenoxid (PO) - oder Butylenoxid (BO)-einheiten oder deren Kombinationen, bevorzugt 1-10 EO im Etherteil zum Beispiel Sodium Laureth Sulfate (2 oder 3 EO) (SLES), Ammonium Laureth Sulfate, Magnesium laureth sulfate, MEA-Laurethsulfate, MIPA-Laureth Sulfate, Sodium Trideceth Sulfate, Sodium Myreth Sulfate, Sodium $C_{10\text{-}16}$ Pareth-2 sulfate, Sodium Coceth-sulfate; Alkenylsulfate und Alkenylethersulfate, z.B. Sodium oleyl sulfate, *TEA*-Oleyl Sulfate; Sulfonate: petrochemische Sulfonate wie alpha-Olefin Sulfonate, $C_9$-$_{13}$-Alkylbenzolsulfonsäure (LAS), NaLAS, Alkylbenzolsulfonate, MEA Dodecylbenzenesulfonat, lineare und verzweigte Alkansulfonate, und bevorzugt, oleochemische Sulfonate, z.B: lineare $C_{8\text{-}18}$-Alkansulfonate, Alkensulfonate, Alkylethersulfonate, Alkenylethersulfonate, besonders bevorzugte Sulfonate sind folgende Gruppen: Sulfoacetate, Sulfobernsteinsäurester oder Sulfosuccinate, sulfonierte Fettsäuren, sulfonierte Fettsäureester, wie sulfonierte Fettsäuremethylester (MES), wie, z.B. Natriumsalz des $\alpha$-sulfonierte $C_{12}$-$_{14}$-Fettsäuremethylester (MES), sulfonierte Fettsäureglycerinester und sulfonierte Fettsäuremethylester, Sodium Lauryl Sulfoacetat; Sulfosuccinate: z.B. Alk(en)ylsulfosuccinate, Dialk(en)ylsulfosuccinate, Sulfosuccinamate, ethoxylierte Alk(en)ylsulfosuccinat ,Disodium Laureth Sulfosuccinate, Disodium Lauryl Sulfosuccinat, Sulfobernsteinsäuremono- und di-Alkylester; insbesondere auch ethoxyliert, Sulfoacetate: Alkylsulfoacetate, Alkenylsulfonate, z.B. Sodium Lauryl Sulfoacetate, Sodium Methyl 2-Sulfolaurate, Disodium 2-Sulfolaurate, Fettalkoholcarboxylate, z.B. Alkylethercarboxylate, Potassium Laureth-4 Carboxylate, Sodium Laureth-11 Carboxylate, N-Acylaminosäuretenside und N-Acylpolypeptide:Eiweiss-Fettsäurekondensate und Aminosäure-Fettsäurekondensate, z.B. N-Acylaspartat, N-Acylglycinat, N-Acylalaninat, N-Acylglutamate, N-Acylsarkosinat, oder acylierte Polypeptiden, N-Acylaminosulfonsäuren, beispielsweise Sodium Lauroyl Sarcosinate, Disodium cocoyl Glutamate, Sodium Cocoyl Glutamate, Cocoylglycinate, Natrium Lauroylglycinat, u.a., Alkylisethionate, Acylisethionate, Acyl methyl isethionate, z.B. Sodium Lauroyl Methyl isethionate, Sodium Cocoyl Isethionate; Acyltaurine, Acyl methyl taurat, z.B. Sodium Methyl Cocoyl Taurate, Sodium Methyl Oleoyl Taurate, Carbonsäureamidethersulfate, Sulfatierte Fettsäuren, Amide oder Öle, z.B.sulfatiertes Rizinusöl; Sulphatierte Alkan- oder Alkenolamid ethoxylate, Sulfatierte Polyoxyethylenamide, Mono-, di- oder triglyceridsulfate, Acyllactylate und Lactyl Lactate: z.B. Lauryl lactate, Palmitoyl lactate, Lauryl Lactyl lactate, anionische Glykolipide, z.B. Alk(en)ylglucoside Citrat oder Tartrat, Sodium Lauryl Glucose-Carboxylate Acyl sulfate, z.B. Sodium acyl sulfate; Alkylphosphate und Alkyletherphosphate, Phosphor- und Polyphosphorsäureester z.B. Oleylalcohol-EO-phosphate Isodecanol-EO-phosphate, Alkoxylated cetyl ether phosphate, Alkyl- und Alkenyltartrate, z.B. Di-C12-13 Alkyl Tartrate. Gegebenenfalls können jeweils diese Vertreter zusätzlich ethoxyliert oder propoxyliert vorliegen.

Mindestens eine Ausführungsform enthält anionische alkoxylierte Tenside. Erfinderisch bevorzugte anionische alkoxylierte Tenside sind ausgewählt aus der Gruppe Alkyl(en)ethersulfaten mit 6 bis 22 Kohlenstoffatomen und 1-6 EO-Einheiten, bevorzugt C8-C16, insbesondere Sodium laureth sulfaten; Monoester und/oder Diester der Sulfobernsteinsäure mit alkoxylierten Fettalkoholen, Salze von Alkylethercarbonsäuren, oder Alkyletherphophate, z.B. Sodium laureth-5-carboxylat.

Eine besonders bevorzugte sulfatfreie Ausführungsform enthält anionische PEG-haltige Tenside ausgewählt aus Monoester und/oder Diester der Sulfobernsteinsäure mit alkoxylierten Fettalkoholen, Salze von Alkylethercarbonsäuren, Alkyletherphophate.

Ganz besonders bevorzugt ist die Ausführungsvariante der Zusammensetzung, die frei von anionischen alkoxylierten Tensiden ist.

Eine bevorzugte Ausführungsform ist frei von Alkylethersulfaten, die von einigen Konsumentengruppen als ökologisch nachteilig angesehen werden. Besonders bevorzugt sind die optionalen anionischen Tenside dann ausgewählt aus den obigen Gruppen ohne den Alkylethersulfaten. Noch bevorzugter sind die optionalen anionischen Tenside ausgewählt aus den obigen Gruppen ohne Alkylethersulfate und ohne Alkylsulfate, da diese als stark haut- und augenreizend eingestuft sind und die erfinderischen Zusammensetzung auch ohne diese Tenside die gewünschten Eigenschaften erbringen.

Mindestend eine bevorzugte, besonders milde, Ausführungsform der Erfindung stellt die Zusammensetzung dar, enthaltend ein oder mehrere N-Acylglycamine (vorzugsweise wie vorstehend als bevorzugt bezeichnet) und gegebenenfalls den bevorzugt enthaltenen ein oder mehreren Biotensiden, und zusätzlich optionalen anionische Tensiden ausgewählt aus den bevorzugten Gruppen Gruppen der Seifen (Salze von Fettsäuren), Sulfonate, Fettalkoholcarboxylate, Alkylethercarboxylate, Alkenylethercarboxylate, Amidethercarboxylate, Alkylisethionate, Alkenylisethionate, Acylisethionate, Acyl methyl isethionate, Acyltaurine, Acyl Methyl taurate, Acyl sulfate, Acyllactylate und Lactyl Lactate, anionische Glycolipide, z.B. Polyhydroxyalk(en)ylether, -amide derivatisiert mit anionischen Gruppen, wie bspw. Citrat, Tartrat, Carboxylat, Sulfat, Sulfonat oder Phosphate; Sulfosuccinate, Sulfoacetate; Alkyletherphosphate, Alkyleneetherphosphate, Phosphor- und Polyphosphorsäureester, Alkyl- und Alkenyltartrate; und zusätzlich optionalen zwitterionischen und optionalen kationischen Tenside.

Mindestens eine weitere alternative bevorzugte Ausführungsform enthält die ein oder mehrere N-Acylglycamine (vorzugsweise wie vorstehend als bevorzugt bezeichnet) und gegebenenfalls die bevorzugt enthaltenen ein oder mehreren Biotensiden, und als optionale weitere Tenside zwitterionische und kationische Tenside. In dieser Ausführungsform sind also keine weiteren anionischen Tenside enthalten. Diese bevorzugten Ausführungsformen zeichnen sich durch besondere Milde aus, da anionische Tenside Haut- und Augenirritationen hervorrufen können. Diese Ausführungsvariante ist besonders überraschend, da in herkömmlichen Reinigungsmitteln das anionische Tensid, insbesondere Alkylsulfate oder Alkylethersulfate, weitgehend die Reinigungsaufgabe übernimmt. Beispiele für diese Ausführungsform sind insbesondere kosmetische Reinigungs- und Pflegemittel (z.B. Haarshampoo, Conditioner, Hautpflege, Sensitiv-Produkte) und Handgeschirrspülmittel und Weichspüler.

Jeweils mindestens eine Ausführungsform enthält folgende bevorzugte, besonders bevorzugte oder äusserst bevorzugte Tensidkombination mit weiteren anionischen Tensiden, wobei vorzugsweise jeweils die bevorzugten N-Acylglycamine und optionalen, aber bevorzugten, Biotenside wie vorgehend beschrieben eingesetzt werden. Jeweils ganz besonders bevorzugt sind für die Kombinationen mit weiteren anionischen Tensiden die Biotenside ausgewählt aus Rhamnolipiden, Sophorolipiden und Mannosylerytrhillipiden und deren beliebige Kombinationen, insbesondere Rhamnolipide und Sophorolipide oder deren Kombination.

Jeweils mindestens eine Ausführungsform enthält die bevorzugten Tensidkombinationen:

- Mindestens ein N-Acylglycamin + bevorzugt mindestens ein Biotensid + mindestens ein C8-C22-Alkylethersulfat

oder Alkylsulfat, bevorzugter C8-C12, insbesondere bevorzugt sind die Alkylsulfate, äusserst bevorzugt Natrium Cocoylsulfat. Im Rahmen einer bevorzugten Ausführungsform enthalten die Kombinationen zusätzlich mindestens ein weiteres anionisches Tensid ausgewählt aus Seife, Sulfobernsteinsäureestern, Aminosäuretensiden.

- N-Acylglycamin + bevorzugt Biotensid + C8-C22-Alkyl- oder Alkenylethercarboxylate oder Amidethercarboxylate

Mindestens eine Ausführungsform enthält die besonders bevorzugten Tensidkombinationen:

- Mindestens ein N-Acylglycamin + bevorzugt mindestens ein Biotensid + mindestens ein N-Acylaminosäuretensid oder N-Acylpolypeptid, besonders bevorzugt C8-22-acylierten Aminosäure, insbesondere auch deren N-alkylierten Derivate. Im Rahmen einer bevorzugten Ausführungsform ist das Aminosäuretensid ausgewählt aus der Gruppe bestehend aus Acylglycinat, Acylaspartat, Acylglutamat, Acylsarcosinat, z.B. Sodium cocoyl glutamate, Sodium lauroyl sarcosinate, Sodium cocoyl glycinate.

Jeweils mindestens eine Ausführungsform enthält die äusserst bevorzugten Tensidkombinationen:

- Mindestens ein N-Acylglycamin + bevorzugt mindestens ein Biotensid + mindestens eine Seife, insbesondere bevorzugt sind die Seifen von Fettsäureresten abgeleitet von C-18-Pflanzenölen bevorzugt gemäss den als bevorzugt beschriebenen Fettsäureverteilungen, z.B. Olivenölseife, Sonnenblumenseife oder einer Seife abgeleitet von Kokos-, Palm-, oder Palmkernöl, besonders bevorzugt abgeleitet von Kokos-, Palm-, oder Palmkernöl.

- Mindestens ein N-Acylglycamin + bevorzugt mindestens ein Biotensid + mindestens ein Sulfonate, insbesondere oleochemische Sulfonate, bevorzugt ausgewählt aus Sulfoacetaten, Sulfosuccinaten, sulfonierten Fettsäuren, sulfonierten Fettsäureester, wie sulfonierte Fettsäuremethylester (MES), Sodium lauryl sulfoacetat.

- Mindestens ein N-Acylglycamin + bevorzugt mindestens ein Biotensid + mindestens ein Acyltaurin oder Acyl Methyl taurat, z.B. Sodium methyl cocoyl taurate, bevorzugt Sodium methyl oleoyl taurate, insbesondere bevorzugt sind Aminosäuretenside mit Fettsäureresten abgeleitet von C-18-Pflanzenölen bevorzugt gemäss den als bevorzugt beschriebenen Fettsäureverteilungen, z.B. enthaltend Sodium methyl oleoyl taurate. In einer bevorzugten Ausführungsform liegen zusätzlich Isethionate oder N-Acylaminosäuretenside vor.

- Mindestens ein N-Acylglycamin + bevorzugt mindestens ein Biotensid + mindestens ein Isethionat (Alkylisethionate, Alkenylisethionate, Acylisethionate, oder Acyl methyl isethionate), z.B. Sodium Cocoyl Methyl isethionate, Sodium cocoyl isethionate. In einer bevorzugten Ausführungsform liegen zusätzlich Taurate oder N-Acylaminosäuretenside vor.

- sowie jeweils deren Kombinationen.

Optional können jeweils zusätzlich amphotere oder nichtionische Tenside enthalten sein. Das N-Acylglycamin folgt bevorzugt jeweils den vorhergehend als bevorzugt definierten Strukturen.

**Nichtionische Tenside**

Mindestens eine Ausführungsform der Erfindung stellt die Zusammensetzung dar, enthaltend ein oder mehrere N-Acylglycamine und bevorzugt enthaltend ein oder mehrere Biotenside, und zusätzlich enthaltend mindestens ein nichtionisches Tensid.

Bevorzugte nichtionische Tenside sind Alkoholpolyglykolether, d.h. ethoxylierte und/oder propoxylierte Alkohole, insbesondere Fettalkohole oder Carbonsäuren mit 1-40 Ethylenoxid (EO) und/oder Propylenoxid (PO)- Einheiten, sowie deren Ester, wie beispielsweise ethoxylierte Fettsäuremethylester oder - ethylester, Aminoxide, Polyethylenglykolmercaptane, Glykolipide, wie zum Beispiel Alkylpolyglykoside mit 1-10 Glykosideinheiten, zusätzliche Polyhydroxyfettsäureamide, Polyhydroxyfettsäureester, sowie Glyceryl- und Polyglycerylester mit 1-20 Glycerineinheiten, Carbonsäureester, Zuckerester: Sorbitanester, sowie alkoxylierte Sorbitanester, Saccharoseester; Alkanolamin-Carbonsäure-Kondensate, auch ethoxyliert, Monoethanolamine, Diethanolamine, Fettaminethoxylate, N-Alkylpyrrolidone, Amidoalkyl-2-pyrrolidone, sowie jeweils deren alkoxylierte, insbesondere ethoxylierte Formen.

Besonders bevorzugte nichtionische Tenside sind ausgewählt aus den Gruppen: Fettalkohole oder Carbonsäuren mit 1-40 Ethylenoxid (EO) und/oder Propylenoxid (PO)- Einheiten, Aminoxide, Polyethylenglykolmercaptane, Glykolipide, wie zum Beispiel Alkylpolyglykoside mit 1-10 Glykosideinheiten, zusätzliche Polyhydroxyfettsäureamide, Polyhydroxyfettsäureester, sowie Glyceryl- und Polyglycerylester mit 1-20 Glycerineinheiten, Carbonsäureester, Zuckerester, wie Sorbitanester, sowie alkoxylierte Sorbitanester, Saccharoseester; Alkanolamin-Carbonsäure-Kondensate, auch ethoxyliert, Fettaminethoxylate, N-Alkylpyrrolidone, Amidoalkyl-2-pyrrolidone.

In mindestens einer bevorzugten Ausführungsform, enthält die Zusammensetzung mindestens ein N-Acylglycamid, bevorzugt ein Sunfloweroyl methyl glucamid, und bevorzugt mindestens ein Biotensid und weitere nichtionische PEG-haltige Tenside, wobei die Tenside gesättigte Fettreste, Alkanol- oder Acylgruppen, mit Kettenlängen zwischen 6-24 Kohlenstoffatomen, oder Gemische aus > 60 Gew.-% gesättigten Alkanol- oder Acylgruppen mit 8 bis18 Kohlenstoffatomen und < 40 Gew.-% ungesättigten C-18-Alkenol- oder Acylgruppen enthalten, wie sie aus Kokosöl, Palmkernöl oder Babassuöl erhalten werden, bevorzugt ausgewählt aus Alkoholpolyglykolether, z.B. ethoxylierte primäre Alkohole mit vorzugsweise 8 bis 18 C-Atomen und durchschnittlich 1 bis 12 Mol Ethylenoxid (EO) pro Mol Alkohol und propoxylierte Alkohole; Fettalkohole oder Carbonsäuren mit 1-40 Ethylenoxid (EO) und/oder Propylenoxid (PO)- Einheiten, sowie deren Ester, wie beispielsweise ethoxylierte Fettsäuremethylester oder - ethylester; alkoxylierte Sorbitanester; ethoxylierte Alkanolamin-Carbonsäure-Kondensate; Fettaminethoxylate.

Besonders bevorzugt sind als nichtionischen Tenside die Sorbitanester ausgewählt aus Sunflower seed oil Sorbitan esters und Sorbitan olivate.

Vorzugsweise werden nichtionische Tenside verwendet, deren hydrophiler Teil pflanzlichen Ursprungs ist, wie beispielsweise Glykolipide, weitere Polyhydroxyfettsäureester, Glyceryl- und Polyglycerylester, Carbonsäureester oder Sorbitanester.

Jeweils mindestens eine Ausführungsform enthält folgende bevorzugte, besonders bevorzugte oder äusserst bevorzugte Tensidkombination mit weiteren nichtionischen Tensiden, wobei vorzugsweise jeweils die bevorzugten N-Acylglycamine und optionalen, aber bevorzugten, Biotenside wie vorgehend beschrieben eingesetzt werden. Jeweils ganz besonders bevorzugt sind für die Kombinationen mit weiteren nichtionischen Tensiden die Biotenside ausgewählt aus Rhamnolipiden, Sophorolipiden und Mannosylerytrhillipiden und deren beliebige Kombinationen, insbesondere Rhamnolipide und Sophorolipide oder deren Kombination.

Mindestens eine Ausführungsform enthält die bevorzugten Tensidkombinationen:

- Mindestens ein N-Acylglycamin + bevorzugt mindestens ein Biotensid + mindestens ein ethoxylierter Alkohol, z.B. ethoxylierte und/oder propoxylierte Guerberetalkohole, lineare oder verzweigte, geradzahlig oder ungeradzahlige Alkohole, z.B Isotridecyl alcohol polyglycol ether, Iso-C10-Oxo-alcohol polyglycol ether, C12/C15-Oxo-alcohol polyglycol ether, C9 /C11-Oxo-alcohol polyglycol ether, u.s.w. bevorzugt ethoxylierte Fettalkohole, z.B. Coconutfatty alcohol polyglycol ether (EO2-25), Laureth-7, C16/C18-Fatty alcohol polyglycol ether, C12/C18-Fatty alcohol polyglycol ether, Oleyl alcohol polyglycol ether u.s.w. Besonders bevorzugt sind die Ausführungsformen enthaltend zusätzlich mindestens eine Seife. Eine bevorzugte Ausführungsform enthält zusätzlich mindestens einen weiteren t, Natriumgluconat, Natriumcarbonat, Natriumhydogencarbonat, Ethanol, Komplexbildner, Konservierungsmittel, Parfüm.

Mindestens eine Ausführungsform enthält die besonders bevorzugte Tensidkombinationen:

- Mindestens ein N-Acylglycamin + bevorzugt mindestens ein Biotensid + mindestens ein C8-C22-Alkylglycosid oder - alkylpolyglycosid, bevorzugter Decyl glucosid, Cocoglucosid, Laurylglucosid, PEG-6 Caprylic/Capric Glyceride, Caprylyl/Capryl Glucoside, C8-C10 Polypentose. Insbesondere bevorzugt sind die Ausführungsformen enthaltend zusätzlich mindestens ein Alkylethersulfat oder noch bevorzugter mindestens ein Alkylsulfat oder Aminosäuretensid, z.B. Cocosulfat, oder die Ausführungsform mit Cocoamidopropylbetain.

Jeweils mindestens eine Ausführungsform enthält die ganz besonders bevorzugte Tensidkombinationen:

- Mindestens ein N-Acylglycamin, bevorzugt ausgwählt aus Sunfloweroyl methyl glucamid oder N-Acyl-N-methyl cyclic glucamide + bevorzugt mindestens ein Biotensid + mindestens ein zusätzliches N-Acylglycamin, z.B. Caproyl/Caproyl Methyl Glucamide, N-C8/10 Acyl-N-Methyl Glucamine Lauroyl/Myristoyl Methyl Glucamide, N-C12/14

Acyl-N-Methyl Glucamine Lauroyl/Myristoyl Methyl Glucamide, Cocoylmethylglucamide, Arachidyl Glucoside. Eine bevorzugte Ausführungsform enthält zusätzlich mindestens ein Tensid ausgewählt aus Cocosulfate, Cocobetaine und Cocoamidopropylbetain.

- Mindestens ein N-Acylglycamin + bevorzugt mindestens ein Biotensid + mindestens ein Alkanolamin-Carbonsäure-Kondensate, z.B. Coconut fatty acid diethanolamide, Coconut fatty acid monoethanolamide,
- Mindestens ein N-Acylglycamin + bevorzugt mindestens ein Biotensid + mindestens ein Zuckerester, z.B. Sucrose stearate, Sunflower seed oil sorbitol ester
- Mindestens ein N-Acylglycamin + bevorzugt mindestens ein Biotensid + mindestens ein Glyceryl- und Polyglycerinester natürlicher Fettsäuren:

    - sowie jeweils deren Kombinationen.

Optional können jeweils zusätzlich anionische oder amphotere Tenside enthalten sein. Das N-Acylglycamin folgt bevorzugt jeweils den vorhergehend als bevorzugt definierten Strukturen.

Überraschenderweise weisen die erfindungsgemässen Mittel mit mindestens einem N-Acylglycamin und bevorzugt mindestens einem Biotensid auch in der APG-freien Ausführungsform eine mindestens gleich gute Leistung in Bezug auf Schaumverhalten, Spülleistung, Fettlösen und Rückstandsarmut auf. Mittelkettige APGs weisen ein erhebliches Irritationspotential auf. Eine bevorzugte Ausführungsvariante enthaltend mindestens ein N-Acylglycamin und bevorzugt mindestens einem Biotensid ist daher frei von Cocoglucosid oder Laurylglucosid, besonders bevorzugt ist die Ausführungsvariante, welche frei ist von APGs mit Alkylkettenlängen von C8-C18.

**Amphotere Tenside**

Mindestens eine Ausführungsform der Erfindung stellt die Zusammensetzung dar, enthaltend ein oder mehrere N-Acylglycamine und bevorzugt enthaltend ein oder mehrere Biotenside, und zusätzlich enthaltend mindestens ein amphoteres Tensid.

Bevorzugte amphotere Tenside sind Alkylaminopropionsäuren und Alkenylaminopropionsäuren, Alkyliminodipropionsäuren und Alkenyliminodipropionsäuren, Imidazolcarboxylate, Amphoacetate und Amphodipropionate, N-Alkylbetaine und N-Alkenylbetaine, Amidoamine und Amidobetaine, Amidoalkylbetaine, Aminoxide, Sulfobetaine und Sultaine. besonders bevorzugt sind die amphoteren Tenside ausgewählt aus Alkylaminopropionsäuren und Alkenylaminopropionsäuren, Alkyliminodipropionsäuren und Alkenyliminodipropionsäuren, Imidazolcarboxylate, Amphodipropionate, N-Alkylbetaine und N-Alkenylbetaine, Amidoamine und Amidobetaine, Amidoalkylbetaine, Aminoxide, Sulfobetaine und Sultaine, ganz besonders bevorzugt sind die amphoteren Tenside ausgewählt aus Alkylaminopropionsäuren und Alkenylaminopropionsäuren, Alkyliminodipropionsäuren und Alkenyliminodipropionsäuren, Imidazolcarboxylate, Amphodipropionate, N-Alkylbetaine und N-Alkenylbetaine, Amidoamine und Amidobetaine, Aminoxide, Sulfobetaine und Sultaine.

In mindestens einer bevorzugten Ausführungsform, enthält die Zusammensetzung mindestens ein N-Acylglycamid, bevorzugt ein Sunfloweroyl methyl glucamid, und bevorzugt mindestens ein Biotensid und weitere amphotere Tenside, wobei die Tenside gesättigte Fettreste, Alkanol- oder Acylgruppen, mit Kettenlängen zwischen 6-24 Kohlenstoffatomen, oder Gemische aus > 60 Gew.-% gesättigten Alkanol- oder Acylgruppen mit 8 bis18 Kohlenstoffatomen und < 40 Gew.-% ungesättigten C-18-Alkenol- oder Acylgruppen enthalten, wie sie aus Kokosöl, Palmkernöl oder Babassuöl, tierischem oder petrochemischen Usprung erhalten werden, bevorzugt ausgewählt aus den Gruppen Alkylaminopropionsäuren und Alkenylaminopropionsäuren, Alkyliminodipropionsäuren und Alkenyliminodipropionsäuren, Imidazolcarboxylate, Amphoacetate und Amphodipropionate, N-Alkylbetaine und N-Alkenylbetaine, Amidoamine und Amidobetaine, Amidoalkylbetaine, Aminoxide, Sulfobetaine und Sultaine.

Jeweils mindestens eine Ausführungsform enthält folgende bevorzugte, besonders bevorzugte oder äusserst bevorzugte Tensidkombination mit zusätzlichen amphoteren Tensiden, wobei vorzugsweise jeweils die bevorzugten N-Acylglycamine und optionalen, aber bevorzugten, Biotenside wie vorgehend beschrieben eingesetzt werden. Jeweils ganz besonders bevorzugt sind für die Kombinationen mit zusätzlichen amphoteren Tensiden die Biotenside ausgewählt aus Rhamnolipiden, Sophorolipiden und Mannosylerytrhillipiden und deren beliebige Kombinationen, insbesondere Rhamnolipide und Sophorolipide oder deren Kombination.

Mindestens eine Ausführungsform enthält die bevorzugten Tensidkombinationen:

- Mindestens ein N-Acylglycamin + bevorzugt mindestens ein Biotensid + mindestens ein Betain , z.B. Cocoamidopropylbetain, Cocobetain, u.s.w. Eine besonders bevorzugte Ausführungsform enthält zusätzlich mindestens ein weiteres Tensid ausgewählt aus Alkylsulfat, Alkylethersulfat, Aminosäuretensiden, Alkylglucosid, weiteres N-Acyl-

glycamin, Isethionaten, Tauraten, insbesondere ausgewählt aus Aminosäuretensiden, Alkylglucosid, weiteres N-Acylglycamin, Isethionaten, Tauraten.

Jeweils mindestens eine Ausführungsform enthält die besonders bevorzugten Tensidkombinationen:

Mindestens ein N-Acylglycamin + bevorzugt mindestens ein Biotensid + mindestens ein Amphoacetat, z.B. Disodium cocoamphoacetat, Eine besonders bevorzugte Ausführungsform enthält zusätzlich mindestens ein weiteres Tensid ausgewählt aus Alkylsulfat, Alkylethersulfat, Aminosäuretensiden, Alkylglucosid, weiteres N-Acylglycamin, Isethionaten, Tauraten, insbesondere ausgewählt aus Aminosäuretensiden, Alkylglucosid, weiteres N-Acylglycamin, Isethionaten, Tauraten.

- Mindestens ein N-Acylglycamin + bevorzugt mindestens ein Biotensid + mindestens ein Sultain, z.B. Capryl Sultaine, Cetyl/Lauryl/Myristyl Hydroxysultaine, Coco-Hydroxysultaine, Coco-Sultaine, Lauramidopropyl Hydroxysultaine, Lauryl Hydroxysultaine, Cocamidopropyl hydroxysultaine, Babassuamidopropyl Hydroxysultaine. Eine besonders bevorzugte Ausführungsform enthält zusätzlich mindestens ein weiteres Tensid ausgewählt aus Alkylsulfat, Alkylethersulfat, Aminosäuretensiden, Alkylglucosid, weiteres N-Acylglycamin, Isethionaten, Tauraten, insbesondere ausgewählt aus Aminosäuretensiden, Alkylglucosid, weiteres N-Acylglycamin, Isethionaten, Tauraten.

- Mindestens ein N-Acylglycamin + bevorzugt mindestens ein Biotensid + mindestens ein N-Alkylaminopropionsäure oder N-Alkenylaminopropionsäure. Eine besonders bevorzugte Ausführungsform enthält zusätzlich mindestens ein weiteres Tensid ausgewählt aus Alkylsulfat, Alkylethersulfat, Aminosäuretensiden, Alkylglucosid, weiteres N-Acylglycamin, Isethionaten, Tauraten, insbesondere ausgewählt aus Aminosäuretensiden, Alkylglucosid, weiteres N-Acylglycamin, Isethionaten, Tauraten,

- sowie jeweils deren Kombinationen.

Optional können jeweils zusätzlich anionische oder nichtionische Tenside enthalten sein. Das N-Acylglycamin folgt bevorzugt jeweils den vorhergehend als bevorzugt definierten Strukturen.

Ferner entwickeln die erfindungsgemässen Mittel mit mindestens einem N-Acylglycamin und bevorzugt mindestens einem Biotensid einen stabilen Schaum, damit kann in den Zusammensetzungen wahlweise auf Amidopropylbetaine, Alkylbetaine, Monoethanolamide oder Diethanolamide oder deren Kombinationen verzichtet werden.

Mindestens eine bevorzugte Ausführungsform der Zusammensetzung enthaltend mindestens ein N-Acylglycamin und bevorzugt mindestend ein Biotensid, ist daher Cocoamidopropylbetain (CAPB)- frei. Die erfindungsgemässen Mittel weisen eine mindestens gleich gute Leistung in Bezug auf Schaumverhalten, Spülleistung, Fettlösen und Rückstandsarmut auf. Eine besonders bevorzugte Ausführungsvariante ist frei von allen Alkylamidobetaine, äusserst bevorzugt frei von allen Betaintensiden.

Mindestend eine weitere bevorzugte Ausführungsvariante, mit mindestens einem N-Acylglycamin und bevorzugt mindestens einem Biotensid, welche besonders bevorzugt in einer fliessfähigen Form vorliegt, und ist frei von Alkylamidobetainen und Alkylethersulfaten, besonders bevorzugt frei von Betaintensiden und Alkylethersulfaten und Alkylsulfaten. Eine weitere bevorzugte Ausführungsvariante ist weiterhin frei von Mono- und Diethanolamiden, wie Cocamid DEA, Cocamid MEA u.a.

**Weitere Inhaltsstoffe**

Die Zusammensetzung enthaltend ein oder mehrere N-Acylglycamine und bevorzugt enthaltend ein oder mehrere Biotenside, kann mit weiteren Inhaltsstoffen kombiniert werden.

Jeweils mindestens eine Ausführungsform enthält folgende bevorzugte Kombinationen mit zusätzlichen Inhaltsstoffen, wobei vorzugsweise jeweils die bevorzugten N-Acylglycamine und optionalen, aber bevorzugten, Biotenside wie vorgehend beschrieben eingesetzt werden. Jeweils ganz besonders bevorzugt sind für die Kombinationen mit zusätzlichen amphoteren Tensiden die Biotenside ausgewählt aus Rhamnolipiden, Sophorolipiden und Mannosylerythrillipiden und deren beliebige Kombinationen, insbesondere Rhamnolipide und Sophorolipide oder deren Kombination.

- Mindestens ein N-Acylglycamin + bevorzugt mindestens ein Biotensid + mindestens eine Fettsäure oder Fettalkohol, z.B. Stearinsäure, Cetylalkohol, Cetearyl alkohol, Myristinsäure, Palmitinsäure, Stearylalkohol, Isostearyl Alcohol, , etc., bevorzugt abgeleitet aus eine C-18-Pflanzenöl., z.B. Oleoylalkohol, Brassica-Alkohol, Ölsäure, Arachidyl Alcohol, Behenyl alkohol und deren Kombinationen

- Mindestens ein N-Acylglycamin + bevorzugt mindestens ein Biotensid + mindestens ein Pflanzenöl oder Fettsäureglycerid, z.B. Kokosöl, Jojobaöl, Glycerylstearat, Glyceryl caprylate/caprate etc., bevorzugt abgeleitet aus eine C-18-Pflanzenöl., z.B. Sonnenblumenöl, Glyceryloleat, Olivenöl, Rapsöl, Rapsglyceride, Sweet almond oil, und deren Kombinationen

- Mindestens ein N-Acylglycamin + bevorzugt mindestens ein Biotensid + mindestens ein Wachs oder Butter, z.B. Bienenwachs, Carnaubawachs., Butyrospremum parkii butter, Theobroma cacao seed butter, Mango butter, Candelilla Wachs, Oryza sativa cera, bevorzugt abgeleitet aus eine C-18-Pflanzenöl., z.B. Sunflower seed wax, hydriertes Sonnenblumenöl, hydriertes Rapsöl, hydrogenated vegetable oil, und deren Kombinationen.

- Mindestens ein N-Acylglycamin + bevorzugt mindestens ein Biotensid + mindestens ein Saccharid, bevorzugt ausgewählt aus Amylose, Amylopectin, Guar und Guarderivate, Sorbitol, Maltodextrin, Isomalt, Xylitol, Glykogen, Callose, Cellulose (auch mikrokristallin) oder Cellulosederivate, Pectin, Carrageenan, Xanthane, Dextrane, Dextrin, Tunicin, Fructane, Inulin, Alginate, Curdlane, Gellan, Guar, Glucane, Chitosane, Chitine, Chrondroitine, Heparine, Dextrane, Pullulane, Welane, Rhamsane, Keratane, Dermatane, Gellane, Schizophyllane, Agar, Arabisch Gummi, Tragant, Fenugreek Gum, Locust been gum, Tara gum, Monosaccharide, Oligosacchariden; Stärke, z.B. Tapioka-, Reis-, Kartoffel- oder Maisstärke, modifizierte Stärken, Mehle z.B. Weizenmehl, Roggenmehl, Johannisbrotkernmehl, Reismehl sowie Derivate dieser Verbindungen oder Mischungen dieser Verbindungen sowie deren Derivate.

Besonders bevorzugt werden diese Kombinationen in festen Produktformen eingesetzt.

Cellulosederivate umfassen z.B.Ethylcellulose, Propylcellulose, Methylethylcellulose, Carboxymethylcellulose, Ethylcarboxymethylcellulose, Hydroxyethylcellulose, Hydroxypropylcellulose, Carboxymethylhydroxyethylcellulose, Methylhydroxyethylcellulose, Hydroxypropylmethylcellulose, Ethylhydroxyethylcellulose, Methylethylhydroxyethylcellulose, quaternisierte Cellulose, quaternisierte Cellulosederivate, Amin-modifizierte Cellulose, Amin-modifizierte Cellulosederivate oder Mischungen dieser Verbindungen. Modifizierte Stärken sind z.B. Distarchphosphate, Hydroxypropylstarchphosphate. Monosaccharide und deren Derivate umfassen Allose, Altrose, Arabinose, Fructose, Erythrose, Galactose, Glucose, Gulose, Idose, Lyxose, Mannose, Ribose, Talose, Threose, oder Xylose, oder deren Derivaten wie Desoxymonosaccharide, z.B. Desoxyribose oder Rhamnose, Carboxyzucker umfassen z.B. Glucuronsäure oder Aminozucker z.B. Glucosamin. Oligosacchariden umfassen z.B. Saccharose, Lactose, Trehalose, Maltose, Cellobiose, Gentiobiose, Raffinose. Fructane umfassen 1-Kestosen: Inulin, 6-Kestosen, z.B. Levane (bakteriell) oder Phleine, Neokestosen Monosaccharide und deren Derivate umfassen Allose, Altrose, Arabinose, Fructose, Erythrose, Galactose, Glucose, Gulose, Idose, Lyxose, Mannose, Ribose, Talose, Threose, oder Xylose, und deren Derivaten wie Desoxymonosaccharide, z.B. Desoxyribose oder Rhamnose, und Carboxyzucker umfassen z.B. Glucuronsäure oder Aminozucker z.B. Glucosamin. Oligosacchariden bestehen aus 2-20 Saccharideinheiten und umfassen z.B. Saccharose, Lactose, Trehalose, Maltose, Cellobiose, Gentiobiose, Raffinose.

Besonders bevorzugt werden diese Kombinationen in festen Produktformen eingesetzt.

- Mindestens ein Biotensid + bevorzugt mindestens ein N-Acylglycamin + mindestens ein Saccharid, bevorzugt ausgewählt aus Amylose, Amylopectin, Guar und Guarderivate, Sorbitol, Maltodextrin, Isomalt, Xylitol, Glykogen, Callose, Cellulose (auch mikrokristallin) oder Cellulosederivate, Pectin, Carrageenan, Xanthane, Dextrane, Dextrin, Tunicin, Fructane, Inulin, Alginate, Curdlane, Gellan, Guar, Glucane, Chitosane, Chitine, Chrondroitine, Heparine, Dextrane, Pullulane, Welane, Rhamsane, Keratane, Dermatane, Gellane, Schizophyllane, Agar, Arabisch Gummi, Tragant, Fenugreek Gum, Locust been gum, Tara gum, Monosaccharide, Oligosacchariden; Stärke, z.B. Tapioka-, Reis-, Kartoffel- oder Maisstärke, modifizierte Stärken, Mehle z.B. Weizenmehl, Roggenmehl, Johannisbrotkernmehl, Reismehl sowie Derivate dieser Verbindungen oder Mischungen dieser Verbindungen sowie deren Derivate.

Besonders bevorzugt werden diese Kombinationen in festen Produktformen eingesetzt.

- Mindestens ein N-Acylglycamin + bevorzugt mindestens ein Biotensid + mindestens ein Alkohol oder Fettsäureester oder - ether, insbesondere Propylenglykol, Butylenglykol, Pentylenglycol, Propandiol, Caprylic/Capric Triglyceride, Isopropyl myristate, Propylenglykol Di (Caprylate/Caprate), Ethanol, Glycerin, Isopropanol, Propanol, Dipropylenglykol, Dicaprylyl ether, besonders bevorzugt Glycerin, Ethanol, Propylenglykol, Butylenglykol, und deren Kombinationen, insbesondere zusätzlich auch mit Wasser.

- Mindestens ein N-Acylglycamin + bevorzugt mindestens ein Biotensid + mindestens ein Enzym, bevorzugt ausgewählt aus den Gruppen der Proteasen, Amylasen, Cellulasen, Lipasen, Pektat Lyase, Mannase, und deren Kombinationen, besonders bevorzugt aus den Gruppen der Proteasen und Amylasen und deren Kombinationen.

- Mindestens ein N-Acylglycamin + bevorzugt mindestens ein Biotensid + mindestens ein Komplexbildner, bevorzugt

ausgewählt aus Tetrasodium Glutamate Diacetate (GLDA), Tetraacetylethylendiamin (TAED), Methylglycindiessigsäure MGDA, Carboxymethyl Inulin, Zitronensäure, Milchsäure, Natrium gluconat, Ethylendiamintetraacetat (EDTA), Dicarboxymethyl Alaninate, Polyasparaginsäure, Gluconolacton, Bernsteinsäurederivate, insbesondere Iminodibernsteinsäure (IDS), oder Ethylendiamindibernsteinsäure EDDS , jeweils als Salz, insbesondere als Alkalisalze oder in der Säureform, sowie deren Kombinationen

- Mindestens ein N-Acylglycamin + bevorzugt mindestens ein Biotensid + mindestens ein Polymer, z.B. Xanthan gum, Guar gum, Guar hydroxypropyltrimonium chlorid, Sclerotiumgum, und deren Kombinationen.
- Mindestens ein N-Acylglycamin + bevorzugt mindestens ein Biotensid + mindestens ein Pflanzenextrakt oder Aromastoff, z.B. Aloe Barbadensis Leaf Juice Powder, Citrus aurantium dulcis peel oil, Camelia sinesis (green tea) extract, Rosmarinus Officinalis Leaf, Peppermint oil, Salvia Officinalis Leaf Powder, Eucalyptus Globulus Leaf Oil, Chamomilla Recutita Flower Extract, und deren Kombinationen.
- Mindestens ein N-Acylglycamin + bevorzugt mindestens ein Biotensid + mindestens ein Konservierungsmittel oder Booster, bevorzugt Benzoesäure, Natrium oder Kalium Benzoat, Potassium Sorbate, Phenoxyethanol, Benzyl alcohol, Sodium Levulinate, Caprylyl Glycol, Sorbic Acid, Lactic acid, und deren Kombinationen.
- Mindestens ein N-Acylglycamin + bevorzugt mindestens ein Biotensid + mindestens ein Polyvinylalkohol.

In einer bevorzugten Ausführungsform liegen die Produkte in einer wasserlöslichen Folie vor, bevorzugt in einem Polyvinylalkohol-Derivat.

**Spezifische Zusammensetzung - Tenside K**

Die spezifische Ausführungsform der erfinderischen Zusammensetzung enthaltend mindestens ein N-Acylglycamin, und bevorzugt zusätzlich enthaltend das mindestens eine Biotensid, enthält zusätzlich ein oder mehrere Tenside ("Tenside K") ausgewählt aus den kationischen Tensiden der Formel oder Amidoamine oder Esteramine, insbesondere den sekundären oder tertiären Amido- oder Esteraminen. Diese Zusammensetzungen eignen sich insbesondere für konditionierende Produkte (Haut- und Haarpflege) und für Textilpflegeprodukte, z.B. Weichspüler. Diese erfinderischen Zusammensetzungen lassen sich gut formulieren und zeigen synergistische Effekte mit den Tensiden K. Die N-Acylglycamine und gegebenenfalls die Biotenside unterstützen den Emulgiervorgang. Es werden stabile Zusammensetzungen erhalten. Dank der erfinderischen Zusammensetzung enthaltend N-Acylglycamine und bevorzugt Biotenside entstehen keine unerwünschten Rückstände (z.B. kein Build-up). Die Komponenten, insbesondere die lipophilen Inhaltsstoffe, lassen sich gut mischen. Vorteilhaft ist die zudem gute Rückbenetzung der Textilien. Die Zusammensetzungen sind speziell auch für hartes Wasser geeignet. Man erhält so einen guten Weichgriff von Haar und Textilien, eine gute Kämmbarkeit von Haar. Durch die erfinderische Zusammensetzung werden umweltschonende Produkte mit einem geringen $CO_2$-Fussabdruck erhalten, die sich selbst parfümfrei formulieren lassen. Die festen Ausführungsformen der Zusammensetzung zeigen zudem eine überraschend gute Adhäsion an den Träger, insbesondere an die Polysaccharide. Es wird eine gute Wasserlöslichkeit, insbesondere bei festen Ausführungsformen, gute Mischbarkeit der Komponenten, geringer Rückstand und zusätzliche Stabilisierung erzielt. Diese Zusammensetzungen mit Tensiden K wirken zudem antistatisch.

**Kationischen Tenside**

Das mindestens eine kationische Tenside K in der Zusammensetzung ist bevorzugt eine quartäre Ammoniumverbindungen ("Quats"), oder ein protoniertes Amin, z.B. ein Amidoamin oder ein Esteramin, mit der generellen Formel

(VII) $[N+(RI)(RII)(RIII)(RIV)]_y X-$

mit RI, RII, Rill, RIV gleichen oder verschiedenen Resten mit 1 bis 30 Kohlenstoffatomen, gegebenenfalls enthalten die Reste ein oder mehrere Heteroatome, bevorzugt enthält mindestens einer der Reste RI bis Rill einen Ester oder eine Amidgruppe; bevorzugter enthalten zwei der Reste RI bis Rill eine Ester- oder eine Amidgruppe; RIV ist Wasserstoff oder ein organischer Rest mit 1 bis 30 Kohlenstoffatomen, gegebenenfalls mit einem oder mehreren Heteroatome. X ist ein Anion, y gibt die Valenz von X an;

(VII i) Quat: Mit RI, RII, Rill, RIV gleichen oder verschiedenen Resten mit 1 bis 30 Kohlenstoffatomen, gegebenenfalls

enthalten die Reste ein oder mehrere Heteroatome, z.B. N oder O, bevorzugt enthält mindestens einer der Reste RI bis RiII einen Ester oder eine Amidgruppe; RIV ist ein organischer Rest mit 1 bis 30 Kohlenstoffatomen, gegebenenfalls enthalten die Reste ein oder mehrere Heteroatome, z.B. N oder O.
Bevorzugte sind die Quats ausgewählt aus den Glucoquats, Amidoaminquats, Amidoesterquats oder Esterquats, insbesondere Diesterquats, Betain Esterquats, Amide Esterquats, Choline Esterquats.

X ist ein Anion, y gibt die Valenz von X an;

(VII ii) protoniertes Amin: mit RI, RII, RIII, gleichen oder verschiedenen Resten mit 1 bis 30 Kohlenstoffatomen, gegebenenfalls enthalten die Reste ein oder mehrere Heteroatome, bevorzugt enthält mindestens einer der Reste RI bis RiII einen Ester oder eine Amidgruppe;; und mit RIV einem Wasserstoffatom, X ist ein Anion, y gibt die Valenz von X an.

(VII iii) Es sind auch Strukturen mit mehreren positiven Ladungen möglich, z.B. bei denen einer der Reste RI-RVI zwei Stickstoffatome verbindet, zur Illustration, jedoch nicht limitierend, schliesst die Erfindung zum Beispiel den Vertreter ein, wobei einer der Reste RI bis RIV - $(RI')_xN^+(RI')(RII')RIII')$ darstellen kann wobei RII', RIII' und RVI' unabhängig voneinander gleiche oder verschieden Reste darstellt wie für RII, Rill, RIV, definiert, und RI' einem Rest, der in diesem Beispiel 2 Stickstoffe verbindet, z.B. - $(CH_2)_x$- mit x = 6.

Als Gegenanionen X sind bevorzugt Halogenide, insbesondere Chlorid, Methosulfat, Lactat, Citrat, Glutamat, Glykolat, besonders bevorzugt Chlorid oder Methosulfat.

Mindestens eine bevorzugte Ausführungsform ist die Zusammensetzung enthaltend mindestens ein N-Acylglycamin und zusätzlich bevorzugt ein Biotensid, und das mindestens eine kationische Tensid K ausgewählt aus den Gruppen der Esterquats, Amidoaminquats, protonierten Amidoamine, oder Amidoesterquats.

Erfinderisch bevorzugte kationische Tenside sind die Esterquats bevorzugt nach Formel (VIIc-e), Amidoaminquats, protonierte Amidoamine, protonierte Amidoester, Glucoquats, oder Amidoesterquats. Bevorzugt trägt mindestens einer der Rest RI-RIV eine Amidgruppe oder mindestens einer der Reste RI-RIV eine Estergruppe, bevorzugter sind zwei der Reste RI-RIV Ester- oder Amidgruppen. Besonders bevorzugt tragen die vorstehenden Tenside in mindestens einer der Amid- oder Esterfunktionen einen Fettsäureacylrest, insbesondere abgeleitet sind von einem C18-Pflanzenöl. Ganz besonders bevorzugt ist dabei mindestens einer der Fettsäureacylreste der Amid- oder Esterfunktionen ein- oder mehrfach ungesättigt. Bevorzugt werden erfinderisch die Esterquats und Amidoaminquats, sowie die protonierten Amidoester und Amidoamine, insbesondere die Esterquats und protonierten Amidoester, aufgrund der guten Umwelteigenschaften im Vergleich zu anderen kationischen Tensiden.

Gegenstand des Patents sind Zusammensetzungen enthaltend mindestens ein N-Acylglycamin (vorzugsweise wie vorstehend als bevorzugt bezeichnet) und ein oder mehrere Tenside K ("Tenside K") ausgewählt aus den kationischen Tensiden der Formeln (VII), (VIIi), (VIIii) und (VIIiii), oder den sekundären oder tertiären Amidoaminen oder den sekundären oder tertiären Esteraminen, , wobei die Tenside K vorzugsweise aus mindestens einem C-18-Pflanzenöle abgeleitet sind,

und in einer bevorzugten Ausführungsform zusätzlich enthaltend mindestens ein Biotensid, wobei die Tenside K vorzugsweise aus mindestens einem C-18-Pflanzenöle abgeleitet sind,
sowie deren jeweilige Verwendung als Weichspüler, Waschadditive, Textilpflege- und -reinigungsprodukte einschliesslich Teppichen und Polster, Waschmittel, Haarspülungen, Konditioner, Shampoos, Haar- und Hautreinigungsprodukte oder Haar- und Hautpflegeprodukte, Desinfektionsmittel, Algenentfernungsmittel, Reinigungs- und Pflegeflüssigkeiten für Feuchttücher, und Zusammensetzungen zur Ölförderung, Agroformulierungen.

Bevorzugte Verwendungen sind Weichspüler, Waschadditive, Textilpflege- und -reinigungsprodukte einschliesslich Teppichen und Polster, Waschmittel, Haarspülungen, Konditioner, Shampoos, Haar- und Hautreinigungsprodukte oder Haar- und Hautpflegeprodukte, Desinfektionsmittel, Reinigungs- und Pflegeflüssigkeiten für Feuchttücher.

Die erfinderischen Zusammensetzungen enthaltend mindestens ein Tensid K liegen in mindestens einer Ausführungsform als fliessfähige Produkte vor.

In mindestens einer weiteren Ausführungform liegen die erfinderischen Zusammensetzungen, in der festen Ausführungsform vor. Eine bevorzugte Ausführungsform der Erfindung enthaltend mindestens ein Tensid K sind feste Conditioner für Haare oder feste Weichspüler für Textilien.

Gegenstand des Patents sind weiterhin die Verfahren zu deren Herstellung und die Verfahren zur Anwendung der erfinderischen Zusammensetzungen enthaltend mindestens ein Tensid K.

Aufgrund der guten Formulierbarkeit und Stabilität von Zusammensetzungen enthaltend mindestens ein N-Acylglycamin, (vorzugsweise wie vorstehend als bevorzugt definiert), insbesondere bevorzugt abgeleitet von einem C-18-Pflanzenöl, und in einer alternativen Ausführungsform weiterhin bevorzugt mindesten ein Biotensid, enthaltend zusätzlich Tenside K, eignen sich diese Zusammensetzungen auch als Desinfektionsmittel oder Biozidformulierungen mit kationischen Wirkstoffe, z.B. Quartären Ammoniumsalzen.

Generell Tenside K

Bevorzugt ist die Zusammensetzung, dadurch gekennzeichnet, dass mindestens einer der Fettreste der Tenside K, also mindestens einer der Fettsäureacylreste oder gegebenenfalls mindestens einer der Fettalkoholreste der Ester- oder Amidgruppe, von einer Ölsäure-, einer Linolsäure- einer Linolensäure-, einer konjugierten Linolsäure- (CLA)-, einer Stearin-, einer Palmitin-, einer Erucasäure-, einer Gadolein-, einer Nervonsäure-, einer Behensäure, einer Arachidonsäure-, oder einer Lignocerinsäure, oder dem entsprechenden Fettalkohol, d.h. Oleylalkohol, Linolyl, Linolenyl, u.s.w. abgeleitet ist.

Die Fettreste können gegebenenfalls jeweils auch hydrogeniert, oder teilweise hydrogeniert sein.

Erfinderisch besonders bevorzugt enthalten die Tenside K, die eingesetzten Quats oder protonierten Amine oder die Ester- oder Amidoamine, mindestens einen Fettrest abgeleitet von der Ölsäure, Linolsäure, Linolensäure, konjugierte Linolsäure (CLA), welche jeweils besonders bevorzugt von C-18-Pflanzenöle abgeleitet sind.

Bevorzugt liegt mindestens ein Produktgemisch von mindestens einer der Tenside K, den kationischen Tensid oder den Ester- oder Amidoaminen, in der Zusammensetzung vor, welches sich durch Kettenlängen oder Sättigungsgraden des oder der Fettreste unterscheiden, besonders bevorzugt ist der Fettrest abgeleitet von einem C-18-Pflanzenöl und weist ganz besonders bevorzugt einen Anteil an Fettresten mit 18 und mehr Kohlenstoffatomen über 60 Gew.-%, besonders bevorzugt über 72 Gew.- % und ganz besonders bevorzugt von über 77 Gew.-% aufweist, Gew.-% bezogen auf das mindestens eine Produktgemisch. Fettrest kann hierbei einen Fettalkoholrest darstellen, zum Beispiel ein Gemisch aus Behenyl und Arachidyl-; Fettrest kann auch ein Fettsäureacylrest darstellen, z.B. ein Gemisch aus Oleoyl- und Linoleoyl.

Das Produktgemisch von mindestens einem der Tenside K wird dabei bevorzugt erhalten aus der Umsetzung von Fettderivaten, welches eine Fettverteilung gemäss einem nativen Pflanzenöl aufweisen, bevorzugt gemäss einem C-18-Pflanzenöl, welches einen Anteil an Fettsäuren mit 18 und mehr Kohlenstoffatomen über 60 Gew.-%, besonders bevorzugt über 72 Gew.-% und ganz besonders bevorzugt von über 77 Gew.-% aufweist und besonders bevorzugt liegt dabei der Anteil an ungesättigten Fettsäureacylresten über 55 Gew.-%, vorzugsweise über 65 Gew.-% und besonders bevorzugt über 72 Gew.-% liegt, Gew.-% bezogen auf das Pflanzenöl.

Es können auch zwei Produktgemische von mindestens einem der Tenside K aus der Umsetzung einer Mischung von mehr als einem Pflanzenöl, bevorzugt mehr als ein Pflanzenöl, wobei das mindestens eine Pflanzenöl bevorzugt ein C-18-Pflanzenöl ist, zum Beispiel Sonnenblumenöl und Rapsöl bzw. deren Fettsäurederivatgemische, stammen.

Bevorzugt enthält die Zusammensetzung mindestens ein Produktgemisch von mindestens einem der Tenside K mit einer Verteilung an Fettresten (Fettsäureacyl- oder Fettalkoholreste, bevorzugt Fettsäureacylreste) mit 18 und mehr Kohlenstoffatomen über 60 Gew.-%, besonders bevorzugt über 72 Gew.-% und ganz besonders bevorzugt von über 77 Gew.-% und äusserst bevorzugt liegt dabei der Anteil an ungesättigten Fettresten über 55 Gew.-%, vorzugsweise über 65 Gew.-% und besonders bevorzugt über 72 Gew.-% liegt, Gew.-% bezogen auf das jeweilige Produktgemischs, insbesondere abgeleitet von einem C-18-Pflanzenöl.

Besonders bevorzugt werden C-18-Pflanzenöle mit einem hohen Anteil an Ölsäure eingesetzt. Bevorzugt enthält das mindestens eine Produktgemisch einen Anteil an Ölsäureresten von > 40 Gew.-%, bevorzugter > 49 Gew.-% besonders bevorzugt > 57 Gew.-%, bezogen auf das jeweilige Produktgemisch des kationischen Tensids.

Die Fettreste des mindestens einen Tensids K sind bevorzugt abgeleitet von Fettsäurederivaten aus einem C-18-Pflanzenöl oder einem C-18- Pflanzenöl, insbesondere bevorzugt aus einem C-18-Pflanzenöl der Pflanzen, wie vorstehend als bevorzugt und besonders bevorzugt etc. bezeichnet, äusserst bevorzugt sind die Fettreste, insbesondere die Fettsäureacylreste, aus Sonnenblumenöl, oder einem Rapsöl, z.B. Canola, Low erucic rapeseed oil, oder deren Mischungen abgeleitet.

In einer alternativen Zusammensetzung können die Fettreste des mindestens einen Tensids K von einem der anderen C-18-Pflanzenöle, wie vorgehend beschrieben, abgeleitet werden. Die C-18-Pflanzenöle können auch teilweise oder vollständig hydrogeniert werden.

Die Zusammensetzungen enthaltend mindestens eines der Tenside K enthalten in einer bevorzugten Ausführungsform weitere Fettderivate, welche aus dem Herstellungsprozess stammen können oder der Zusammensetzung zugegeben werden. Bevorzugt sind diese weiteren Fettderivate ausgewählt aus Fettalkoholen, Fettsäuren, Mono-, Di- oder Triglyceride, Fettsäureester und jeweils deren Salze. Besonders bevorzugt liegen die Fettderivate als Gemische von Fettderivaten mit vor, deren Verteilung an Kettenlängen und Sättigungsgraden dem in der Zusammensetzung enthaltenen Produktgemisch des Tensids K entspricht.

Das ein oder die mehreren Produktgemische der Tenside K, insbesondere die Esterquats, können bevorzugt

Mono-, Di- oder Triglyceride des entsprechenden Pflanzenöls enthalten können. Das Mengenverhältnis der Glyceride zu dem entsprechenden Tensid kann von 0,01 : 1 bis 1:1 Glycerid: Tensid betragen, vorzugsweise liegt es zwischen 1: 10 und 1: 1 und, besonders bevorzugt zwischen 1: 4 und 1: 1, bevorzugt vorliegend als Gemisch von Fettsäurederivaten wie oben beschrieben.

Das ein oder die mehreren Produktgemische der Tenside K, insbesondere der Betainquats, können bevorzugt Fettalkohole enthalten. Das Mengenverhältnis der Fettalkohole zu dem entsprechenden Tensid kann von 0,1: 10 bis 10:0,1 Fettalkohol: Tensid betragen, vorzugsweise liegt es zwischen 8: 2 und 6: 4 und, besonders bevorzugt zwischen 1: 4 und 1: 1. bevorzugt vorliegend als Gemisch von Fettsäurederivaten wie oben beschrieben.

Die erfinderischen Zusammensetzungen mit mindestens einem der Tenside K enthalten zusätzlich bevorzugt Saccharide, bevorzugt ausgewählt aus der vorhergehend beschriebenen Gruppe, insbesondere bevorzugt ausgewählt aus, Guar, Maltodextrin, Cellulose, Cellulosederivate, Pectin, Carrageenan, Xanthane, Dextrane, Curdlane, Chitosane, Chitine, Sacharose, Getreidemehl, Stärke, z.B. Roggenmehl, Tapioka-, Reis- oder Maisstärke, Weizenmehl, ggf. kationisch modifizierte Polysaccharide.

Die erfinderischen Zusammensetzungen mit mindestens einem der Tenside K enthalten zusätzlich bevorzugt protische Lösungsmittel enthalten, insbesondere bevorzugt ausgewählt aus Wasser, Alkoholen, Glykolen und Glycerin, ganz besonders bevorzugt ausgewählt aus Wasser, Propylenglykol, Ethanol, Isopropylalkohol, Glycerin. Äusserst bevorzugt enthält die Zusammensetzung Propylenglykol.

Die erfinderischen Zusammensetzungen mit mindestens einem der Tenside K enthalten zusätzlich bevorzugt einen Komplexbildner, bevorzugt gemäss vorhergehender Liste, besonders bevorzugt ausgewählt aus Zitronensäure, Milchsäure,

Reaktion zu Esterquats

Die Esterquats resultieren aus der Umsetzung eines oder mehrerer Öle (Triglyceriden) oder ein oder mehreren Fettsäurederivaten, z.B. Säurehalogenide, Fettsäurealkylester, Fettsäureglyceride, oder Fettsäuren, mit einem mono-, di- oder Tri- Alkanolamin, bevorzugt mit einem Alkanrest, bzw. Alkanolreste mit 1-10 Kohlenstoffatomen, besonders bevorzugt mit 1-6 Kohlenstoffatomen, äusserst bevorzugt mit 2 Kohlenstoffatomen (Mono-, Di- oder Tri-Ethanolamin, z.B. Triethanolamin (TEA), Methyldiethanolamin (MDEA)).

Bevorzugt wird ein Gemisch aus Fettsäurederivaten oder ein Öl eingesetzt, insbesondere ein C-18-Pflanzenölen oder von diesem abgeleitet, so dass die erhaltenen Produktgemische die Fettsäureverteilungen der eingesetzten Fettsäurederivate, bzw. des Öls, aufweisen. In der Regel wird zudem ein Produktgemisch aus Mono-, Di- und Triestern erhalten, erfinderisch bevorzugt enthält die Zusammensetzung mindestens einen Diester.

Es können für die Erfindung ebenfalls die erhaltenen Esteramine, Mono-, Di- oder die Triester oder deren Gemische, insbesondere in deren protonierter Form, eingesetzt werden oder zusätzlich in der Zusammensetzung enthalten sein, besonders bevorzugt als Produktgemisch von unterschiedlichen Fettsäureacylresten, insbesondere abgeleitet von C-18-Pflanzenölen. Exemplarisch, nicht limitierend, wird eine Struktur eines Esteramins in (VIIa) bzw. dessen protonierter Form (VIIb) dargestellt, Bsp. Triethanolamin und Diester

(VIIa) $[N(C_2H_4OCOR^{38})(C_2H_4OCOR^{39})(C_2H_4OH)]$

Bsp. Esteramin (hier: Diester aus Triethanolamin)

(VIIb) $[N+(C_2H_4OCOR^{38})(C_2H_4OCOR^{39})(H)(C_2H_4OH)]X^-$

Bsp. Protoniertes Esteramin (hier: Diester aus Triethanolamin)
mit $COR^{38}$ und $COR^{39}$ wie unten definiert

Das erhaltene Produktgemisch der Esteramine kann anschliessend zu den Esterquats alkyliert werden (mit einem Alkylrest von 1-10 Kohlenstoffatomen, bevorzugt 1-4, besonders bevorzugt 1 = Methyl). Fettsäureedukte oder -nebenkomponenten, insbesondere Glyceride, können dabei in dem Produktgemisch verbleiben. Bevorzugte Produktgemische resultieren z.B. nach EP19835193. Je nach Alkylierungsmittel, die der Fachperson bekannt sind, und eventuellen Folgeschritten erhält man Salze mit X-, z.B. Chloride, Sulfate, Dimethylsulfate u.s.w. Bevorzugt ist das Gegenion X- ausgewählt aus Chlorid, Bromid, Methosulfat, Tosylat, Esylate, Phosphat, Sulfat, Nitrat, Hydrogensulfat, Lactat und Citrat.

Exemplarisch, nicht limitierend, sind Vertreter der Esterquats aus der Reaktion mit Triethanolamin und anschliessender Methylierung in Formel (VIIc-e) dargestellt:

(VIIc) $[N^+(C_2H_4OCOR^{37})(CH_3)(C_2H_4OH)_2]X^-$ Monoester

(VIId) $[N^+(C_2H_4OCOR^{38})(C_2H_4OCOR^{39})(CH_3)(C_2H_4OH)]X^-$ Diester

(VIIe) $[N^+(C_2H_4OCOR^{40})(C_2H_4OCOR^{41})(C_2H_4OCOR^{42})(CH_3)]X^-$ Triester

$-COR^{37}-$ bis $-COR^{42}$ sind unabhängig voneinander gesättigte oder ungesättigte, verzweigte oder lineare, gegebenenfalls hydroxy- oder alkoxysubstituierte Fettsäureacylreste mit 6 bis 30 Kohlenstoffatomen, bevorzugt stammt mindestens einer der Fettsäureacylreste in dem Produktgemisch aus einer natürlichen Fett oder Öl, insbesondere einem C-18-Pflanzenöl, besonders bevorzugt stammen die Fettsäureacylreste aus einem oder mehreren Fetten oder Ölen, insbesondere C-18-Pflanzenölen.

Erfinderisch geeignete Vertreter für Esterquats sind beispielsweise, jeweils exemplarisch hier Diesterquats mit Methosulfat als Anion, Dialkanoylethyl-hydroxyethyl-methyl-ammonium methosulfate, Dialkenoylethyl-hydroxyethyl-methyl-ammonium methosulfate, und deren Mischungen, Ditallowoylethyl Hydroxyethylmonium Methosulfate, auch hydrogeniert, Distearoylethyl Hydroxyethylmonium Methosulfate, Dihydrogenated Palmoylethylhydroxyethylmonium Methosulfate, Palmoylethylhydroxyethylmonium Methosulfate, Dicocooylethyl hydroxyethylmonium methosulfate Disunfloweroylethyl hydroxyethylmonium methosulfate, Dicanolaoylethyl hydroxyethylmonium methosulfate, Dioleoylethyl Hydroxyethylmonium methosulfate, u.s.w., exemplarisch mit den bevorzugten Esterquats: Disunfloweroylethyl hydroxyethylmonium methosulfate, Dicanolaoylethyl hydroxyethylmonium methosulfate, Dioleoylethyl Hydroxyethylmonium methosulfate,

Geeignet bzw. bevorzugt sind auch die entsprechenden Vertreter mit Gegenion X- wie oben definiert, z.B. Disunfloweroylethyl Dimonium Chloride u.s.w.

In mindestens einer Ausführungsform enthält die Zusammensetzung Esterquats oder Amidoquats mit Alkylresten. Ein Beispiel für einen erfindungsgemäss geeignete Vertreter sind z.B. Mono-, Di-, und Triester mit entsprechend 3, 2 oder einer Alkylgruppe am Stickstoffatom. Exemplarisch ist die Strukturformel eines Diesters dargestellt

$$[N^+(C_2H_4OCOR^{43})(C_2H_4OCOR^{44})(CH_3)_2]X-$$

$-COR^{43}-$ bis $-COR^{44}$ sind unabhängig voneinander gesättigte oder ungesättigte, verzweigte oder lineare, gegebenenfalls hydroxy- oder alkoxysubstituierte Fettsäureacylreste mit 6 bis 30 Kohlenstoffatomen, bevorzugt stammt mindestens einer der Fettsäureacylreste in dem Produktgemisch aus einer natürlichen Fett oder Öl, insbesondere einem C-18-Pflanzenöl, besonders bevorzugt stammen die Fettsäureacylreste aus einem oder mehreren Fetten oder Ölen, insbesondere C-18-Pflanzenölen. Mindestens eine bevorzugte Ausführungsform der Zusammensetzung enthält Esterquats oder Amidoquats mit Alkylresten, wobei mindestens eine der Acylgruppen ungesättigt vorliegt, bevorzugt stammt dieser mindestens eine Acylrest aus einer natürlichen Fett oder Öl, insbesondere einem C-18-Pflanzenöl, besonders bevorzugt stammen die Fettsäureacylreste aus einem oder mehreren Fetten oder Ölen, insbesondere C-18-Pflanzenölen.

Mindestens eine bevorzugte Ausführungsform der Zusammensetzung enthält Esterquats oder Amidoquats mit Alkylresten, wobei mehr als eine der Acylgruppen ungesättigt vorliegt, bevorzugt liegen alle der Acylgruppen ungesättigt vor; und bevorzugt stammen diese Acylreste aus einer natürlichen Fett oder Öl, insbesondere einem C-18-Pflanzenöl, besonders bevorzugt stammen die Acylreste aus einem oder mehreren Fetten oder Ölen, insbesondere C-18-Pflanzenölen.

Besonders bevorzugt enthält die Zusammensetzung mindestens einen Esterquat der Formel (VIIf)

(VIIf) $[N^+(C_2H_4OCOR^{38})(C_2H_4OCOR^{39})(CH_3)(C_2H_4OH)]X^-$ Diester

wobei $COR^{38}$ und $-COR^{39}$ Fettsäureacylreste darstellen und von C-18-Pflanzenölen abgeleitet sind und eine Fettsäureacylverteilung von Fettsäuren mit 18 und mehr Kohlenstoffatomen über 60 Gew.-%, besonders bevorzugt über 72 Gew.-% und ganz besonders bevorzugt von über 77 Gew.-% liegt und bevorzugt der Anteil an ungesättigten Fettsäureacylresten über 55 Gew.-%, vorzugsweise über 65 Gew.-% und besonders bevorzugt über 72 Gew.-% liegt.

Es gelten vorzugsweise die Fettsäureverteilungen wie bei den C-18-Pflanzenölen als bevorzugt angegeben. Gegebenenfalls, weniger bevorzugt, können die Fettsäureacylreste abgeleitet von C-18-Pflanzenölen in diesem Fall auch hydrogeniert vorliegen. In diesem Fall gelten vorzugsweise die Fettsäureverteilungen für die Kettenlängen wie bei den C-18-Pflanzenölen als bevorzugt angegeben.

Erfinderisch besonders bevorzugt sind Zusammensetzungen enthaltend mindestens ein Produktgemisch aus Esterquats und/ oder Esteraminen abgeleitet von C-18-Pflanzenölen, bevorzugt enthaltend Fettsäuracylreste in Gewichtsverhältnissen wie als bevorzugt definiert für die Fettsäuren der C-18-Pflanzenöle, bezogen auf die Fettsäureacylreste des Produktgemisch, ganz besonders bevorzugt enthält die Zusammensetzung ein oder mehrere Esterquats oder

Esteramine mit Fettsäureacylresten, die vollständig aus C-18-Pflanzenölen abgeleitet sind. Die Esterquats oder Esteramine in der erfinderischen Zusammensetzung können auch von mehr als einem C-18-Pflanzenöl abgeleitet sind, z.B. durch die Mischung mindestens zweier Pflanzenöle vor der Umsetzung zu den Esterquats oder Esteraminen oder durch Mischung zweier Produktgemische von Esterquats aus unterschiedlichen Pflanzenölen nach der Umsetzung.

Äusserst bevorzugt enthält die Zusammensetzung abgeleitet von C-18-Pflanzenölen die entsprechenden Esterquats mit Gegenionen wie oben genannt. Diese bevorzugten Zusammensetzungen können zusätzlich C-18-Glyceride enthalten. Diese werden beispielsweise mit der allgemeinen Form nach dem Pflanzenöl (am Beispiel des Methosulfat-Salzes) Di-"C-18-Pflanze-oylethyl Dimonium methosulfate, Di-"C-18-Pflanze"-oylethyl hydroxethylmonium methosulfate, benannt z.B. Disunfloweroylethyl hydroxyethylmonium methosulfate, oder Disunfloweroylethyl Dimonium Chloride oder nach der hauptsächlich vorliegenden Fettsäure, z.B. Di-"Fettsäure-oylethyl hydroxethylmonium methosulfate. Z.B. kann die erfinderische Zusammensetzung den bevorzugten Diesterquat enthalten, insbesondere mit zwei ungesättigten Fettsäureacylresten mit mindestens 18 C-Atomen abgeleitet von einem C-18-Pflanzenöl, z.B. die besonders bevorzugten Vertreter mit der INCI Disunfloweroylethyl Dimonium Chloride, bevorzugt (and) Sunflower Seed Oil Glycerides, gegebenenfalls (and) Alkyl Lactyl Lactat, z.B. Lauryl lactyl lactat; oder der INCI: Dioleoylethyl Hydroxyethylmonium methosulfate - und bevorzugt (and) Sunflower Seed Oil Glycerides; oder Disunfloweroylethyl hydroxyethylmonium methosulfate, oder - chloride; Dicanolaoylethyl hydroxyethylmonium methosulfate; oder Mischungen z.B. Dicanolaoyl/sunfloweroylethyl hydroxyethylmonium methosulfate oder Dicanolaoylethyl hydroxyethylmonium methosulfate and Disunfloweroylethyl hydroxyethylmonium methosulfate.

Ganz besonders bevorzugt sind die C-18-Pflanzenöle ausgewählt aus Sonnenblumenöl, Canolaöl, dem Rapsöl LEAR (low erucic acid rapeseed), oder eine Kombination derselben.

Amidoamine

Geeignet für die Erfindung sind weiterhin Amidoamine, insbesondere in protonierter Form und Amidoesterquats. Bevorzugte Vertreter für Amidoamine sind Alkylamidopropylamin, Alkylamidoethylamine. Beispiele sind Amidopropyl dimethylamine, Amidoethyl dimethylamine, Amidopropyl diethylamine, Diamidopropyl methylamine, Diamidopropyl ethylamine, Diamidopropyl methylamine.

Die Amidgruppen stellen dabei -NH-COR$^{43}$ und gegebenenfalls -NH-COR$^{44}$, im Falle der Diamidoverbindungen, dar, dabei sind - COR$^{43}$ und -COR$^{44}$ unabhängig voneinander, gesättigten oder ungesättigten, verzweigten oder linearen, gegebenenfalls hydroxy- oder alkoxysubstituierte Fettsäureacylrest mit 6 bis 30 Kohlenstoffatomen, bevorzugt stammt der Fettsäureacylreste aus einem natürlichen Fett oder Öl, besonders bevorzugt aus einem C-18-Pflanzenöl. Mindestens einer der Fettsäureacylrest - COR$^{43}$ oder -COR$^{44}$ ist bevorzugt ein Behensäure- ,Ölsäure-, Linolsäure-, CLA- oder Linolensäurerest. Ein erfinderisch bevorzugter Vertreter ist Behenamidopropyl dimethylamin, insbesondere in seiner protonierten Form. Ganz besonders bevorzugt liegt ein Gemisch an Amidoaminen mit unterschiedlichen Fettsäureacylresten mit einer Verteilung von -COR$^{43}$ - und -COR$^{44}$ - Fettsäureacylresten vor wie als bevorzugt, besonders bevorzugt u.s.w. bei den C-18-Pflanzenölen beschrieben, bezogen auf die eingesetzten Amidoamine in der Zusammensetzung.

Die quaternisierten Amidoamine werden über eine Quaternisierungsreaktion der oben genannten Amidoamine, analog den Esterquats erhalten. Dabei wird eine Alkyl-, oder Alkenylgruppe am (nicht-amidischen) Stickstoff eingeführt, die linear oder verzweigt sein kann. Die Alkyl- oder Alkenylgruppe kann dann weiter derivatisert werden, z.B. mit Alkoholgruppen oder Alkoxylierungen. Die quaternisierten Amidoamine können in den eingesetzten erfinderischen Zusammensetzungen desweiteren Amidoamine enthalten.

Bevorzugte Vertreter sind Methyl bis("C18-Pflanzenöl"- amidoethyl)-2-hydroxyethyl ammonium methylsulfate, z.B. Methyl bis(canolaamidoethyl)-2-hydroxyethyl ammonium methylsulfate ((z.B. hergestellt aus Canolaöl mit Diethylentriamin).

Erfinderisch bevorzugt sind desweiteren Amidoesterquats, die eine Ester und eine Amidgruppe tragen, exemplarisch ist ein geeigneter Vertreter abgebildet in Formel (VIIg)

(VIIg) $[N^+\{(CH_2)_tNH\text{-}COR^{45}\}\{(CH_2)_u\ O\text{-}COR^{46}\}(CH_3)_2]X^-$ Amidesterquat

-COR$^{45}$ und COR$^{46}$ sind unabhängig voneinander gesättigte oder ungesättigte, verzweigte oder lineare, gegebenenfalls hydroxy- oder alkoxysubstituierte Fettsäureacylreste mit 6 bis 30 Kohlenstoffatomen, bevorzugt stammt mindestens einer der Fettsäureacylreste in dem Produktgemisch aus einer natürlichen Fett oder Öl, insbesondere einem C-18-Pflanzenöl, besonders bevorzugt stammen die Fettsäureacylreste aus einem oder mehreren Fetten oder Ölen, insbesondere C-18-Pflanzenölen. u und t = jeweils unabhängig voneinander 1-10, bevorzugt 2-5, besonders bevorzugt 2-3

Bevorzugt werden auch Mischungen aus Esterquats und Amidoesterquats, jeweils auf Basis von C-18-Pflanzenölen eingesetzt. Dabei können jeweils Esteramine oder Amidoamine in den Mischungen anwesend sein.

Weiterhin besonders bevorzugt ist die Gruppe der kationischen Tenside der Ester von Cholinen, z.B. mit RI = $-CH_2CH_2OCOR^{47}$, RII, RIII und RIV Methylgruppen, $-COR^{47}$ ist ein gesättigter oder ungesättigter, verzweigter oder linearer, gegebenenfalls hydroxy- oder alkoxysubstituierter Fettsäureacylrest mit 6 bis 30 Kohlenstoffatomen, bevorzugt ein gesättigter oder ungesättigter Fettsäureacylreste mit 18 bis 22 Kohlenstoffatomen, bevorzugt liegt ein Produktgemisch an Cholinestern mit unterschiedlichen Fettsäureacylresten vor und mindestens einer der Fettsäureacylreste in dem Produktgemisch stammt aus einem natürlichen Fett oder Öl, insbesondere einem C-18-Pflanzenöl.

Eine weitere Gruppe besonders bevorzugter kationischer Tenside sind Betainate

Betainate oder Betain esterquats z.B. mit RI = $-CH_2CO-OR^i$, RII, RIII und RIV Methylgruppen, Ri Alkyl oder Hydroxyalkyl, ein bevorzugtes Vertreter ist z.B. Arachidyl/Behenyl betainate esylate gegebenenfalls mit Arachidyl/Behenylalkohol in der Zusammensetzung.

Bevorzugte kationischer Tenside sind weiterhin Esterquats von Zuckerderivaten ("Glucoquats"), bei denen einer der Reste RI bis RVI eine Glycosylgruppe darstellt,
Andere geeignete kationische Tenside sind beispielsweise andere quartäre Ammoniumverbindungen ("Quats"), Amidquats, Imidazolinquats, Alk(en)ylquats, Betainate, protonierte Amine und jeweils deren Kombinationen, Vertreter sind z.B. Diamidoamine von DETA, Alkyltrimonium Chloride, z.B. BTAC, (Behentrimonium chloride), CETAC (Cetrimonium chloride), u.a.

Jeweils bevorzugt enthalten sind kationische Tenside mit gesättigten oder ungesättigten Alk(en)yl- oder Acylreste mit 16 bis 22 Kohlenstoffatome, insbesondere bevorzugt mit ungesättigten Resten, äusserst bevorzugt abgeleitet aus C-18-Pflanzen.

Die erfinderischen Zusammensetzungen mit kationischen Tensiden können desweiteren andere Fettsäurederivate, bevorzugt aus C-18-Pflanzenölen mit bevorzugten Anteilen an verschiedenen Kettenlängen und Sättigungsgraden der Fettsäureacyl- , bzw. Fettalkoholmengen bezogen auf die jeweils entsprechenden Fettsäurederivate enthalten, z.B. Fettsäureester, Fettsäureamide, Fettalkohole. Die bevorzugten Anteile entsprechen den vorher definierten bevorzugten Anteilen an Fettsäuren der C-18-Pflanzen.

Die erfinderischen Zusammensetzungen enthalten bevorzugt sind in der festen Ausführungsformen Saccharide, wie in der Anmeldung definiert, enthalten.

Zusammensetzungen mit Tenside K

Die erfinderischen Zusammensetzungen mit einem oder mehreren Tensiden K enthalten ein oder mehrere Acylglycamide (vorzugsweise wie vorstehend als bevorzugt bezeichnet), bevorzugt liegen diese mit einem Lösungsmittel, besonders bevorzugt ausgewählt aus Alkoholen, niedrigen Alkylethern, Propylenglykol, Polyethylenglykol und Polypropylenglykol vor, besonders bevorzugt mit Propylenglykol vor, mit einer Gesamtmenge (mit Lösungsmittel) von bevorzugt 0,2 bis 20 Gew.-%, Bevorzugt sind die ein oder mehreren Biotenside zusätzlich in der Zusammensetzung enthalten. Es sind auch Ausführungsformen ohne Biotenside geeignet.

0,01 bis 40 Gew.-% eines oder mehrerer der Tenside K, i.e. Amidoamine oder Esteramine oder kationischen Tenside bevorzugt Ammoniumverbindungen ("Quats"), oder protonierte Amine, z.B. Amidoamine oder Esteramine, besonders bevorzugt 0,1 bis 20 Gew.-%, bevorzugter 0,1 bis 15 Gew.-% bevorzugter 0,1 bis 7 Gew.-%;.

Desweiteren stellt eine bevorzugte Ausführungsform Konzentrate oder feste Mittel dar - diese zeichnen sich durch höhere Konzentrationen der Tenside K aus, bevorzugt 10-40 Gew.-%, insbesondere bevorzugt liegen die Tenside K in Konzentrationen von > 10 Gew.-% bis 40 Gew.-% vor, Gew.-% bezogen auf die Gesamtzusammensetzung.

Optional können bis 40 Gew.-% eines oder mehrerer weiterer Fettsäurederivate enthalten sein, bevorzugt bis 10 Gew.-%, besonders bevorzugt bis 7 Gew.-%, insbesondere ausgewählt aus Pflanzenölen, Glyceriden, Fettsäuren oder Fettalkoholen, bzw. deren Salze wie Alkali-, Erdalkali-, Magnesium, Zink, Titan, Ammonium, oder organische Ammoniumsalze (z.B. TEA, DEA, MEA, etc.), bevorzugt jeweils abgeleitet von C-18-Pflanzenölen, enthalten sein.

Gegebenenfalls enthält die Zusammensetzung in der flüssigen Ausführungsform Lösungsmittel, wie Wasser, Propylenglykol, Isopropylalkohol, Glycerin, Glykole und andere, oder in der festen Ausführungsform ein Trägersystem, d.h. Gerüststoffe oder strukturgebende Mittel, in einem Mengenbereich in Summe von vorzugsweise 99,8 Gew.-%, bevorzugter bis 95 Gew.-%, noch bevorzugter bis 90 Gew.-%; weiterhin können auch in der festen Ausführungsform Lösungsmittel z.B. Propylenglykol enthalten sein. Bevorzugt besteht das Trägersystem aus ein oder mehreren organischen Materialien, besonders bevorzugt Saccharide, wie vorhergehend beschrieben, z.B. Di-, Oligo- oder Polysaccharide, ggf. kationisch modifizierte Polysaccharide, Guar, Maltodextrin, Cellulose oder Cellulosederivate, Pectin, Carrageenan, Xanthane, Dextrane, Curdlane, Chitosane, Chitine, Sacharose, Stärke z.B. Tapioka-, Reis- oder Maisstärke, Weizenmehl, Glucose; desweiteren Harnstoff oder Harnstoffderivate, Carbonsäuren, z.B. Citronensäure und alternativ oder zusätzlich ein oder mehrere anorganischen Materialien, besonders bevorzugt Alkali- und Erdalkalisalze und Mineralien, wie Natriumcarbonat, Siliciumdioxid, amorphes oder gefälltes Silika, Calciumsilicate, Silikagel, Kreide, Zeolite,

Bentonite, Schichtsilikate, Natrium aluminiumsilikate, Kaolin, Natriumbicarbonat, Natriumsulfat, Natrium Triphosphate, Natriumchlorid, Wasserglas, Magnesiumchlorid, Calciumchlorid, Aluminiumchlorid, Magnesiumsulfat, Calciumcarbonat, Calciumoxid, Calciumsulfat;

Das Trägersystem kann auch aus wasserlöslichen Polymeren, zum Beispiel Blockcopolymere basierend auf Ethylenoxid und Propylenoxid. Falls diese zum Einsatz kommen, sind solche Bockpolymere bevorzugt, welche aus natürlichen Rohstoffen zugänglich sind. Erfinderisch sind die Blockcopolymere aufgrund von Umweltgedanken weniger bevorzugt.

Sowie gegebenenfalls weitere Inhaltsstoffe bevorzugt gesamthaft in einem Mengenbereich von 0,1 bis 25 Gew.-%, bevorzugt 1 bis 20 Gew.-%, wie Duftstoffe, Farbstoffe, Vernetzungsmittel, Viskositätsregler, Bindemittel, Tenside oder Emulgatoren (z.B. ethoxylierte Fettalkohole), vornehmlich nichtionisch oder zwitterionisch; Ölkörper, Polymere, Filmbildner, Überfettungsmittel, Konservierungsmittel, Antimicrobials, Antioxidantien, Pflanzenextrakte, Actives, Salze, Silikone, Stabilisatoren, Perlglanzkomponenten, pH-Stellmittel und allenfalls Silikonöle (aufgrund Umweltbetrachtungen weniger bevorzugt). Gewichtsprozent jeweils bezogen auf die Menge Aktivsubstanz in der Zusammensetzung.

Anwendungstechnisch bevorzugt enthalten die Ausführungsformen, enthaltend ein oder mehrere Acylglycamine und gegebenenfalls ein oder mehrere Biotenside und ein oder mehrere Tenside K, allenfalls anionische Tenside ausgewählt aus den Gruppen wie vorhergehend beschrieben, vorzugsweise aus den bevorzugten Gruppen. Insbesondere bevorzugt enthalten diese Ausführungsformen keine weiteren anionische Tenside. Nichtionische und allenfalls zwitterionische Tenside können optional enthalten sein.

Bevorzugt werden feste erfinderische Ausführungsformen mit Sacchariden als Trägersystem, da die erfinderischen Zusammensetzungen gut an Saccharide adsorbieren, leicht in Wasser dispergiert werden können und keine Rückstände bilden.

In bevorzugten Ausführungsformen enthaltend Tenside K, werden N-Acylglycamine und die Tenside K und die Fettsäurederivate von C-18-Pflanzenölen abgeleitet. Der Anteil an Inhaltsstoffen, ausgewählt aus Tensiden (einschliesslich ggf. den bevorzugten Biotensiden), Emulgatoren, Fettsäurederivaten beträgt in einer besonders bevorzugten Ausführungsform > 40 Gew.-%, bevorzugter > 57 Gew.-%, äusserst bevorzugt > 75 Gew.-%, Biotenside können in einer bevorzugten Ausführungsvariante enthalten sein.

Diese Zusammensetzung ist insbesondere als Weichspüler, weichspülendes Additiv für Textilanwendungen oder als kosmetischer Conditioner, z.B. Haarconditioner oder konditionierendes Additiv für kosmetische Zwecke geeignet, bevorzugt als Weichspüler oder Haar- oder Hautconditioner.

Die Verwendung der erfinderischen Zusammensetzungen als Pflegemittel enthaltend Tenside K, insbesondere Weichspüler und Haar- und Hautconditioner zeichnen sich dadurch aus, dass sie bevorzugt keine Sulfattenside, besonders bevorzugt keine anionischen Tenside enthält. Eine bevorzugte Ausführungsform für die Verwendung als Weichspüler und Haar- und Hautconditioner enthält desweiteren keine Betaintenside.

Verfahren

Gegenstand des Patents ist weiterhin ein Verfahren zur Herstellung der erfinderischen Zusammensetzungen in fester Form, indem die flüssigen und geschmolzenen Komponenten, das mindestens eine Tensid K und das mindestens eine Acylglycamid (und gegebenenfalls das optional mindestens eine Biotensid), bevorzugt vorliegend mit bis zu 30% Lösungsmittel, bei einer Temperatur von 25-90°C, bevorzugter 35-80°C, besonders bevorzugt bei ca. 60°C geschmolzen und vermischt werden. Die Schmelze wird dann mit dem organischen Material vermischt, vorzugsweise einem Saccharid, um ein Tensid-Saccharid-Aggregat zu erhalten.

Das Endprodukt nach Abkühlung und Aushärtung zu erhalten. Bevorzugt werden die Duftstoffe und gegebenenfalls andere hitzeempfindlichen Inhaltsstoffe, z.B. Actives erst bei einer Temperatur < 40 °C oder nach Abkühlen zugegeben. Dies kann auch durch Aufsprühen erfolgen. Optional wird ein Trocknungsschritt durchgeführt.

Alternatives Verfahren ohne Aggregate:

a) Bereitstellung des mindestens einen N-Acylglycamin, bevorzugt in Schmelze oder flüssigem Trägermaterial; bevorzugte Temperaturen von 40 °C - 80 ° C, besonders bevorzugt 40-49,9° C

b) Zugabe des mindestens einen Tensids K zu a) und gegebenenfalls weiteren schmelzbaren Inhaltsstoffen bei 60-80° C

c) Vermengen von b) mit mindestens einem oder mehreren weiteren Inhaltsstoffen für Reinigungs- oder Pflegemittel, bevorzugt mit Tensiden, insbesondere mit dem bevorzugten mindestens einen Glycolipid-Biotensid.

In einer bevorzugten Ausführungsform des Verfahrens werden ein, mehrere oder alle Komponenten in flüssiger Form gemischt oder aufgebracht. Hierbei werden z.B. Duftstoffe aufgesprüht, härtende Substanzen eingebracht oder mithilfe eines Lösungsmittels oder fliessfähigen Tensids eine Mischung herstellt.

d) Optional (Aus-)Härten oder Trocknen der Zusammensetzung, oder Überführen der Schmelze zu Schritt e)

e) In Form bringen der Zusammensetzung, bevorzugt durch Zerkleinern, Zuschneiden, Tablettieren, Aufbringen auf oder Umhüllung mit Materialien, z.B. wasserlösliche Materialien wie PVOH, Pressen, Granulieren, Sprühen, Mahlen, Einbringen in Schablonen oder Giessformen, oder deren Kombinationen.

e) Optional (Aus-)Härten oder Trocknen der Zusammensetzung, falls nicht erfolgt als Schritt d)

f) Konfektionieren der festen Produktform

Der pH-Wert der Zusammensetzungen mit kationischen Tensiden ist unter 9, bevorzugt unter 8 und besonders bevorzugt unter 7. In mindestens einer Ausführungsform liegt der pH zwischen 3 und 6, bevorzugter zwischen 3,5 und 5.
Eine bevorzugte Ausführungsvariante stellt die feste Produktform dar.

Ergänzend wird offenbart:

1. Zusammensetzung enthaltend mindestens ein N-Acylglycamin der Formel (I), (vorzugsweise wie als bevorzugt, besonders bevorzugt usw. beschrieben)
und ein oder mehreren Tensiden K ausgewählt aus den Gruppen der kationischen Tensiden oder den sekundären oder tertiären Amidoaminen oder den sekundären oder tertiären Esteraminen,
2. Zusammensetzung gemäss 1 zusätzlich enthaltend mindestens ein Biotensid.
3. Zusammensetzung, gemäss 1-3, wobei das ein oder die mehreren kationischen Tenside ausgewählt sind aus den quartäre Ammoniumverbindungen ("Quats") oder protonierten Aminen mit der generellen Formel (VII).
4. Zusammensetzung gemäss 1-3, dadurch gekennzeichnet, dass das ein oder die mehreren kationischen Tenside ausgewählt sind aus den Gruppen der Esterquats, Amidoaminquats, protonierten Amidoamine, protonierte Amidoester oder Amidoesterquats, Glucoquats, wobei mindestens einer der Amid- oder Esterfunktionen der vorstehenden Tenside einen Fettsäureacylrest trägt.
5. Zusammensetzung gemäss 1-4 enthaltend mindestens einen Esterquat ausgewählt aus den Gruppen der Diesterquats, Betain Esterquats, Amide Esterquats, Choline Esterquats.
6. Zusammensetzung gemäss 1-5, dadurch gekennzeichnet, dass mindestens einer der Fettreste von mindestens einem der Tenside K, also mindestens einer der Fettsäureacylreste oder gegebenenfalls der Fettalkoholreste der Ester- oder Amidgruppe, von einer Ölsäure-, einer Linolsäure- einer Linolensäure-, einer konjugierten Linolsäure-(CLA)-, einer Stearin-, einer Palmitin-, einer Erucasäure-, einer Gadolein-, einer Nervonsäure-, einer Behensäure, einer Arachidonsäure-, oder einer Lignocerinsäure, oder dem entsprechenden Fettalkohol abgeleitet ist.
7. Zusammensetzung gemäss 1-6, dadurch gekennzeichnet, dass die Fettreste von mindestens einem der Tenside K, also mindestens einer der Fettsäureacylreste oder gegebenenfalls der Fettalkoholreste der Ester- oder Amidgruppe ungesättigte Fettreste mit 18 oder mehr Kohlenstoffatomen. Ganz besonders bevorzugt ist dabei mindestens einer der Fettsäureacylreste der Amid- oder Esterfunktionen ein- oder mehrfach ungesättigt
8. Zusammensetzung gemäss 1-7, dadurch gekennzeichnet, dass mindestens ein Produktgemisch von mindestens einer der Tensidgruppen K in der Zusammensetzung enthalten ist, welches sich durch Kettenlängen oder Sättigungsgraden des oder der Fettrestes unterscheiden, bevorzugt erhalten aus Fettsäurederivaten, welches eine Fettsäureverteilung gemäss einem nativen Pflanzenöl aufweisen.
9. Zusammensetzung gemäss 1-8, dadurch gekennzeichnet, dass das mindestens eine Produktgemisch von mindestens einer der Tensidgruppen K von einem C-18-Pflanzenöl, bevorzugt von Canola, Raps, Sonnenblume abgeleitet ist.
10. Zusammensetzung gemäss 1-9, dadurch gekennzeichnet, dass das mindestens eine Produktgemisch eine Fettsäureacyl- oder Fettalkoholverteilung von Fettresten mit 18 und mehr Kohlenstoffatomen über 60 Gew.-%, besonders bevorzugt über 72 Gew.-% und ganz besonders bevorzugt von über 77 Gew.-% vor und äusserst bevorzugt liegt dabei der Anteil an ungesättigten Fettsäureacylresten über 55 Gew.-%, vorzugsweise über 65 Gew.-% und besonders bevorzugt über 72 Gew.- % liegt, Gew.-% bezogen auf das jeweilige Produktgemisch eines kationischen Tensids.
11. Zusammensetzung gemäss 1-10, dadurch gekennzeichnet, dass sie ungesättigte Esterquats oder Amidoaminquats enthält.
12. Zusammensetzung gemäss 1-11 zusätzlich enthaltend mindestens ein Saccharid
13. Zusammensetzung gemäss 1-12, wobei das N-Acylglycamin bevorzugt ausgewählt ist aus N-Acyl-N-methyl glucamin mit Fettsäureacylresten abgeleitet aus mindestens einem C-18-Pflanzenöl, insbesondere Sunfloweroyl

Methylglucamid; und N-Acyl-N-methyl cyclic glucamide, insbesondere mit den Acylresten ausgewählt aus $-C(O)-(CH_2)_8CH_3$ oder $-C(O)-(CH_2)_6CH_3$ oder eine Mischung derselben; sowie die Kombination der N-Acylglycamine.

14. Zusammensetzung gemäss 1-13, wobei das Tensid K ausgewählt ist aus Di(C-18-Pflanzenöloyl) hydroxyethylmonium, Di(C-18-Pflanzenöl amidoethyl) hydroxyethyl ammonium, Alkyl bis(-18-Pflanzenöloyl) hydroxyethyl ammonium, Alkyl bis(C-18-Pflanzenöl amidoethyl) hydroxyethyl ammonium, Di(C-18-Pflanzenöloyl) dialkylammonium, Di(C-18-Pflanzenöl amidoethyl) dialkylammonium, Dimethyl bis(C-18-Pflanzenöloyl) ammonium, Dimethyl bis(C-18-Pflanzenöl amidoethyl) ammonium und deren Kombinationen; jeweils mit einem Gegenion, bevorzugt sind die Vertreter Dioleoylethyl Hydroxyethylmonium, Methyl bis(canola amidoethyl)-2-hydroxyethyl ammonium, Disunfloweroylethyl Hydroxyethylmonium, Disunfloweroylethyl dimethyl ammonium, Disunfloweroylethyl Dimonium, Dicanolaoylethyl hydroxyethylmonium Dicanolaoyl/sunfloweroylethyl hydroxyethylmonium sowie deren Kombinationen, mit jeweils den bevorzugten Gegenionen Methosulfat und Chlorid.

15. Zusammensetzung gemäss 1-14 wobei das Tensid K ausgewählt ist aus Arachidyl/Behenyl Betainate Esylate (and)Arachidyl/Behenyl Alkohol,

16. Zusammensetzung gemäss 1-15 zusätzlich enthaltend C-18-Pflanzenöle oder deren Glyceride, bevorzugt Sunflower seed oil glycerides, und/oder C-18-Fettalkohole.

17. Zusammensetzung gemäss 1-16 wobei das Biotensid bevorzugt ausgewählt ist aus Rhamnolipiden, Sophorolipiden und Mannosylerythritollipiden, bevorzugt Mannosylerythritollipiden

18. Zusammensetzung gemäss 1-17 enthaltend Sunfloweroyl Methylglucamid und Dioleoylethyl Hydroxyethylmonium und Sunflower seed oil glycerides oder Disunfloweroylethyl hydroxyethylmonium und Sunflower seed oil glycerides Disunfloweroylethyl Dimonium und Sunflower Seed Oil Glycerides mit jeweils Chlorid oder Methosulfat als Gegenion.

19. Zusammensetzung gemäss 1-18 enthaltend Sunfloweroyl Methylglucamid und Dioleoylethyl Hydroxyethylmonium Methosulfate und Sunflower seed oil glycerides.

20. Zusammensetzung gemäss 1-19 enthaltend Sunfloweroyl Methylglucamid und Methyl bis(canola amidoethyl)-2-hydroxyethyl ammonium methyl sulfate

21. Zusammensetzung gemäss 1-20, zusätzlich enthaltend Sorbitol ester, bevorzugt Sunflower seed oil Sorbitol esters

22. Zusammensetzungen gemäss 1-21 als fliessfähige Ausführungsform oder aufgebracht auf feste Träger wie Blätter ("sheets") oder auf Tücher ("wipes")

23. Zusammensetzung gemäss 1-21 oder in der festen Ausführungsform

24. Verwendung der Zusammensetzungen gemäss 1-23 als weichspülendes Additiv oder konditionierendes Additiv

**Verwendung & Verfahren**

Gegenstand der Erfindung ist daher weiterhin die Verwendung der Zusammensetzungen enthaltend N-Acylglycamin, und bevorzugt enthaltend mindestens ein Glycolipid-Biotensid, zur Herstellung rückstandsarmer Reinigung- und Pflegemittel oder die Verwendung von mindestens einem N-Acylglycamin, und bevorzugt zusätzlich mindestens einem Glycolipid-Biotensid, als Additiv für rückstandsarme Reinigungs- und Pflegemitteln.

Die N-Acylglycamine gemäss der Erfindung, bevorzugt in Kombination mit Biotensiden, können daher als Additive für eine gute Schaumbildung und -stabilität in hartem Wasser ≥ 15°F, bevorzugt > 16°dH, oder bei hohen Ionenstärken verwendet werden.

Die erfinderischen Zusammensetzungen sind auch für Wasser ohne Enthärterzugaben mit einem Calciumcarbonatgehalt von > 0,89 mmol/l (5° dH) bevorzugt von ≥ 1,5 mmol/l (8,43°dH), besonders bevorzugt von > 3,2 mmol/l (18°dH). (≥ 15°F, bevorzugt > 16°d) geeignet.

Die Eignung bei hartem Wasser gilt auch bei Zugabe von Citrat-Enthärtern. Zum Beispiel bei Zugabe von Citrat-Enthärter < 2 Gew.-% (bezogen auf den Citratteil ohne Gegenkation) ist die Zusammensetzung selbst über ≥ 1,5 mmol/l (8,43°dH), bevorzugt über 16°dH und besonders bevorzugt über > 3,2 mmol/l (18°dH) (≥ 15°F, bevorzugt > 16°d) geeignet. Weitere Enthärter-Substanzen sind nicht erforderlich.

**Haut- und Haarpflege**

Ein weiterer Gegenstand der Erfindung ist ein Haut- oder Haarreinigungs- oder -pflegemittel, enthaltend mindestens ein N-Acylglycamin, insbesondere entsprechend den als bevorzugt beschriebenen Vertretern, und bevorzugt zusätzlich enthaltend mindestens ein Glycolipid-Biotensid. Das Mittel kann in der festen oder fliessfähigen Ausführungsform vorliegen. Eine bevorzugte Ausführungsform sind rinse-off Formulierungen, insbesondere Conditioner oder Haut- oder Haarreinigungsmittel, z.B: Waschlotionen, Duschcreme, Badezusätze, Öl- oder Schaumbad, Handseifen, Shampoos, Spezialausführungen von Shampoos wie Anti-Schuppen-Shampoo, 2-in-1-Produkte, konditionierende Shampoos,

Kinder- und Babyshampoos, no tear shampoo, u.s.w.; Gesichtsreinigungslotionen, Intimwäschen, u.s.w.. Erfindungsgegenstand ist weiterhin die Verwendung der Mittel für die oben genannten Verwendungen.

Bevorzugte Ausführungsformen enthalten vorzugsweise zusätzliche Tenside gemäss den als bevorzugt beschriebenen Tensiden. Inbesondere sind zusätzliche Tenside enthalten entsprechend der oben ausgeführten bevorzugten Tensid-Kombinationen.

**Feste Formulierungen**

Eine besonders bevorzugte Ausführungsform sind feste Formulierungen.

In mindestens einer bevorzugten Ausführungsform werden kosmetische rinse-off Produkte mit mindestens einem N-Acylglycamine oder mit mindestens einem Biotensid oder deren Kombinationen, jeweils vorzugsweise wie vorhergehend als bevorzugt beschrieben. Zum Beispiel sind dies Haarkonditioner, Haarshampoos, Hautreinigung, Zahnpflege, Peeling.

In mindestens einer weiteren bevorzugten Ausführungsform werden feste Reinigungs- und Pflegeprodukte für Haushalt und Gewerbe mit mindestens einem N-Acylglycamine oder mit mindestens einem Biotensid oder deren Kombinationen, jeweils vorzugsweise wie vorhergehend als bevorzugt beschrieben, offenbart, z.B. Weichspüler, WC-Steine, Waschmittel, Geschirrspülmittel, Klarspüler, u.s.w. In einer bevorzugten festen Ausführungsform liegen die Produkte als Pearls, Pastillen, Pucks, Sticks, Kapseln, Extrudate vor.

In mindestens einer alternativen bevorzugten Ausführungsform werden kosmetische leave-on Produkte mit mindestens einem N-Acylglycamine oder mit mindestens einem Biotensid oder deren Kombinationen, jeweils vorzugsweise wie vorhergehend als bevorzugt beschrieben, offenbart. Beispiele hierfür sind Sunblocker, Deodorants und Antiperspirants, Haar- und Hautmasken.

Mindestens eine bevorzugte Ausführungsform für feste Reinigungs- und Pflegeprodukte enthält die bevorzugten die N-Acyl-N-Methylglucamine abgeleitet von einem C-18-Pflanzenöl. In mindestens einer weiteren bevorzugten Ausführungsform sind zusätzlich Biotenside enthalten.

Mindestens eine weitere bevorzugte Ausführungsform für feste Reinigungs- und Pflegeprodukte enthält die bevorzugten N-Acyl-N-methyl cyclic glucamide. In mindestens einer weiteren bevorzugten Ausführungsform sind zusätzlich Biotenside enthalten.

Mindestens eine weitere bevorzugte Ausführungsform für feste Reinigungs- und Pflegeprodukte enthält die bevorzugten N-Acyl-N-methyl cyclic glucamide und die N-Acyl-N-Methylglucamine abgeleitet von einem C-18-Pflanzenöl. In mindestens einer weiteren bevorzugten Ausführungsform sind zusätzlich Biotenside enthalten.

Mindestens eine weitere bevorzugte Ausführungsform für feste Reinigungs- und Pflegeprodukte enthält mindestens ein Biotensid.

Eine besonders bevorzugte Ausführungsform sind feste Formulierungen, bevorzugt rinse-off, insbesondere gelten die bevorzugten Strukturen der N-Acylglycamine wie oben beschrieben, ganz besonders bevorzugt sind in dieser Ausführungsform die N-Acylglycamine erhalten über Fettsäuregemische von C-18-Pflanzenölen als Rohstoffe für die Acylreste, insbesondere bevorzugt Sunfloweroyl Methylglucamide. Besonders bevorzugt enthält die Zusammensetzung ein Gemisch an N-Acylglycaminen abgeleitet von einem C-18-Pflanzenöl bestehend aus N-Alkyl-N-Methylglucaminen der Formel (Ia) mit Anteilen an ein- und mehrfach ungesättigten Fettsäureacylresten mit 18 Kohlenstoffatomen von mind. 80 Gew.-% und mit Anteilen von summarisch max. 15 Gew.-% von Palmitoyl- und Stearoylresten; ganz besonders bevorzugt beträgt dabei der Anteil an einfach ungesättigten Fettsäureacylresten mit 18 Kohlenstoffatomen (Oleoylreste) mind. 75 Gew.-%, Gew.-%, zum Beispiel aus Sonnenblumenfettsäuregemischen, jeweils bezogen auf die Fettsäureacylreste RaCO in dem N-Acyl-N-Methylglucamine (Ia). Ganz besonders bevorzugt enthält die Zusammensetzung ein Gemisch an N-Acylglycaminen abgeleitet von einem C-18-Pflanzenöl bestehend aus N-Alkyl-N-Methylglucaminen der Formel (Ia) mit Anteilen an mehrfach ungesättigten Fettsäureacylresten mit 18 oder mehr Kohlenstoffatomen von > 8 Gew.-%, Gew.-% jeweils bezogen auf die Fettsäureacylreste RaCO in dem jeweiligen Gemisch von N-Acyl-N-Methylglucaminen (Ia).

Zusätzlich sind bevorzugt weitere Tenside enthalten entsprechend der oben beschriebenen Bevorzugungen, speziell in festen rinse-off-Formulierungen sind insbesondere bevorzugt Tenside ausgewählt aus Seifen oder Fettsäuren, Sodium Acylisethionaten, Sodium Acyl Methyl Isethionaten, Tauraten, Sodium Acyl Methyl Tauraten, Sulfaten, z.B. Cocosulfate; Sodium lauryl sulfate, Alkylpolyglycoside, z.B. Cocoglucosid, weitere N-Acylglucamide, Sulfonaten, Alpha Olefin Sulfonaten, Sulfoacetaten, Sulfosuccinate, z.B. Disodium Alkyl Sulfosuccinaten, Betainen z.B. Cocobetain, Alkylamidopropylbetainen, z.B. Cocoamidopropylbetain, Glycinaten, Glutamaten und deren Kombinationen. Die anionischen Tenside können jeweils als Säure oder Salze vorliegen, vorzugsweise als Alkali-, Erdalkali-, Ammoniumsalze oder als Salze organischer Basen, z.B: der organischen primären, sekundären oder tertiären Amine

Bevorzugte Tensid-Kombinationen, enthaltend das mindestens eine N-Acylglucamin, und:

- bevorzugt mindestens ein Biotensid und mindestens ein weiteres Tensid ausgewählt aus Alkyl(poly)glucosid , bevorzugt Coco Glucoside und Sulfaten, bevorzugt Sodium Coco-Sulfate, Sodium Cetearyl Sulfate, Sodium lauryl sulfate und deren Kombinationen.

- bevorzugt mindestens ein Biotensid und mindestens ein weiteres Tensid ausgewählt aus der Gruppe der N-Acylglycamine, bevorzugt Capryloyl/Caproyl Methyl Glucamide, Lauroyl/Myristoyl Methyl Glucamide und/oder Cocoyl Methyl Glucamide und deren Kombinationen
- bevorzugt mindestens ein Biotensid und mindestens ein weiteres Tensid ausgewählt aus der Gruppe der Sulfate, bevorzugt Sodium Coco-Sulfate, und eine weiteres N-Acylglycamin, bevorzugt Capryloyl/Caproyl Methyl Glucamide, Lauroyl/Myristoyl Methyl Glucamide und/oder Cocoyl Methyl Glucamide und optional Seife oder Fettsäuren, z.B. der Stearinsäure und deren Kombinationen.
- bevorzugt mindestens ein Biotensid und mindestens ein weiteres Tensid ausgewählt aus der Gruppe der Sulfate, bevorzugt Sodium Coco-Sulfate, und eine weiteres N-Acylglycamin, bevorzugt Capryloyl/Caproyl Methyl Glucamide, Lauroyl/Myristoyl Methyl Glucamide und/oder Cocoyl Methyl Glucamide und optional Seife oder Fettsäuren, z.B. der Stearinsäure und deren Kombinationen.
- bevorzugt mindestens ein Biotensid und mindestens ein weiteres Tensid ausgewählt aus den Isethionaten, insbesondere den Sodium Acylisethionaten, Sodium Acyl Methyl Isethionaten bevorzugt Sodium cocoyl isethionate, Sodium cocoyl methyl isethionate, Sodium lauroyl isethionate, Sodium lauroyl methyl isethionate, und deren Kombinationen.
- bevorzugt mindestens ein Biotensid und mindestens ein Tensid ausgewählt aus den Isethionaten und mindestens ein weiteres Tensid ausgewählt aus, Seifen oder Fettsäuren, Sodium Acylisethionaten, Sodium Acyl Methyl Isethionaten, Tauraten, Sodium Acyl Methyl Tauraten, Sulfaten, z.B. Cocosulfate; Sodium lauryl sulfate, Alkylpolyglycoside, z.B. Cocoglucosid, weitere N-Acylglucamide, Sulfonaten, Sulfoacetaten, Alpha Olefin Sulfonaten, Disodium Alkyl Sulfosuccinaten, Betainen z.B. Cocobetain, Alkylamidopropylbetainen, z.B. Cocoamidopropylbetain, Glycinaten, Glutamaten, und deren Kombinationen.
- bevorzugt mindestens ein Biotensid und mindestens ein Tensid ausgewählt aus den Isethionaten und mindestens ein weiteres Tensid ausgewählt aus den Seifen, Sodium Acylisethionaten, Sodium Acyl Methyl Isethionaten, Tauraten, Sodium Acyl Methyl Tauraten, Sulfaten, z.B. Cocosulfate; Sodium lauryl sulfate, Alkylpolyglycoside, z.B. Cocoglucosid, weitere N-Acylglucamide, Sulfonaten, Sulfoacetaten, Alpha Olefin Sulfonaten, Disodium Alkyl Sulfosuccinaten, Betainen z.B. Cocobetain, Alkylamidopropylbetainen, z.B. Cocoamidopropylbetain, Glycinaten, Glutamaten,und deren Kombinationen.
- mindestens ein Tensid ausgewählt aus den Betainen z.B. Cocobetain oder Alkylamidopropylbetainen, z.B. Cocoamidopropylbetain, und deren Kombinationen.
- mindestens ein Biotensid, und mindestens ein Tensid ausgewählt aus den Betainen z.B. Cocobetain oder Alkylamidopropylbetainen, z.B. Cocoamidopropylbetain, und deren Kombinationen.
- mindestens ein Tensid ausgewählt aus den Aminosäuretensiden z.B. Glycinate, Glutamate und oder Tauraten, und deren Kombinationen.
- mindestens ein Biotensid, und mindestens ein Tensid ausgewählt aus aus den Aminosäuretensiden und/ oder Tauraten, z.B. Glycinate, Glutamate, und deren Kombinationen.
- mindestens ein Sulfoacetat, besonders bevorzugt Sodium Lauryl Sulfoacetate.
- mindestens ein Biotensid, und mindestens ein Sulfoacetat, besonders bevorzugt Sodium Lauryl Sulfoacetate.

Bevorzugt sind desweiteren Ausführungsformen durch Kombinationen der bevorzugten Tensidkombinationen beansprucht.

Eine bevorzugte Zusammensetzungen fester Rinse-Off-Formulierungen besteht aus 0,2 und 12 Gew.-%, Gew.-% N-Acylglycamin, 5-65 Gew.-% eines weiteren Tensids, bevorzugt ausgewählt aus Seifen bzw. Fettsäuren, Sodium Acylisethionaten, Sodium Acyl Methyl Isethionaten, Tauraten, Sodium Acyl Methyl Tauraten, Sulfaten, Sulfonaten, Alpha Olefin Sulfonaten, Sulfoacetaten, Disodium Alkyl Sulfosuccinaten, und deren Kombinationen, ganz besonders bevorzugt ausgewählt aus Biotensiden und deren Kombinationen; optional 0 - 20 Gew.% eines weiteren Tensids bevorzugt ausgewählt aus Betainen, Alkylamidopropylbetainen, Glycinaten, Glutamaten, Alkylpolyglycoside, N-Acylglucamide und deren Kombinationen, Füllstoffe 0-25 Gew.-%, Konsistenzgeber und Emollients 20-30 Gew.-%, bevorzugt Fettalkohole; Wasser 0-8%, unter 5% je nach Produkt Duftstoffe, Wirkstoffe, Farbstoffe, Konservierung, Stabilisatoren, Hilfsstoffe.

Die Zusammensetzungen zeichnen sich neben den genannten Vorteilen durch einen konditionierenden Effekt aus. Es werden milde Reinigungsprodukte mit einer guten Sensorik erhalten.

Feste Formulierung mit Biotensid

Weiterer Erfindungsgegenstand sind feste Formulierungen enthaltend mindestens ein Biotensid. Bevorzugt enthalten die festen Formulierungen mindestens ein Biotensid, umfassend Rhamnolipide, Sophorolipide, Mannosylerythritollipide, Cellobioselipide, Trehaloselipide und deren Kombinationen, besonders bevorzugt Sophorolipid oder

Rhamnolipid, ganz besonders bevorzugt Rhamnolipid. Optional enthalten diese Formlierungen zusätzlich N-Acylglycamine. Feste Formulierungen beinhalten kosmetische Reinigungs- oder Pflegemittel und Reinigungs-oder Pflegemittel für den Haushalt oder den Gewerbesektor. Bevorzugte feste Ausführungsformen, insbesondere für den Haushalt oder den Gewerbesektor sind hergestellt über gehärtete Schmelzen oder adsorbierte Tensiden auf Sacchariden oder Harnstoff. Solche besonders bevorzugte festen Produktformen für den Haushalt oder den Gewerbesektor sind Blätter ("sheets"), Stücke ("Bars"), Flocken, (z.B. Extrudiert), Nudeln ("Noodles"), Pearls, Pastillen, Pucks, Sticks, Kapseln, gehärtete Schmelzen;

Weitere bevorzugte Ausführungsformen für den Haushalt oder den Gewerbesektor mit adsorbierten Tensiden auf Sacchariden oder Harnstoff sind Pulver, Tabletten und Granulate . Beispiele bevorzugten fester Produkte sind Toilettenblocks, Weichspüler, Putzsteine, feste Waschmittel, feste Reinigungsmittel, Reinigungsmittel zum Auflösen, ggf. vordosiert.

Alternativ bevorzugt sind weiterhin feste Ausführungsformen für den Haushalt oder den Gewerbesektor enthaltend zusätzlich mindestens ein N-Acylglycaminen.

Weitere bevorzugte Ausführungsformen der festen Formulierungen sind kosmetische Produkte, besonders bevorzugt ausgewählt aus Produkten für die Haut- und Haarpflege oder Haut- und Haarreinigung, insbesondere Deodorants und Antitranspirantien, Mund- und Zahnhygiene, Haut- und Haarreinigung, Haut- und Haarkonditionierung, Haut- und Haarmasken und - packungen. Die Haut- und Haarreinigung schliesst Wasch-, Dusch- und Badepräparate, Gesichtsreinigung, Haarpackungen, Gesichtsmasken, Rasiermittel, Handseifen, Syndets, Haarwaschmittel, Shampoos, Haarpflegemittel, Haarspülungen, Badezusätze, Make-up Entferner .

Bevorzugt sind diese festen Formulierungen rinse-off-Produkte, welche nach Anwendung vorzugsweise mit Wasser abgespült werden. Besonders bevorzugt sind die festen rinse-off-Produkte Reinigungsprodukte für Haut oder Haar, äusserst bevorzugt Reinigungsprodukte für die Haut.

Gegenstand der Anmeldung ist weiterhin die Verwendung der festen Formulierungen für die kosmetische Haut- und Haarreinigung oder -pflege wie oben ausgeführt, bevorzugt die Verwendung der festen rinse-off-Produkte als Reinigungsprodukte für Haut oder Haar, äusserst bevorzugt dieVerwendung der Reinigungsprodukte für die Haut. sowie ein Verfahren für die Herstellung der adsorbierten Tenside auf Sacchariden oder Harnstoff, und ein Verfahren zur Herstellung fester Produkte mit diesen adsorbierten Tensiden.

Besonders bevorzugt enthält die Formulierung zusätzlich zu dem mindestens einen Biotensid mindestens ein weiteres Tensid ausgewählt aus der Gruppe umfassend: Seifen, Acylisethionate, Acyl Methyl Isethionaten, Tauraten, Sodium Acyl Methyl Tauraten, Sulfaten, z.B. Cocosulfate; Sodium lauryl sulfate, Alkylpolyglycoside, z.B. Cocoglucosid, N-Acylglucamide, Sulfonaten, Alpha Olefin Sulfonaten, Sulfoacetaten, Sulfosuccinate, z.B. Alkyl Sulfosuccinaten, z.B. Disodium Lauryl Sulfosuccinat, Betainen z.B. Cocobetain, Alkylamidopropylbetainen, z.B. Cocoamidopropylbetain, Glycinaten, Glutamaten und deren Kombinationen. Die anionischen Tenside können jeweils als Säure oder Salze vorliegen, vorzugsweise als Alkali-, Erdalkalisalze, Ammoniumsalze oder als Salze organischer Basen, z.B. organische primäre, sekundäre oder tertiäre Amine.

Besonders bevorzugt sind feste Tensidzusammensetzungen enthaltend mindestens ein Biotensid, und zusätzlich enthaltend:

- mindestens ein weiteres Tensid ausgewählt aus den Seifen oder Fettsäuren, z.B. Natriumstearat, Natriumpalmitat, Kokoseifen, bevorzugt liegt die Seife oder die Fettsäuren als ein Gemisch von Fettsäuren oder Seife aus einem Pflanzenöl vor, insbesondere bevorzugt entsteht das Gemisch von Fettsäuren bzw. die Seifen aus der Umsetzung eines Pflanzenöls, ganz besonders bevorzugt ohne Auftrennung der Fettsäurefraktionen.
- mindestens ein weiteres Tensid ausgewählt aus Alkyl(poly)glucosid , bevorzugt Coco Glucoside und Sulfaten, bevorzugt Sodium Coco-Sulfate, Sodium Cetearyl Sulfate, Sodium lauryl sulfate und deren Kombinationen.
- mindestens ein weiteres Tensid ausgewählt aus den Sulfaten, bevorzugt Sodium Coco-Sulfate, Sodium Cetearyl Sulfate, Sodium lauryl sulfate und einem Betaintensid, z.B. Cocoamidopropylbetain, Cocobetain, oder deren Kombinationen.
- Bevorzugt mindestens ein weiteres Tensid ausgewählt aus der Gruppe der N-Acylglycamine, vorzugsweise gemäss oben beschriebenen bevorzugten Strukturen und deren Kombinationen
- mindestens ein weiteres Tensid ausgewählt aus der Gruppe der Sulfate, bevorzugt Sodium Coco-Sulfate, und eine weiteres N-Acylglycamin, bevorzugt Capryloyl/Caproyl Methyl Glucamide, Lauroyl/Myristoyl Methyl Glucamide und/oder Cocoyl Methyl Glucamide und optional Seife oder Fettsäuren, z.B. der Stearinsäure und deren Kombinationen.
- mindestens ein weiteres Tensid ausgewählt aus den Isethionaten, insbesondere den Sodium Acylisethionaten, Sodium Acyl Methyl Isethionaten bevorzugt Sodium cocoyl isethionate, Sodium cocoyl methyl isethionate, Sodium lauroyl isethionate, Sodium lauroyl methyl isethionate, und deren Kombinationen.
- mindestens ein Tensid ausgewählt aus den Isethionaten und mindestens ein weiteres Tensid ausgewählt aus, Seifen oder Fettsäuren, Sodium Acylisethionaten, Sodium Acyl Methyl Isethionaten, Tauraten, Sodium Acyl Methyl

Tauraten, Sulfaten, z.B. Cocosulfate; Sodium lauryl sulfate, Alkylpolyglycoside, z.B. Cocoglucosid, weitere N-Acylglucamide, Sulfonaten, Sulfoacetaten, Alpha Olefin Sulfonaten, Disodium Alkyl Sulfosuccinaten, Betainen z.B. Cocobetain, Alkylamidopropylbetainen, z.B. Cocoamidopropylbetain, Glycinaten, Glutamaten, und deren Kombinationen.

- mindestens ein Tensid ausgewählt aus den Betainen z.B. Cocobetain oder Alkylamidopropylbetainen, z.B. Cocoamidopropylbetain, und deren Kombinationen.
- mindestens ein Tensid ausgewählt aus den Aminosäuretensiden z.B. Glycinate, Glutamate und oder Tauraten, und deren Kombinationen.
- und mindestens Tensid ausgewählt aus der Gruppe umfassend Sulfoacetate, besonders bevorzugt Sodium Lauryl Sulfoacetate und Sulfosuccinat, bevorzugt Disodium Alkyl Sulfosuccinaten, z.B. Disodium Lauryl Sulfosuccinat und deren Kombinationen.

Typische Zusammensetzungen fester Formulierungen, insbesondere von Rinse-off-Formulierungen, für die Reinigung von Haut und Haar bestehen aus dem Biotensid, bevorzugt einem weiteren Tensid vorzugsweise wie oben als bevorzugt beschrieben, Konsistenzgeber, Trägermaterial oder Füllstoffe, pH- Stellmittel, bevorzugt Emollients, optional Konditionierungsmittel, Lösungsmittel, Duftstoffe, Wirkstoffe, z.B. Antioxidantien, Farbstoffe, Konservierungsmittel, Stabilisatoren, Emulgatoren, Pigmente, Abrasiva, Hilfsstoffe.

Die festen Formulierungen enthalten Konsistenzgeber, Trägermaterial oder Füllstoffe bevorzugt ausgewählt aus der Gruppe der hydrogenierten Pflanzenöle, Fettalkohole, Fettsäuren, Wachse, Pflanzenbutter, Saccharide, Harnstoff, wie oben beschrieben, bevorzugt sind diese Konsistenzgeber bei Raumtemperatur fest.

Die festen Formulierungen enthalten pH- Stellmittel bevorzugt ausgewählt aus den Gruppen der Milchsäure, Citronensäure, Äpfelsäure, Glykolsäure und deren Salze und/oder gegebenenfalls Bicarbonat.

Die festen Formulierungen enthalten bevorzugt Emollients ausgewählt aus der Gruppe der Pflanzenöle, Fettsäureester, Fettalkoholether, Fettsäureglyceride, Fettsäurepolyglyceride oder Emollients aus der Gruppe der Konsistenzgeber.

Die festen Formulierungen enthalten bevorzugt Konditionierungsmittel ausgewählt aus der Gruppe der Proteinhydrolysate oder kationischen Verbindungen, z.B. quaternisierten Ammoniumverbindungen, wie Cetrimonium chloride, Amidoester, Amidoamine, Polyquaternium-7, quaternisierte Polymerverbindungen, z.B: Guar hydroxypropyltrimonium chloride, Inulin und der Tenside K, vorzugsweise entsprechend der als bevorzugt definierten Vertreter.

Die festen Formulierungen enthalten bevorzugt Konservierungsmittel ausgewählt aus der Gruppe der Sodium Levulinate, Caprylyl Glycol, Milchsäuren, Sorbinsäuren, Benzoesäuren und deren Salzen, z.B. Benzoesäure, Natrium oder Kalium Benzoat, Potassium Sorbate, Sorbic Acid,

Hilfsstoffe sind beispielsweise Komplexbildner, Feuchthaltemittel, Stabilisatoren, Antioxidationsmittel, Verdicker, z.B. Lecithin, Gleitmittel (Lubricants), anorganische Salze, Farbschutzmittel.

Wirkstoffe sind beispielsweise Antischuppenmittel, Pflanzenextrakte, Koffein, Panthenol, Vitamine, physiologisch wirksame Aktivstoffe ("Actives"), Anti-ageing, UV-absorber, Charcoal powder, Teebaumöl, Allantoin, Tocopherol

Eine besonders bevorzugte Zusammensetzungen fester Formulierungen, bevorzugt Rinse-Off-Formulierungen, für die Reinigung von Haut und Haar besteht aus 0,2-40 Gew.-% Biotensid, 0- 50 Gew.-% eines weiteren Tensids, bevorzugt ausgewählt aus der Gruppe umfassend Seifen bzw. Fettsäuren, Acylisethionaten, Acyl Methyl Isethionaten, Tauraten, Acyl Methyl Tauraten, Sulfaten, Sulfonaten, Alpha Olefin Sulfonaten, Sulfoacetaten, Sulfosuccinaten, sowie deren Kombinationen, optional 0 - 20 Gew.% eines weiteren Tensids bevorzugt ausgewählt aus Betainen, Alkylamidopropylbetainen, Glycinaten, Glutamaten, Alkylpolyglycoside, N-Acylglucamide, N-Acylglycamine und deren Kombinationen, bevorzugt 20-55 Gew.-% von mindestens einem Inhaltsstoffe ausgewählt aus den Konsistenzgebern, Füllstoffen, Trägermaterial und Emollients, Wasser oder Lösungsmittel bevorzugt 0-8 Gew.%, bevorzugt unter 5 Gew.% je nach Produkt optional Konditionierungsmittel, Duftstoffe, Wirkstoffe, Farbstoffe, Emulgatoren, Konservierungsmittel, Pigmente, Abrasiva, Hilfsstoffe.

Folgende Kombinationen sind für die festen Formulierungen besonders bevorzugt:

- Mindestens ein Biotensid + bevorzugt mindestens weiteres Tensid, vorzugsweise wie vorhergehend als bevorzugt beschrieben + mindestens eine Fettsäure und/oder Fettalkohol, insbesondere als Konsistenzgeber, Trägermaterial oder Füllstoffe, bevorzugt ausgewählt aus Stearinsäure, Myristinsäure, Palmitinsäure, Cetylalkohol, Cetearyl alkohol, Stearylalkohol, Isostearyl Alcohol, und deren Mischungen, besonders bevorzugt ist die Fettsäure und/oder der

Fettalkohol abgeleitet aus eine C-18-Pflanzenöl., z.B. Oleoylalkohol, Brassica-Alkohol, Ölsäure, Arachidyl Alcohol, Behenyl alkohol u.s.w.

Bevorzugt weisen die Fettsäuren und Fettalkohole oder deren Mischungen einen Schmelzpunkt zwischen 28 und 40°C auf.

Mindestens ein Biotensid + bevorzugt mindestens weiteres Tensid, vorzugsweise wie vorhergehend als bevorzugt beschrieben + mindestens ein Wachs oder Butter, insbesondere als Konsistenzgeber, Trägermaterial oder Füllstoffe z.B. Bienenwachs, Carnaubawachs., Butyrospremum parkii butter, Theobroma cacao seed butter, Mango butter, Mandel Butter, Candelilla Wachs, Oryza sativa cera, hydrierte Pflanzenölglyceride, bevorzugt Butyrospremum parkii butter, Theobroma cacao seed butter, und deren Mischungen, besonders bevorzugt sind die Wachse oder Butter abgeleitet aus eine C-18-Pflanzenöl., z.B. Sunflower seed wax, hydriertes Sonnenblumenöl, hydriertes Rapsöl, hydrogenated vegetable oil. Desweiteren bevorzugt weisen die Wachse oder Butter oder deren Mischungen einen Schmelzpunkt zwischen 28 und 40°C auf.

Mindestens ein Biotensid + bevorzugt mindestens weiteres Tensid, vorzugsweise wie vorhergehend als bevorzugt beschrieben + mindestens ein Saccharid als Konsistenzgeber, Trägermaterial oder Füllstoffe, bevorzugt ausgewählt aus den Sacchariden wie vorhergehend beschrieben.

Mindestens ein Biotensid + bevorzugt mindestens weiteres Tensid, vorzugsweise wie vorhergehend als bevorzugt beschrieben + mindestens ein Pflanzenöl oder Fettsäureglycerid, insbesondere als Emollients, z.B. Kokosöl, Jojobaöl, Glycerylstearat, Glyceryl caprylate/caprate etc., Arganöl, Jojobaöl, Marulaöl, bevorzugt abgeleitet aus eine C-18-Pflanzenöl., z.B. Sonnenblumenöl, Glyceryloleat, Olivenöl, Rapsöl, Aprikosenkernöl, Rapsglyceride, Mandelöl (Sweet almond oil). u.s.w., sowie deren Mischungen.

Mindestens ein Biotensid + bevorzugt mindestens weiteres Tensid, vorzugsweise wie vorhergehend als bevorzugt beschrieben + mindestens ein Lösungsmittel, insbesondere ausgewählt aus den Alkoholen und Fettsäureester und -ethern, bevorzugt Propylenglykol, Butylenglykol, Pentylenglycol, Propandiol, Caprylic/Capric Triglyceride, Isopropyl myristate, Propylenglykol Di (Caprylate/Caprate), Ethanol, Glycerin, Isopropanol, Propanol, Dipropylenglykol, Dicaprylyl ether, sowie deren Mischungen besonders bevorzugt Glycerin, Ethanol, Propylenglykol, Butylenglykol, ganz besonders bevorzugt Propylenglykol, sowie deren Mischungen

Mindestens ein Biotensid + bevorzugt mindestens weiteres Tensid, vorzugsweise wie vorhergehend als bevorzugt beschrieben + mindestens ein Komplexbildner bevorzugt - bevorzugt ausgewählt aus Tetrasodium Glutamate Diacetate (GLDA), Tetraacetylethylendiamin (TAED), Methylglycindiessigsäure (MGDA), Carboxymethyl Inulin, Zitronensäure, Natrium gluconat, Ethylendiamintetraacetat (EDTA), Dicarboxymethyl Alaninate, Polyasparaginsäure, Gluconolacton, Bernsteinsäurederivate, insbesondere Iminodibernsteinsäure (IDS), oder Ethylendiamindibernsteinsäure EDDS, jeweils als Salz, insbesondere als Alkalisalze oder in der Säureform, sowie deren Kombinationen

- Mindestens ein Biotensid + bevorzugt mindestens weiteres Tensid, vorzugsweise wie vorhergehend als bevorzugt beschrieben + mindestens ein Polymer, z.B. Xanthan gum, Guar gum, Guar hydroxypropyltrimonium chlorid, Sclerotiumgum, und deren Mischungen

- Mindestens ein Biotensid + bevorzugt mindestens weiteres Tensid, vorzugsweise wie vorhergehend als bevorzugt beschrieben + mindestens ein Pflanzenextrakt oder Aroma, z.B. Aloe Barbadensis Leaf Juice Powder, Citrus aurantium dulcis peel oil, Camelia sinesis (green tea) extract, Rosmarinus Officinalis Leaf, Peppermint oil, Salvia Officinalis Leaf Powder, Eucalyptus Globulus Leaf Oil, Chamomilla Recutita Flower Extract, Menthol, und deren Mischungen

- Mindestens Biotensid + bevorzugt weiteres Tensid, vorzugsweise wie vorhergehend als bevorzugt beschrieben + mindestens ein Emulgator ausgewählt aus der Gruppe der Sorbitanester, auch ethoxyliert, und der Polyglycerylester, besonders bevorzugt ist der Sorbitanester ausgewählt aus einem Ester mit C-18-Pflanzenölfettsäuren, inbesondere Sunflower seed oil Sorbitan esters und Sorbitan olivate und deren Mischungen.

- Mindestens ein Biotensid + bevorzugt weiteres Tensid, vorzugsweise wie vorhergehend als bevorzugt beschrieben + Polyvinylalkohole.

Verfahren zur Herstellung fester Formulierungen:

Bevorzugt werden feste erfinderische Ausführungsformen mit mindestens einem Biotenside oder mindestens einem N-Acylglycamin, oder mit einem Gemisch derselben, vorzugsweise entsprechend der bevorzugten Ausführungsformen, zusätzlich enthaltend Sacchariden, vorzugsweise mit den Saccharid-Vertretern wie vorhergehend (unter "weitere Inhaltsstoffe") beschrieben, ganz besonders bevorzugt als Tensid-Saccharriden-Aggregate. Eine alternative Ausfüh-

rungsform zu den Sacchariden ist Harnstoff. Bevorzugt wird Harnstoff in Reinigungs-oder Pflegemittel für Haushalt oder den Gewerbesektor verwendet.

Saccharide werden besonders bevorzugt ausgewählt aus der Gruppe umfassend Monosaccharide, Oligosaccharide, bevorzugt bis 10 Saccharideinheiten, Amylose, Amylopectin, Sorbitol, Maltodextrin, Isomalt, Xylitol, Pectine, Carrageenan, Xanthane, Dextrane, Dextrin, Fructane, Alginate, Gellan, Guar, Glucane, Stärke, modifizierte Stärke, sowie Stärke- und Cellulosenderivate und Mischungen derselben, in einer bevorzugten Ausführungsform sind die Trägersaccharide aufgrund der Ökonomie und Eigenschaften ausgewählt aus der Gruppe der Monosacchariden Mannose, Ribose, Rhamnose, Galactose, ausgewählt aus der Gruppe der Oligosacharide Lactose, Trehalose, Cellobiose, Raffinose, ausgewählt aus der Gruppe der Fructane die Levane, Phleine und Neokestosen, ausgewählt aus der Gruppe der Cellulosederivate die neutral geladenen Cellulosederivate, zusätzlich besonders bevorzugt sind die Stärkederivate, Sorbitol, Isomalt, Xylitol, und Pectin, sowie deren Mischungen. Überraschenderweise wurde festgestellt, dass die Zuckertenside, N-Acylglycamine und Biotenside, und deren erfinderischen Zusammensetzungen besonders gut mit Sacchariden aggregieren, sie können als Aggregat in den Produkten eingesetzt werden, können leicht in Wasser - auch hartem Wasser- dispergiert bzw. gelöst werden können und bilden keine Rückstände. Sie sind biologisch abbaubar und werden aus erneuerbaren, gut verfügbaren Quellen durch ein CO2-armes Verfahren gewonnen. Die Produkte mit den Saccharid-Aggregaten zeigen keine verhärtende Oberflächen oder ungewolltes schnelles Auflösen. Die Produkte verfügen insbesondere über eine genügenden Halt während der Anwendung, z.B: in den Handflächen und sorgen so für eine angenehme Handhabung, insbesondere eine Rutschfestigkeit bei der Handhabung. Die Produkte weisen zudem bei Anwendung eine gute Sensorik auf. Diese besonderen Eigenschaften zeichnen die Saccharid- aggregate mit den erfinderischen Zuckertensiden N-Acylglycaminen oder den Biotensiden oder deren Kombinationen aus.

Ein weiterer Aspekt der Erfindung ist daher die Saccharid-Aggregate und ein Verfahren für deren Herstellung und deren Verwendung; sowie Produkte enthaltend diese Saccharid-Aggregate, ein Verfahren zur Herstellung von Produkten mit Saccharid-Aggregaten und die Verwendung von Produkten enthaltend Saccharid-Aggregate.

Ein weiterer Aspekt der Erfindung ist daher die Harnstoff-Aggregate und ein Verfahren für deren Herstellung und deren Verwendung; sowie Produkte enthaltend diese Harnstoff-Aggregate, ein Verfahren zur Herstellung von Produkten mit Harnstoff - Aggregaten und die Verwendung von Produkten enthaltend Harnstoff -Aggregate.

Ein Aspekt der Erfindung sind Produkte enthaltend solche Harnstoff- oder bevorzugt Saccharid-Aggregate mit mindestens einem Biotensid oder mindestens einem N-Acylglycamin oder deren Kombination. Die Aggregate können auch als Additive verwendet werden.

Ein Gegenstand der Erfindung ist das Verfahren zur Herstellung fester Formulierungen, vorzugsweise wie oben als bevorzugt beschrieben, enthaltend Harnstoff-Tensid-Aggregate oder Saccharid-Tensid-Aggregate mit Biotensiden oder N-Acylglycaminen oder deren Kombination, umfassend folgende Verfahrensschritte:

a) Bereitstellen eines festen organischen Materials, bevorzugt ein Harnstoff oder Saccharid, besonders bevorzugt ein Saccharid

b) Bereitstellen mindestens eines Tensids ausgewählt aus den Biotensiden und N-Acylglycaminen in fliessfähiger Form, bevorzugt als Lösung oder als Schmelze, besonders bevorzugt als Lösung

c) In Kontakt bringen des festen Materials mit dem mindestens einen Tensid in fliessfähiger Form (Aggregation)

d) Für feste Produkte, Verfestigung des Aggregats durch Temperatursenkung oder teilweise oder vollständige Trocknung

e) Optional Konfektionierung der festen Formulierung z.B. durch Pressung, Tablettierung, Mahlen, u.s.w.

f) Optional Mischen des Harnstoff-Tensid-Aggregats oder des Harnstoff-Tensid-Aggregats mit weiteren Inhaltsstoffen, inbesondere nach einem der Verfahren ausgewählt aus dem Extruded-Molten-Prozess, Extruded-Flake-Prozess, Melt & Pour-Prozess, oder Kompressions-Prozess oder Bereitsstellung weiterer Inhaltsstoffe als zusätzliche Kompartimente zum Produkt, gegebenenfalls nach einem der genannten Prozesse.

g) Optional Konfektionierung des Produktes

Für flüssige Produkte kann direkt das Aggregat aus Schritt c) für Flüssigformulierungen verwendet werden.

In mindestens einer weiteren Ausführungsform, werden die Biotensiden und/oder N-Acylglycaminen gemeinsam mit weiteren Inhaltsstoffen auf dem organischen Material adsorbiert. Beispiele für geeignete Co-adsorbierende Tenside sind die Tenside K, kationische Tenside, organische Aminverbindungen, amphotere Tenside, weitere geeignete co-adsorbierende Inhaltsstoffe sind kationische Polymere. Desweiteren können aus verfahrenstechnischen Gründen Ölkörper wie z.B. Fettalkohole, Butter, Wachse, Ester, gleichzeitig auf das organische Material mit aufgebracht werden.

Die Tenside und Saccharide entsprechen vorzugsweise den in der Anmeldung als bevorzugt, besonders bevorzugt, etc. definierten Vertretern. Bei dem Lösungsmittel eignen sich äussert bevorzugt C1-C8-Alkohole, Glykole, Glycerin oder Wasser, insbesondere enthaltend Wasser. Die fliessfähige Form des Tensids kann als Schmelze mit einer bevorzugten Konzentration von 60-100% bezogen auf die Schmelze vorliegen. Die Schmelze ist bevorzugt für N-Acylglycamine. Bevorzugte Temperatur für das N-Acylglycamin--Aggregat liegt bei 50-80°C. Die Verfestigung erfolgt durch

Abkühlen zu Temperaturen < 50°C, idealerweise auf Raumtemperatur. Biotenside und Gemische von Biotensiden mit N-Acylglycaminen werden bevorzugt als Lösungen oder Dispersionen, insbesondere in Wasser, Alkoholen, Glykolen, Glycerin oder deren Mischungen, mit dem aggregierenden Material in Kontakt gebracht. In Lösung oder Dispersion liegt das jeweils mindestens eine Tensid in einer Konzentration von 10-90 Gew.%, bevorzugt 30 - 78 Gew.-%, ganz besonders bevorzugt 40-60 Gew.-% vor. In-Kontaktbringen kann über z.B. über Aufsprühen, Aufstreichen, Beschichten, Mischen der Komponenten, Tunken erfolgen. Aufsprühen kann bspw. durch einen Wirbelschicht-Sprühgranulator oder Pflugscharmischer erfolgen; via Top-Spray, Bottom-Spray oder Tangential-Spray-Verfahren, bevorzugt dem Top-Spray-Verfahren.

Der Trocknungsschritt zur Verfestigung der Aggregate im Fall von Lösungen oder Dispersionen kann im Vakkum oder bei Normaldruck und/oder thermisch, einschliesslich bei Raumtemperatur (20-25°C), erfolgen. Erfindungsgemäss bevorzugt ist eine Trocknungstemperatur bei Anwendung von wässrigen oder alkoholischen Lösungen zwischen 20 und 100 Grad C, besonders bevorzugte Temperatur bei Normaldruck zwischen 60 und 80°C. Bei glykolischen Lösungen, z.B: in Propylenglykol sind die bevorzugten Trocknungstemperaturen im Vakuum anzuwenden.

Die Aggregate können nach der Aushärtung weiter konfektioniert werden, z.B. Granuliert, gemahlen, extrudiert, tablettiert, etc.. Vorteile dieser Aggregate ist, dass die Tenside in eine stabile, handhabbare, dosierbare, gegebenfalls ästhetische Form gebracht werden, die die Lagerung, den Verkauf als Rohmaterial, und die Verwendung in folgenden Herstellprozessen erleichtert. Vorteil ist weiterhin, dass das organische Material aus natürlichen Quellen stammt und biologisch abbaubar ist, so dass sich die Tensid-Aggregate auch für Verwendungen in der freien Natur, Agroanwendungen, gegebenenfalls in Kombination mit einem Wirkstoff, Herbizid, Pestizid, Insecticide, wie , Bodenreinigung, Öl- und Erzgewinnung eignen. Desweiteren können die Tensid-Aggregate für die Herstellung von Wasch- und Reinigungsmitteln, für die Herstellung von kosmetischen Mitteln, für die Papierherstellung, für Textilanwendungen, für die Kosmetik, für medizinische Zwecke verwendet werden.

Gegenstand der Erfindung sind daher auch Zusammensetzungen enthaltend die Tensid-Saccharid-Aggregate oder Tensid-Harnstoff-Aggregate. Erfindungsgemäss können Zusammensetzungen von 0,2 bis 100 Gew.-% dieser Aggregate enthalten. Feste Wirkstoffe oder Komponenten können durch den Verfahrensschritt f) aufgebracht werden. Flüssige Wirkstoffe oder Komponenten können durch Aufsprühen, Aufstreichen, Beschichten, Mischen der Komponenten, Tunken auf das Tensid-Saccharid-Aggregate oder Tensid-Harnstoff-Aggregate aufgebracht werden. Verfahren hierzu sind in dieser Anmeldung beschrieben.

Exemplarisch wird das Verfahren zur Herstellung der festen Reinigungs- oder Pflegeprodukte enthaltend die Tensid-Aggregate beschrieben:

a) Bereitstellen des Tensid-Saccharid-, oder Tensid-Harnstoff-Aggregats
b) In Kontakt bringen des festen Aggregats mit dem mindestens einer weiteren festen Komponente
c) Überführung in die Produktform über ein Verfahren ausgewählt aus dem Mischprozess, Granulierungsprozess, Tablettierungsprozess, Extruded-Molten-Prozess, Extruded-Flake-Prozess, Melt & Pour-Prozess, oder Kompressions-Prozess .
d) Konfektionierung des Produktes

Mit diesem Verfahren können Pulver, Granulate, Tabletten, aber auch Bars, Sticks, Pastillen us.w. gefertigt werden. Geeignet ist dieses Verfahren beispielsweise für Waschpulver, Geschirrspültabs, insbesondere mehrschichtig, feste Shampoo, feste Konditioner, Deos, u.s.w.

Exemplarisch wird das Verfahren zur Herstellung der fliessfähigen Reinigungs- oder Pflegeprodukte enthaltend die Tensid-Aggregate beschrieben:

a) Bereitstellen des Tensid-Saccharid-, oder Tensid-Harnstoff-Aggregats
b) In Kontakt bringen des festen Aggregats mit mindestens einer weiteren Komponente, die bei 20-25°C in fliessfähiger Form vorliegt. Dies kann eine Reinsubstanz oder eine Emulsion oder Lösung sein. Das Lösungsmittel ist dabei bevorzugt ausgewählt aus protischen Lösungsmitteln, insbesondere Wasser, Alkoholen, Glykolen, Glycerin.
c) Homogenisieren der Mischung

Das Verfahren ist besonders geeignet für pastöse oder hochviskose Produktformen, wie zum Beispiel für Zahnpasta, Scheuermittel, Peelings, u.s.w.

Erfindungsgemäss eignen sich auch alternative oder zusätzliche Verfahren zur Herstellung fester Formulierungen enthaltend mindestens ein N-Acylglycamine oder mindestens ein Biotenside oder deren Mischungen, wobei die Inhaltsstoffe ohne Aggragatsbildung gemischt werden; bevorzugt werden jedoch auch in diesen Verfahren Aggregate gemäss dem erfindungsmässigen Verfahren eingesetzt. Vorteil der Aggregate ist hierbei, vereinfachtes Handling und Transport, flexibler Einsatz in unterschiedlichen Herstellungsverfahren, z.B. neben den genannten Verfahren Pulverherstellung oder Flüssigprodukte, und damit der Möglichkeit des Einsatzes in energiesparenden Herstellverfahren,

insbesondere dem Extruded-Flake-Prozess oder dem Kompressions-Prozess, und der mögliche Einsatz bei Verwendung von wärmeempfindliche Inhaltsstoffen. Ein weiterer Vorteil dieser Aggregate ist die Lagerstabilität und die Reduktion oder Verzicht auf Konservierungsmittel.

Vorteil von Produkten enthaltend Tensid-Aggregate ist die erhöhte Stabilität und die kontrollierte, dosierte Abgabe von Tensiden bei Gebrauch der Produkte.

Weitere Herstellverfahren für feste Formulierungen enthaltend mindestens ein Biotensid, bevorzugt als Tensid-Aggregat, ist das Schmelzen und Giessen (Melt & Pour-Prozess) mit den bevorzugten Prozessschritte:

a) Vermischen aller Inhaltsstoffe mit ausreichender Erwärmung und Agitation zu einer Schmelze
b) Giessen des Gemischs in eine Form
c) Aushärtung des Gemischs durch Abkühlung oder abkühlen lassen,

der Extruder- Schmelzprozess (Extruded-Molten-Prozess) mit den bevorzugten Prozessschritten Vermischen der Inhaltsstoffe mit ausreichender Erwärnumg und Agitation (Schmelze), Abkühlen und Zerkleinern des Gemischs, z.B. in einem Walzenstuhl, gegenenfalls Vermischen in einem Amalgamator, Extrudieren, Konfektionieren (z.B. Zuschneiden der Seife), gegebenenfalls Verdichten durch Pressung;
der Extruder - Flocken-Prozess (Extruded-Flake-Prozess): Vermischen der Inhaltsstoffe, z.B. in einem Amalgamator, Extrudieren, Konfektionieren (z.B. Zuschneiden der Seife), gegebenenfalls Verdichten durch Pressung; und der Kompressions-Prozess: Vermischen der Inhaltsstoffe, z.B. in einem Amalgamator, Einfüllen des Gemischs in Formen, Verdichtung durch Pressung.

Emulsionen & Cremes

Mindestens eine bevorzugte Ausführungsform der Zusammensetzungen enthaltend mindestens ein N-Acylglycamin und bevorzugt mindestens ein Biotensid, sind Hautpflegemittel in Form von Emulsionen, Cremes oder Lotionen, insbesondere Pflegecremes und -lotionen, Sonnenschutzcremes, O/W-Formulierungen, W/O-Formulierungen, Träger für medizinisch oder dermatologische Wirkstoffe, z.B. Aknewirkstoffe, Medikamente, Antimykotika, u.s.w. Besonderer Vorteil der erfinderischen Zusammensetzungen ist ein überraschend gutes Emulgierverhalten und ein gutes Dispergiervermögen. Die Produkte weisen eine gute Sensorik und ein gutes Umweltverhalten auf. Weiterer Vorteil ist die geringe Schleimhautreizung der Produkte. Weiterer Gegenstand der Erfindung ist die Verwendung der Mittel als Haut- oder Haarreinigungs- oder -pflegemittel wie beschrieben. Besonders vorteilhaft für das Emulgieren haben sich die Gemische von N-Acylglycaminen mit unterschiedlichen Fettsäureresten gezeigt. Bevorzugt sind Emulsionen enthaltend mindestens ein Gemisch von N-Acylglycaminen mit einer Verteilung der Fettsäureacylreste RCO entsprechend der Verteilung der Fettsäureacylreste des C-18-Pflanzenöls. Eine weitere Ausführungsform sind Emulsionen enthaltend mindestens ein N-Acyl-N-methyl cyclic glucamide und bevorzugt mindestens ein Biotensid. Eine weitere Ausführungsform sind Emulsionen enthaltend mindestens ein N-Acyl-N-methyl cyclic glucamide und mindestens ein N-Acyl-N-Methylglucamid abgeleitet aus einem C-18-Pflanzenöl und bevorzugt mindestens ein Biotensid. Ein Erfindungsgegenstand ist weiterhin die Verwendung der Zusammensetzung enthaltend mindestens ein N-Acylglycamin und bevorzugt enthaltend mindestens ein Glycolipid-Biotensid, für Emulsionen, Cremes und Lotionen.

Zahn- und Mundpflege

Ein weiterer Gegenstand der Erfindung ist ein Mundreinigungs- oder -pflegemittel, insbesondere eine Zahncreme, Zahnseide oder Mundspülung, besonders bevorzugt eine Zahncreme oder Mundspülung, enthaltend mindestens ein N-Acylglycamin, insbesondere entsprechend den als bevorzugt beschriebenen Vertretern, und bevorzugt zusätzlich enthaltend mindestens ein Glycolipid-Biotensid umfassend Rhamnolipide, Sophorolipide, Mannosylerythritollipide, Cellobioselipide, Trehaloselipide und deren Kombinationen. Bevorzugt enthalten die Zahn- und Mundpflegeprodukte zusätzlich Inhaltsstoffe ausgewählt aus den Gruppen Wirkstoffe, insbesondere Fluoride; Antiplaque-Mittel; Komplexbildner, z.B. Bisphosphonate, Zink-Verbindungen; Alkohole, z.B. Ethanol; Pflanzenextrakte; Glycerin; Adstringentien; Enzyme; Poliermittel, z.B. Kreide, CaCOs, Mica, Calciumhydrogenphosphat und deren Hydrate, Aluminiumoxid und -hydrate, Natriumaluminiumsilicate, Zeolithe, etc.; Lösungsvermittler; Verdickungsmittel z.B. Carrageenane, Guar, Xanthan gum, Carubin, Tragant; Feuchthaltemittel wie Glykole; Aromastoffe, z.B. Menthol, natürliche Öle; Süssstoffe, z.B. Natriumsaccharinat; Cyclamat, Stevia, Xylitol, Glycyrrhizin; Bleichmittel, z.B. Peroxide; Sorbitol; Aktivkohle, optional Konservierungsmittel und/ oder Farbstoffe; gegebenenfalls weitere Tenside, bevorzugt Sodium lauryl sulfate, Coco amidopropylbetaine, Aminosäuretenside; sowie Kombinationen dieser Inhaltstoffe. Ein Erfindungsgegenstand ist weiterhin die Verwendung der Zusammensetzung enthaltend mindestens ein N-Acylglycamin und bevorzugt enthaltend mindestens ein Glycolipid-Biotensid, für die Reinigung oder Pflege von Mundhöhle und Zähnen.

Haarverformung, Rasur & Haarentfernung

Ein weiterer Gegenstand der Erfindung ist ein Rasier- , Haarverformungs- oder Haarentfernungsmittel, enthaltend mindestens ein N-Acylglycamin, bevorzugt gemäss beschriebenen bevorzugten Strukturen, und besonders bevorzugt enthaltend mindestens ein Glycolipid-Biotensid. Das Mittel liegt in der festen oder fliessförmigen Ausführungsform vor, insbesondere sind die Mittel Rasierseife, Rasiercreme, Rasierschaum, Rasiergel, Depiliercremes, Depilierlotion, Depilierschaum, Dauerwellmittel, Fixiermittel, Entkräuselungsmittel, Glättungsmittel. Ein Erfindungsgegenstand ist weiterhin die Verwendung der Zusammensetzung enthaltend mindestens ein N-Acylglycamin und bevorzugt enthaltend mindestens ein Glycolipid-Biotensid, für die Rasur, Haarverformung und die Haarentfernung.

Bevorzugte Ausführungsformen für die Haarverformung und Haarentfernung enthalten bevorzugt weitere Inhaltststoffe ausgewählt aus der Gruppe Thiolactat, Thioglycolsäure oder Thioglycolaten, z.B. Ammoniumthioglykolat, Harnstoff, Ammoniak, Natrium hydroxid, Calciumhydroxid, Cetearylalkohol und deren Kombinationen
Dauerwellmittel enthalten bevorzugt Reduktionsmittel, Alkalisierungsmittel und pH-Regulatoren, Penetrationshilfsmittel, z.B. Harnstoff, Glycolether, Duftstoffe, ggf. weitere oberflächenaktive Substanzen, kämmbarkeitsverbessernde Stoffe, z.B: Öle, Silikone, Proteine, Trübungsmittel, z.B. wässrige Polymerdispersionen, und ggf. Farbstoffe, sowie deren Kombinationen. Besonders bevorzugt sind weiterhin die voranstehend beschriebenen Tenside K enthalten, äusserst bevorzugt gemäss den oben beschriebenen Bevorzugungen. Dauerwellfixiermittel enthalten bevorzugt ein Oxidationsmittel, z.B. Wasserstoffperoxid, Stabilisatoren und pH-Regulatoren, ggf. Duftstoffe. Rasierprodukte enthalten bevorzugt weitere Inhaltsstoffe ausgewählt aus der Gruppe Fettsäuren, insbesondere Stearinsäure, Palmitinsäure, Myristinsäure, Behensäure, Arachidonsäure, insbesondere verseift durch Natriumhydroxid, Kaliumhydroxid, organische Amine, z.B. Triethanolamin, Diethanolamin; Emollients, insbesondere Pflanzenöle, - fette, - butter, - wachse, Lanolin; Emulgatoren; Glycerin; Sorbitol, Hautpflege-Actives, wie z.B. Vitamine, Pflanzenextrakte, Actives zur Hautberuhigung, z.B. Bisabolol, u.s.w., optional Parfümöle und/oder Konservierungsmittel, sowie deren Kombinationen. Weiterer Gegenstand der Erfindung ist die Verwendung der Zusammensetzungen als Rasier-, Haarverformungs- oder Haarentfernungsmittel.

Entrostung/ Rohrreiniger

Ein weiterer Gegenstand der Erfindung ist ein Mittel, in der festen oder fliessförmigen Ausführungsform für die Rohrreinigung oder als Entrostungsmittel enthaltend mindestens ein N-Acylglycamin, bevorzugt gemäss beschriebenen bevorzugten Strukturen, und bevorzugt enthaltend mindestens ein Glycolipid-Biotensid. Ein Erfindungsgegenstand ist weiterhin die Verwendung für die Rohrreinigung und Entrostung. Bevorzugte Ausführungsformen enthalten weitere Inhaltststoffe ausgewählt aus der Gruppe Thioglycolsäure oder Thioglycolaten, Thioglykolsäre und Harnstoff, Harnstoff, Natrium hydroxid, Calciumhydroxid, Cetearylalkohol und deren Kombinationen.

Haarfärbemittel

Ein weiterer Gegenstand der Erfindung ist ein Haarfärbmittel, enthaltend mindestens ein N-Acylglycamin, und bevorzugt enthaltend mindestens ein Glycolipid-Biotensid, sowie die Verwendung dieser Tenside für Haarfärbemittel und die Verwendung der Mittel als Haarfärbemittel.

Abrasiva

Ein weiterer Gegenstand der Erfindung ist eine Zusammensetzung enthaltend mindestens ein N-Acylglycamin, und bevorzugt enthaltend mindestens ein Glycolipid-Biotensid, zusätzlich enthaltend ein Abrasivum, insbesondere Scheuermittel für harte Oberflächen, Peeling oder Grobreiniger. Die Zusammensetzung kann in fliessfähiger oder fester Ausführungsform vorliegen. Bevorzugt enthalten diese Zusammensetzungen ein Abrasivum natürlichen Ursprungs ausgewählt aus organischen oder mineralischen Stoffen, besonders bevorzugt ausgewählt aus CaCOs, z.B. Marmormehl, Eierschalenmehl oder Kreide, Quarzmehl, Silikaten, z.B. hydrated silica, kristalline Cellulose, Kaolinen, Tonerde, Kohle, zerkleinerter oder gemahlene Pflanzenteile oder Algen, z.B. Kernmehle, Walnussschalenmehl, Weizenkleie, und Wachse z.B. Jojoba wax beads. Ein Erfindungsgegenstand ist weiterhin die Verwendung der Zusammensetzung enthaltend mindestens ein N-Acylglycamin und bevorzugt enthaltend mindestens ein Glycolipid-Biotensid, für die vorgenannten Verwendungen.

Waschmittel

Ein weiterer Gegenstand der Erfindung ist ein Waschmittel für Textilien, Polster, Fasern und Teppiche enthaltend mindestens ein N-Acylglycamin (bevorzugt gemäss den als bevorzugt definierten Strukturen), und bevorzugt zusätzlich enthaltend mindestens ein Biotensid, in fester oder fliessfähiger Ausführungsform oder deren Kombination, z.B. in

unterschiedlichen Kompartimenten. Das Waschmittel kann als Pulver, Tabs, Waschblättern, Pods, Gel, Kompaktat, Konzentrat, Lösung vorliegen, portioniert, z.B: in Plastik- oder wasserlöslichem Polymer, oder frei dosierbar. Waschmittel können in der Form von Vollwaschmittel, Colorwaschmittel, Feinwaschmittel, Wollwaschmittel, Spezialwaschmittel, z.B. für schwarze Wäsche, für Sportkleidung, Babywaschmittel, Sensitiv-Waschmittel, Hygienewaschmittel, Waschmittel für die Handwäsche, Waschmittel für Industrie oder Gewerbe, u.s.w. , Fleckenmitteln, Vorbehandlungsmitteln, Waschadditiven vorliegen. Ein Erfindungsgegenstand ist weiterhin die Verwendung der Zusammensetzung enthaltend mindestens ein N-Acylglycamin und bevorzugt enthaltend mindestens ein Glycolipid-Biotensid, für die vorgenannten Verwendungen.

Geschirrspülmittel

Ein weiterer Gegenstand der Erfindung ist ein Geschirrspülmittel enthaltend mindestens ein N-Acylglycamin (bevorzugt gemäss den als bevorzugt definierten Strukturen), und bevorzugt zusätzlich enthaltend mindestens ein Biotensid, in fester oder fliessfähiger Ausführungsform oder deren Kombination, z.B. in unterschiedlichen Kompartimenten. Das Geschirrspülmittel kann als Pulver, Tabs, Waschblättern, Pods, Gel, Kompaktat, Konzentrat, Lösung vorliegen, portioniert, z.B: in Plastik- oder wasserlöslichem Polymer, oder frei dosierbar. Geschirrspülmittel sind für das manuelle Geschirrspülen, sowie für das maschinelle Geschirrspülen offenbart. Erfinderisch eingeschlossen sind auch Klarspüler, Reiniger für Geschirrspülmaschinen, Geschirrspülmittel für Industrie und Gewerbe, Kombinationsprodukte, z.B. klarspülende Geschirrspülmittel, gläser- oder silberschonende Geschirrspülmittel, Geschirrspülmittel mit Salz, u.s.w. Ein Erfindungsgegenstand ist jeweils weiterhin die Verwendung der Zusammensetzung enthaltend mindestens ein N-Acylglycamin und bevorzugt enthaltend mindestens ein Glycolipid-Biotensid, für die vorgenannten Verwendungen.

Reinigen

Ein weiterer Gegenstand der Erfindung ist ein Reinigungs- oder Pflegemittel für harte Oberflächen enthaltend mindestens ein N-Acylglycamin (bevorzugt gemäss den als bevorzugt definierten Strukturen), und bevorzugt zusätzlich enthaltend mindestens ein Biotensid, in fester oder fliessfähiger Ausführungsform oder deren Kombination, z.B. in unterschiedlichen Kompartimenten. Erfindungsgemässe Ausführungsformen sind saure, neutrale und basische Reiniger, bevorzugt zwischen pH 3,5 bis 10, sowie lösungsmittelhaltige Reiniger. Ein Erfindungsgegenstand ist jeweils weiterhin die Verwendung der Zusammensetzung enthaltend mindestens ein N-Acylglycamin und bevorzugt enthaltend mindestens ein Glycolipid-Biotensid, für die vorgenannten Verwendungen.

Deodorant

Ein weiterer Gegenstand der Erfindung ist ein Deodorant oder Antitranspirant enthaltend mindestens ein N-Acylglycamin, und bevorzugt zusätzlich enthaltend mindestens ein Biotensid, in fester oder fliessfähiger Ausführungsform oder deren Kombination, sowie dessen Verwendung.

Flüssig- Verfahren Herstellung

Ein Erfindungsgegenstand ist weiterhin das Verfahren zur Herstellung der Zusammensetzung bevorzugt als flüssiges Reinigungs- oder Pflegemittel umfassend folgende Schritte in bevorzugter Reihenfolge:

a) Bereitstellung des mindestens einen N-Acylglycamins in einem fliessfähigen Zustand,

- in flüssigen Trägermaterial, bevorzugt umfassend ein Lösungsmittel Wasser, Glycerin, Glykole, C1-C8-Alkohole oder deren Kombinationen, wobei insbesondere Wasser, Wasser und Propylenglykol oder Wasser, Propylenglykol und Glycerin bevorzugt sind, oder
- als Schmelze; bevorzugte Temperaturen von 40 °C - 80 ° C, besonders bevorzugt 40-49,9° C

b) Mischen von a) mit einem oder mehreren weiteren Inhaltsstoffen für Reinigungs- und Pflegemittel, bevorzugt mit dem mindestens einen Glycolipid-Biotensid
c) Optionale pH-Wert- Einstellung, bevorzugt zwischen 3,5 und 10
d) Konfektionierung der flüssigen Produkte

Produktformen

Neben den vorhergehend genannten fliessförmigen und festen Ausführungsformen, sind die erfindungsgemässen Zusammensetzungen weiterhin für Fest-Flüssig-Kombinationen (z.B. Kombination Pods mit einem festen Kompartiment) oder Waschblätter geeignet. Bei Waschblättern wird bevorzugt die erfindungsgemässe flüssige Zusammensetzung auf eine Oberfläche aufgebracht, getrocknet und optional auf die Dosiermenge zugeschnitten.

Eine besondere Ausführungsform stellen Kombinationen mit festen Substraten, wie Tücher dar. Tücher werden mit der erfindungsgemässen Zusammensetzung getränkt und haben den Vorteil, dass bereits die richtige Dosierung vorgegeben ist. Die Tücher oder werden aus Textilien hergestellt, welche gewebt, gestrickt, oder gewirkt sein können oder als Verbundstoff in Vlies, Papier, Watte oder Filz vorliegen, wobei Vliese meist aus Polypropylen, Polyester oder Viskose hergestellt werden. Mit Mitteln imprägnierte Substrate und Tücher können auf unterschiedliche Weisen hergestellt werden - dem Tauch-, dem Abstreif- und dem Sprühverfahren.

## Verwendungen

Ein weiterer Gegenstand der Erfindung ist die Verwendung der Zusammensetzung enthaltend mindestens ein N-Acylglycamin und bevorzugt zusätzlich enthaltend mindestens ein Biotensid als festes oder fliessfähiges Reinigungs- oder Pflegemittel für harte oder textile Oberflächen, bevorzugt ausgewählt aus den Bodenpflegemitteln, Handgeschirrspülmittel, Maschinengeschirrspülmittel, Geschirrspülmaschinenreiniger, Klarspüler, Waschmaschinenreiniger, Toilettenreiniger, Toilettenstein, WC-Reiniger, Universal-, Allzweckreiniger, Küchenreiniger, Badreiniger, Sanitärreiniger, Rohrreiniger, Fussbodenreiniger, Backofen- und Grillreiniger, Glas- und Fensterreiniger, Scheuermittel, Metallputzmittel, Polster- und Teppichreiniger, Vollwaschmittel, Colorwaschmittel, Feinwaschmittel, Wollwaschmittel, Textilhilfsmittel, Weichspüler, Holzpflegemittel, Vorbehandlungsmittel, Spezialwaschmittel und -reinigungsmittel, weitere Mitteln zur industriellen & gewerblichen und institutionellen Reinigung, Mittel für die Textil- und Faserbehandlung, Mittel für die Lederbehandlung

Ein weiterer Gegenstand der Erfindung ist die Verwendung der Zusammensetzung enthaltend mindestens ein N-Acylglycamin und bevorzugt zusätzlich enthaltend mindestens ein Biotensid als festes oder fliessfähiges, kosmetisches, dermatologische oder medizinisches Pflegemittel, insbesondere als Mund- und Zahnpflegemittel, Konditioner, Pflegeprodukte insbesondere zur Prävention von Schuppenflechte, Neurodermitis und Akne; Körper- und Gesichtslotion, Feuchttücher, insbesondere mit pflegenden, desodorierenden, antimikrobiellen oder anti-ageing Wirkstoffen, Haarverformungsmittel, Haarentfernung und Rasierprodukte, Haarfärbemittel und Haarstyling-Zubereitungen, Mundhygieneprodukt, Deodorants, Antitranspirantien, Körper- und Gesichtscreme, Sonnenschutzprodukte, Insect repellants,

Ein weiterer Gegenstand der Erfindung ist die Verwendung der Zusammensetzung enthaltend mindestens ein N-Acylglycamin und bevorzugt zusätzlich enthaltend mindestens ein Biotensid als kosmetisches, dermatologische oder medizinisches feste oder fliessfähige Reinigungsmittel, bevorzugt als Rinse-off-Reinigungsmittel, bevorzugt ausgewählt aus Duschzubereitungen, Badezusätze, Handseifen, Seifenstücke, Grobreiniger, Handpasten, Peelings, Intimreinigung, Reiniger des Augenbereichs, Kontaktlinsenreiniger, Makeup-Entferner, Augenmakeup-Entferner, Mittel zur Körperreinigung, Mund- und Zahnreinigung, insbesondere in Form von Zahnpasta, Zahncreme, Zahngel, Zahnputzflüssigkeit, Zahnputzschaum, Mundwasser, Mundwasserkonzentrat, Haarshampoos, konditionierende Shampoos, Haarfärbeshampoos, feste Shampoos, Antischuppen-Shampoo, Babyshampoo, Reinigungsprodukte insbesondere zur Prävention von Schuppenflechte, Neurodermitis und Akne; Körper-, und Gesichtstonic, Gesichtslotion, Feuchttücher,

## Verfahren zur Reinigung

In einem weiteren Erfindungsgegenstand richtet sich die Erfindung auf ein Reinigungs- oder Pflegeverfahren umfassend

a) die Bereitstellung einer Reinigungs- oder Pflegemittel umfassend ein Mittel gemäss einem der obigen Erfindungsgegenstände
b) gegenbenenfalls lösen oder dispergieren des Mittels aus (a) in einem geeigneten Medium, bevorzugt Wasser
c) in Kontakt bringen einer Oberfläche oder Textilien, Teppichen oder Naturfasern, Haut, Haaren oder Zähnen mit der Lösung gemäss (b).
d) optional spülen oder abwischen der Oberfläche oder Textilien, Teppichen oder Naturfasern, Haut, Haaren oder Zähnen

## Ausführungsbeispiele

Verwendete Tenside, soweit nicht anders genannt:
N-Acylglycamin: Sunfloweroyl Methylglucamide, Biotenside: Rhamnolipid Rheance One, Sophorolipide: Sophoclean und Rewoferm, MEL: Toyobo MEL 201 und Toyobo MEL 211.

Beispiel 1: Schaumbildung verhält sich synergistisch

Exemplarisch werden vergleichende Untersuchungen zur Schaumbildung in einer 2,1%-igen Zusammensetzung bei Raumtemperatur abgebildet (Tabelle 1). Dabei werden jeweils sowohl die reinen Substanzlösungen als auch die 1:1-Kombinationen von N-Acylglycamin mit Biotensid gemessen. Der pH-Wert beträgt pH = 4,8 ± 0,5, die Schaumbildung wird bestimmt in Wasser ohne Ionenfracht (destilliertes Wasser) und in Wasser mit 0,5% $CaCl_2$ (45 mmol/l) Weiterhin wird die Schaumbildung bei einem pH-Wert von pH = 7,5 ± 0,3 dargestellt. Es werden, unabhängig vom gewählten pH-Wert oder der Ionenfracht, positive Synergien (syn +) in Bezug auf die Schaumbildung der erfinderischen Kombinationen gefunden (Tab 1).

Tabelle 1: Positive Synergie bei Kombination der Tenside

| Schaumbildung | Rhamnolipid + N-Acylglycamin | Sophorolipid + N-Acylglycamin |
|---|---|---|
| pH = 4,3- 5,3 | Syn + | Syn + |
| Zugabe 0,5 % $CaCl_2$ | Syn + | Syn + |
| pH = 7,5 ± 0,3 | Syn + | Syn + |

Überraschenderweise wurde weiterhin festgestellt, dass die Synergie in Anwesenheit von Ionen, wie hier $CaCl_2$, besonders groß ist. Während die Schaumbildung bei den Biotensiden alleine durch die Anwesenheit von Ionen stark beeinflusst wird, wirken die N-Acylglycamine in der erfinderischen Kombination mit Biotensiden synergistisch: Eine stärkere Schaumbildung als erwartet kann erzielt werden. Besonders ausgeprägt ist die Synergie bei der Schaumbildung der nur mässig schäumenden Sophorolipide durch die Zugabe von N-Acylglycaminen.

Beispiel 2: Schaumstabilität

Exemplarisch werden vergleichende Untersuchungen zur Schaumstabilität der Zusammensetzungen aus Beispiel 1 bei Raumtemperatur nach 60 min abgebildet (Tabelle 2). Es werden, unabhängig vom gewählten pH-Wert und der Ionenfracht positive Synergien (syn +) in Bezug auf die Schaumstabilität der erfinderischen Kombinationen gefunden (Tab 2).

Tabelle 2: Positive Synergie bei Kombination der N-Acylglycamine mit Biotensiden

| Schaumstabilität | Rhamnolipid + N-Acylglycamin | Sophoroplipid + N-Acylglycamin |
|---|---|---|
| pH = 4,3- 5,3 | Syn + | Syn + |
| Zugabe 0,5 % $CaCl_2$ | Syn + | Syn + |
| pH = 7,5 ± 0,3 | Syn + | Syn + |

Überraschenderweise wurde festgestellt, dass auch die Schaumstabilität, insbesondere in Anwesenheit von Ionen, wie hier beispielsweise $CaCl_2$, synergistisch ist. Während der Schaum der Biotenside in Anwesenheit von Ionen schnell zusammenbricht, bleibt in der erfinderischen Kombination der Schaum über die Zeit stabil. Insbesondere ermöglicht die Kombination der Tenside die Stabilisierung von Schaum in hartem Wasser oder bei elektrolythaltigen Formulierungen.

MEL: Die Schaumstabilität in den MEL-Emulsionen mit Glucamid (2,1 % in dest. Wasser, pH 7,2 ± 0,3) zeigen nach 20 min eine geringere Abbaurate des Schaums als bei der Kombination der Einzelkomponenten zu erwarten gewesen wäre. Die Schaumstabilität verhält sich synergistisch.

Sensorik: Geschulte Testpersonen wenden jeweils 20 ml der Kombinationen von N-Acylglycaminen mit Biotensiden aus Beispiel 2 auf der Haut an. Die Nass- und Trockensensorik wird durchgehend als angenehm empfunden.

Beispiel 3: Benetzung

Bestimmung der Benetzungsgeschwindigkeit von Mischgewebe. Es wird vergleichend die Geschwindigkeit bestimmt, mit der 1 ml der Testlösungen aus Beispiel 1 in destilliertem Wasser (pH = 4,8 ± 0,5 bzw. bei pH = 7,2 ± 0,3 im Fall der MEL-Emulsionen) von dem Mischgewebe (aus 60%Polyester/40%Baumwolle) bei Raumtemperatur aufgenommen wird (Tabelle 3);

Tabelle 3: Benetzung.

| Benetzung | Rhamnolipid + N-Acylglycamin | Sophoroplipid + N-Acylglycamin | MEL + N-Acylglycamin |
|---|---|---|---|
| | Syn + | Syn + | Syn + |

Biotenside und N-Acylglycamid zeigen eine positive Synergie betreffend der Benetzungsgeschwindigkeit.

Beispiel 4: Schaum - Konzentrationsreihe Biotensid / N-Acylglycamin

Bestimmung des Schaumbildung in dest. Wasser bei pH = 6,8 ± 0,2 in einer 2,1 %-igen Tensidlösung bei Raumtemperatur (Tabelle 4) am Beispiel Sophorolipid.

Bestimmung der Schaumbildung und der Schaumstabilität durch vergleichende Bestimmung der Schaumhöhe sofort nach Agitation und nach 60 min; Verhältnis der Komponenten in Gew.-%. Aktivsubstanz.

Tab 4: Schaumbildung und -stabilität

| Biotensid : N-Acylglycamin | 0 : 1 | 1 : 2 | 1 : 1 | 2: 1 | 3 : 1 | 5: 1 | 10 : 1 |
|---|---|---|---|---|---|---|---|
| Schaumbildung | Ref | + | ++ | +++ | +++ | +++ | +++ |
| Schaumhöhe 120 min | Ref | + | ++ | +++ | +++ | +++ | +++ |
| (+) = erhöhte Schaumbildung bzw. Schaumstabilität gegenüber N-Acylglycamin als Referenz<br>(++) = erhöhte Schaumbildung bzw. Schaumstabilität gegenüber (+)<br>(+++) = erhöhte Schaumbildung bzw. Schaumstabilität gegenüber (++) | | | | | | | |

Beispiel 5: Rückstandsbildung - Konzentrationsreihe Biotensid / N-Acylglycamin

Rückstand einer 11%-ige Zusammensetzung in einem Gewichtsverhältnis der zwei Komponenten wie angegeben, in dest. Wasser, pH = 6,4 ± 0,1. Rückstandbildung wird visuell bestimmt nach Auftragen der Testlösungen auf eine Spiegeloberfläche (Tab 5) ohne Abspülen.

Tabelle 5: Rückstandsbildung in Abhängigkeit der Mengenverhältnisse der Komponenten

| Biotensid : N-Acylglycamin | 0 : 1 | 1 : 2 | 1 : 1 | 2: 1 | 3 : 1 | 5: 1 | 10 : 1 |
|---|---|---|---|---|---|---|---|
| Rückstand | Ref | + | + | ++ | ++ | ++ | ++ |
| (+) bedeutet eine Verringerung des Rückstands gegenüber der Referenz<br>(++) bedeutet eine Verringerung des Rückstands gegenüber (+)<br>(+++)bedeutet eine Verringerung des Rückstands gegenüber (++) | | | | | | | |

Die erfinderischen Zusammensetzungen zeigen alle eine noch geringe Rückstandsbildung als die Reinsubstanzen.

Der Rückstand der MEL-Emulsionen aus Beispiel 2 wird auf einer Spiegeloberfläche bestimmt. Die Rückstandsbildung der erfinderischen Kombinationen mit N-Acylglycamin ist geringer als bei den reinen MEL-Emulsionen.

**Reinigungs- oder Pflegemittel**

Die folgenden Versuche werden in gleicher Weise für Referenz und erfinderische Zusammensetzung durchgeführt. Die Testlösungen liegen in entmineralisiertem Wasser vor, mit einem pH von 6 (± 0,5) eingestellt, soweit nicht anders angegeben. Die Leistungsbewertung erfolgt jeweils relativ zur Referenz. Konzentrationen gemäss Angaben. Vergleichende Bestimmung des Schäumvermögens (entmineralisiertes Wasser) durch Messung der Schaumhöhe nach Agitation. (+) bedeutet eine höhere Schaumbildung relativ zur Referenz. Bestimmung der Schaumstabilität: Vergleichende Messung der Schaumhöhe nach definierten Zeitintervallen ohne weitere Agitation, Bestimmung der Schaumstabilität in absoluter Schaumhöhe oder relativ (rel.) durch Bestimmung der Differenz der Schaumhöhe zur anfänglichen Schaumhöhe nach einem Zeitintervall und Bezug auf die anfängliche Schaumhöhe (entspricht Schaumabbau). Vergleich dieser Werte mit den Referenzen. (+) bedeutet eine höhere Schaumhöhe bzw.-stabilität relativ zur Referenz.

Spülvermögen nach IKW: jeweils vergleichende Beurteilung angelehnt an den "Empfehlungen zur Qualitätsbewertung der Reinigungsleistung von Handgeschirrspülmitteln", Nitsch, Ch., Hüttmann, G. SÖFW Journal, 128, 5; 2002. Endpunktbestimmung = Punkt, an dem die durchgehende Schaumdecke im Spülvorgang in Anwesenheit von Testschmutz durchbrochen wird. (Testschmutz-Zusammensetzung in der Trockenmasse: 26% Fett, davon 15% gesättigte Fettsäuren, Kohlenhydrate 37%, davon 4% Zucker, 6% Eiweiss, 2% Ballaststoffe, 9% Salz); Spülwasser 20°(± 2) dH, 35-45°C. (+) bedeutet höheres Spülvermögen nach IKW relativ zur Referenz. Fettlösevermögen (11% oder 1.1%-ige Lösungen in entmineralisiertem Wasser: Fettschmutz nach den "Empfehlungen zur Qualitätsbewertung der Produktleistung von Allzweckreinigern", A. Fitzner, U. Aßmus, SÖFW Journal, 130, 10, 2004, vergleichende optische Beurteilung des Emulgierverhaltens der Testlösungen auf Fett ohne mechanische Einwirkung. (+) bedeutet ein besseres Fettlösevermögen relativ zur Referenz. Rückstand: Aufstreichen der Testlösung (27 mg der 11%-igen Lösung) auf Spiegelfläche (30 $cm^2$) und vergleichende relative optische Beurteilung des Rückstands nach Abtrocknen; (+) bedeutet weniger verbleibender Rückstand verglichen zur Referenz. Benetzung: Bestimmung der relativen Geschwindigkeit in der das Textil benetzt wird im Vergleich zur Referenz (100% Baumwolle, weiss, und Mischgewebe aus 60%Polyester/40%Baumwolle, weiss). Angaben der Konzentrationen der Einzelkomponenten in Gew.-% (Aktivgehalt). Soweit nicht anders angegeben: Natrium Laureth sulfate, hier exemplarisch 2 EO (SLES), Cocosulfate, Sodium dodecylsulfate (LAS), Cocoamidopropylbetain (CAPB), Biotensid = Sophorolipid, Rhamnolipid, , N-Acylglycamin = Sunfloweroyl Methylglucamid.

Beispiel 6: Erfindung im Vergleich zu marktüblichen schaumbildenden Formulierungen mit nur einer Komponenten

Die erfinderische Zusammensetzung wird in einer schaumbildenden Formulierung eingesetzt, wie diese typischerweise für die Reinigung von Oberflächen, z.B. Handgeschirrspülmittel oder Personal Care Formulierungen, z.B. Shampoos oder Duschgels verwendet werden. Bevorzugte pH-Werte dieser Formulierungen liegen zwischen 5 und 8.

Beispiel 6

| | Ref 1 | 1 | 2 | Ref 2 |
|---|---|---|---|---|
| SLES | 6 | 6 | 6 | 6 |
| Cocosulfate | 1 | 1 | 1 | 1 |
| CAPB | 1 | 1 | 1 | 1 |
| Sophorolipid | 0 | 1 | 2 | 1 |
| Sunfloweroyl Methylglucamid | 1 | 1 | 1 | 0 |
| PEG-4 Rapeseedamide | 2 | 1 | 0 | 2 |
| Aqua (entmin.) | ad 100 | ad 100 | ad 100 | ad 100 |
| Gesamttensidkonz (%) | 11 | 11 | 11 | 11 |
| Schäumvermögen | Ref | + | + | |
| (Tensidkonz. 1.1%) | | + | + | Ref |
| Schaumhöhe (40 min) | Ref | + | + | |
| (Tensidkonz. 1.1%) | | + | + | Ref |
| Spülleistung (IKW) | Ref | + | + | |
| (Tensidkonz. 0,008%) | | + | + | Ref |
| Emulgieren | Ref | + | + | |
| (Tensidkonz. 11% und 1,1 %) | | + | + | Ref |
| Rückstandsbildung (Tensidkonz. 11%) | Ref | + | + | |
| Sensorik | | + | + | |

Tabelle 6

Die Kombinationen von dem N-Acylglycamin mit Biotensid zeigt eine bessere Leistung in Bezug auf Schaumverhalten, Spülleistung, Fettlösen und Rückstand im Vergleich zu den Referenzen, die jeweils nur eine der Komponenten enthalten (Tabelle 6). Durch die erfindungsgemässe Kombination von Tensiden können zudem PEG-haltige Tenside (hier C-18) verringert oder ersetzt werden. Die Sensorik der erfindungsgemässen Zusammensetzungen wird durch das Versuchspanel als angenehm beurteilt (+).

Beispiel 7

Formulierung 1 analog 1b mit Rhamnolipiden anstelle von Sophorolipiden als Biotensiden:
Wie auch mit Sophorolipid als Biotensid zeigen die Kombinationen mit Rhamnolipiden eine stärkere Schaumstabilität (40 min) als die Referenzen ohne Biotensid (Ref 1). Betreffend Reinigungsleistung zeigen die erfinderischen Formulierungen ein besseres Fettlösevermögen als Ref 1.

Beispiel 8)

Tabelle 7

| | Ref 3 | 3 | 4 |
|---|---|---|---|
| SLES | 6 | 6 | 6 |
| Cocosulfate | 1 | 1 | 1 |
| CAPB | 1 | 0 | 1 |

(fortgesetzt)

| | Ref 3 | 3 | 4 |
|---|---|---|---|
| Cocoglucoside | 1 | 1 | 0 |
| Sophorolipid | 0 | 1 | 1 |
| Sunfloweroyl Methylglucamid | 1 | 1 | 1 |
| PEG-4 Rapeseedamide | 1 | 1 | 1 |
| Aqua (entmin.) | ad 100 | ad 100 | ad 100 |
| Gesamttensidkonz (%) | 11 | 11 | 11 |
| Schäumvermögen (Tensidkonz. 1.1 %) | Ref | = | + |
| Schaumstabilität (rel., 120 min) (Tensidkonz. 1.1 %) | Ref | + | + |
| Spülleistung (IKW) (Tensidkonz. 0,008%) | Ref | + | + |
| Emulgieren (Tensidkonz. 11% und 1,1 %) | Ref | + | + |
| Rückstandsbildung (Tensidkonz. 11%) | Ref | + | + |
| Sensorik | | + | + |

Die erfindungsgemässen Kombinationen zeigen eine mindestens gleich gute Leistung in Bezug auf Schaumverhalten, Spülleistung, Fettlösen und Rückstand im Vergleich zu der Referenz ohne Biotensid (Tabelle 7). Durch die erfindungsgemässe Kombination kann CAPB oder APG (hier Cocoglucosid) bei mindestens gleicher Leistung ersetzt werden. Die CAPB-freie Ausführungsvariante zeigt weiterhin Vorteile betreffend einer verbesserten Benetzungsfähigkeit.

Beispiel 9

Tabelle 8:

| | Ref 4 | 4 | 5 | 6 |
|---|---|---|---|---|
| Cocosulfate | 7 | 7 | 7 | 7 |
| CAPB | 1 | 1 | 1 | 1 |
| Cocoglucoside | 2 | 0 | 0 | 0 |
| Rhamnolipid | 0 | 2 | 2 | 2 |
| Sunfloweroyl Methylglucamid | 1 | 1 | 0 | 0 |
| Cocoyl Methyl Glucamide | 0 | 0 | 1 | 0 |
| Capryloyl / Caproyl Methyl Glucamide | 0 | 0 | 0 | 1 |
| Aqua (entmin.) | ad 100 | ad 100 | ad 100 | ad 100 |
| Gesamttensidkonz (%) | 11 | 11 | 11 | 11 |
| | | | | |
| Schäumvermögen (Tensidkonz. 1.1 %) | Ref | + | + | = |
| Schaumstabilität (rel., 40 min) (Tensidkonz. 1.1 %) | Ref | + | + | + |
| Spülleistung (IKW) | Ref | + | + | + |
| (Tensidkonz. 0,008%) | | | | |
| | | + | + | + |
| Rückstandsbildung (Tensidkonz. 11%) | Ref | + | + | + |
| Sensorik | | + | + | + |

Aufgrund der Nachhaltigkeitsbetrachtungen und der Produktleistungen wird bevorzugt die N-Acylglycamid-Kombination mit Sunfloweroyl Methylglucamid eingesetzt. Diese Beispiele zeigen die Leistungsfähigkeit der erfindungsgemässen Mittel in Abwesenheit von PEG-Tensiden, des Weiteren demonstrieren diese Beispiele, dass zudem auf Cocoglucosid verzichtet werden kann (Tabelle 8).

Beispiel 10

Formulierungen analog 1c mit Sophorolipiden anstelle von Rhamnolipiden als Biotensiden:
Wie auch mit Rhamnolipid als Biotensid zeigen die erfinderischen Formulierungen mit Sophorolipid ein stärkeres Schäumvermögen als die Referenzen ohne Biotensid oder ohne Glucamid. Betreffend Reinigungsleistung zeigen die erfinderischen Formulierungen ein besseres Fettlösevermögen als die Referenzen. Die Reinigungsleistung nach IKW "Handgeschirrspülmittel" verglichen mit der IKW-Standardformulierung zeigt, dass die erfinderischen Formulierungen mindestens gleich gut oder besser als der IKW-Standard abschneiden. Auch hinterlassen die erfinderischen Kombinationen einen geringeren Rückstand als die Referenzen. Aufgrund der Nachhaltigkeitsbetrachtungen und der Produktleistungen wird bevorzugt die Kombination Sunfloweroyl Methylglucamid mit Sophorolipiden eingesetzt.

Beispiel 11:Substitution von PEG-Tensiden

Ersetzt wird das Standardtensid Sodium laureth sulfate durch die erfinderischen Tensidkombination. Schaumbildung und Schaumstabilität in destilliertem Wasser bei einer Tensidkonzentration von 0,5% ist in den erfinderischen Mitteln 7-10 mindestens gleich gut oder besser als in der Referenz. In Anwesenheit von Calcium- und Magnesiumionen im Wasser (26 dH) bei 0,5% Tensidkonzentration bilden die erfinderischen Mittel eine grössere Schaummenge als die Referenz, der Schaum bleibt zudem über die Zeit stabil (Tabelle 9). Die erfindungsgemässen Mitteln, hier exemplarisch gezeigt an den Mitteln 7-10 in unterschiedlichen Mengenverhältnissen zeigen eine schnellere Benetzung, sowohl auf Baumwolle, als auch auf Mischgewebe (Polyester/Baumwolle) als die Referenz (Tab 9). Die erfinderischen Mittel 7-10 lassen sich alle rückstandsarm anwenden und erweisen sich alle als gute Fettlöser. PEG-Tenside, hier am Beispiel Sodium laureth sulfate, können teilweise oder komplett durch die erfinderische Tensidkombination ersetzt werden.

Tabelle 9

| | Ref 6 | 7 | 8 | 9 | 10 |
|---|---|---|---|---|---|
| LAS | 5,4 | 5,4 | 5,4 | 5,4 | 5,4 |
| SLES | 5,4 | 4,5 | 3 | 1,5 | 0 |
| Rhamnolipid | 2,7 | 2,7 | 4,2 | 5,7 | 7,2 |
| Sunfloweroyl Methylglucamid | 0 | 0,9 | 0,9 | 0,9 | 0,9 |
| Gesamttensidkonz . | 13,5 | 13,5 | 13,5 | 13,5 | 13,5 |
| Anteil anion. PEG-Tenside | 40% | 33% | 22% | 11% | 0% |
| Schaumbildung (Tensidkonz. 0,5% in $H_2O$, 26 dH) | Ref | + | ++ | ++ | ++ |
| Schaumstabilität (0,5 % Tensidkonz. in $H_2O$, 26 dH, 2h) | Ref | + | ++ | +++ | +++ |
| Benetzung Mischgewebe (Tensidkonz. 2,6% in $H_2O$, 26 dH) | Ref | + | ++ | ++ | ++ |
| Benetzung Baumwolle (Tensidkonz. 2,6% in $H_2O$, 26 dH) | Ref | ++ | ++ | + | + |
| Rückstandsbildung (Tensidkonz. 2,6%, entmin. Wasser) | | Kein Rückstand erkennbar | | | |

Beispiel 12: Gleichzeitiger Ersatz von PEG-Tensiden und CAPB

Ersetzt wird das Standardtensid Sodium laureth sulfate durch die erfinderischen Tensidkombination. Schaumbildung und Schaumstabilität in destilliertem Wasser bei einer Tensidkonzentration von 0,5% ist in den erfinderischen Mitteln 11-12 mindestens gleich gut oder besser als in der Referenz. In Anwesenheit von Calcium- und Magnesiumionen im Wasser (26 dH) bei 0,5% Tensidkonzentration bilden die erfinderischen Mittel eine grössere Schaummenge als die Referenz, der Schaum bleibt zudem über die Zeit stabil (Tabelle 10). Es können hierbei nicht nur die PEG-Tenside ersetzt werden, es sind auch Ausführungsformen ohne Standardtenside, hier exemplarisch Sodium Laureth Sulfate als PEG-Tensid und ohne CAPB als Schaumbooster möglich, ohne Leistungseinbussen (Tabelle 10). Die Kombination

eines anionischen, PEG-freien Tensids, hier exemplarisch Sodium Dodecylsulfat, mit der erfinderischen Tensidkombination bietet leistungsstarke, breit einzusetzende Alternativen zu üblichen Tensidsystemen und verleiht gleichzeitig eine angenehme Sensorik im Falle von Hautkontakt ohne das Gefühl der Austrocknung.

Neben einem guten Schaumverhalten zeigen die erfinderischen Mittel 11 und 12 gutes Fettlösevermögen und eine rückstandsarme Anwendung.

Tabelle 10

| | Ref 6 | 11 | 12 |
|---|---|---|---|
| LAS | 5,4 | 5,4 | 5,4 |
| SLES | 5,4 | 0 | 0 |
| Rhamno | 2,7 | 6,3 | 6,3 |
| Sun | 0 | 1,8 | 0,9 |
| CAPB | | | 0,9 |
| | 13,5 | 13,5 | 13,5 |
| Schaumbildung (Tensidkonz. 0,5% in $H_2O$, 26 dH) | Ref | + | + |
| Schaumstabilität (Tensidkonz. in H2O, 26 dH, 2h) | Ref | + | + |
| Rückstand | | Kein Rückstand erkennbar | Kein Rückstand erkennbar |
| Sensorik | | + | + |

Beispiel 13: Anwesenheit von organischem Lösungsmittel

Häufig werden Reinigungs- oder Pflegemittel in Kombination mit organischen Lösungsmitteln verwendet. Es wird daher ein organisches Lösungsmittel (1%-ig), hier gezeigt am Beispiel Propylenglykol, zu der 0,5%-ige Tensidlösung aus den erfinderischen Zusammensetzungen 10-12 in hartem Wasser zugegeben.

Propylenglykol mindert das Schaumvermögen und die Schaumstabilität. Verglichen mit der Referenz, erhält man in den erfinderischen Tensidkombinationen auch in Anwesenheit von Propylenglykol eine mindestens gleich gute Schaumleistung betreffend Schaumvermögen und Schaumstabilität, die relativen Verhältnisse werden gewahrt. Unabhängig von der Anwesenheit des organischen Lösungsmittels zeigen die erfinderischen Mittel ein gutes Leistungsspektrum, in Tabelle 11 exemplarisch gezeigt für die Schaumbildung und -stabilität. Beispiele hierzu sind der Tabelle 11 zu entnehmen.

Tabelle 11

| | Ref 6 | 10 | 11 | 12 |
|---|---|---|---|---|
| Tensidkonz. In $H_2O$ (26 dH) | 0,50% | 0,50% | 0,50% | 0,50% |
| Propylenglykol | 1% | 1% | 1% | 1% |
| Schaumbildung (Tensidkonz. 0,5% in $H_2O$, 26 dH) | Ref | + | + | + |
| Schaumstabilität (Tensidkonz. in $H_2O$, 26 dH) | Ref | + | + | + |
| Benetzung: Die 0,5%-igen Tensidlösungen mit 1% Propylenglykol zeigen sowohl in ionenfreiem Wasser, als auch in Wasser des Härtegrades 26 dH eine gute Benetzung auf Baumwolle und Mischgewebe. | | | | |

## Weitere Ausführungsbeispiele

Die Mengenangaben sind jeweils in Gew.-% Aktivsubstanz

Tabelle 12: Weichspüler

Tabelle 13: Conditioner
Tabelle 14: Conditioner
Tabelle 15: Körperreinigung
Tabelle 16: Körperpflege
Tabelle 17: Zahnpflege
Tabelle 18: Geschirrspülmittel
Tabelle 19: Waschmittel
Tabelle 20: Waschmittel
Tabelle 21: Reiniger

Tabelle 12

**Softener**

| 14 | |
|---|---|
| ad 100 | Aqua |
| 5 | Dioleoylethyl Hydroxyethylmonium Methosulfate (and) |
| | Sunflower seed oil glycerides |
| 1,5 | Isopropylalkohol |
| 0,8 | Sunflower methylglucamid |
| 0,5 | Propylene Glycol |
| | |
| | Lactic acid ad pH = 3 |
| | optional: Parfum, Farbstoff, Konservierung, Salze |

**Softener**

| 15 | |
|---|---|
| ad 100 | Aqua |
| 5 | Disunfloweroylethyl Hydroxyethylmonium Methosulfate |
| | |
| 1,5 | Isopropylalkohol |
| 1,5 | N-Acyl-N-methyl cyclic glucamide |
| 0,5 | Propylene Glycol |
| | |
| | Lactic acid ad pH = 3 |
| | optional: Parfum, Farbstoff, Konservierung, Salze |

**Softener**

| 16 | |
|---|---|
| ad 100 | Aqua |
| 5 | Dioleoylethyl Hydroxyethylmonium Methosulfate (and) |
| | Sunflower seed oil glycerides |
| 1,5 | Isopropylalkohol |
| 0,8 | Sunflower methylglucamid |
| 0,5 | Sophorolipid |
| 0,1 | Propylene Glycol |
| | Parfum |
| | optional: Farbstoff, Konservierung |
| | Lactic acid ad pH = 3,5 |

**Softener Fest**

| 17 | |
|---|---|
| 6 | Dioleoylethyl Hydroxyethylmonium Methosulfate |
| | Sunflower seed oil glycerides |
| 7,5 | Propylenglykol |
| 4,5 | Sunflower methylglucamid |
| 56 | Saccharose |
| 24 | Maisstärke |
| 1 | Silikon dioxide |
| 1 | Parfüm |
| opt. | Farbstoff |

**Softener fest**

| 18 | |
|---|---|
| 5,5 | Dioleoylethyl Hydroxyethylmonium Methosulfate |
| | Sunflower seed oil glycerides |
| 7,5 | Propylenglykol |
| 0,7 | Sunflower methylglucamid |
| 60,1 | Saccharose |
| 24 | Maisstärke |
| 1 | Silikon dioxide |
| 1,21 | Mannosylerytritollipid |
| opt. | Farbstoff |
| opt. | Parfüm |

**Softener fest**

| 19 | |
|---|---|
| 20 | Methyl bis(canola amidoethyl)-2-hydroxyethyl ammonium methyl sulfate |
| 20 | Maltodextrin |
| 5 | Citric acid |
| 8 | Sunflower methylglucamid |
| 47 | Maisstärke |
| opt. | Farbstoff |
| opt. | Parfüm |
| | Glucamid-Saccharid-Agglomerat<br>Schmelze von Glucamid auf 60C<br>Mischen mit Maisstärke<br>Zugabe Inhaltsstoffe<br>Abkühlen in Form |

**Softener fest**

| 20 | |
|---|---|
| 60 | Methyl bis(canola amidoethyl)-2-hydroxyethyl ammonium methyl sulfate |
| 15 | Citric acid |
| 16 | Sodium acetate |
| 2 | Sunflower methylglucamid |
| 1 | Canola fatty acids |
| 6 | Polysiloxan |
| opt. | Farbstoff |
| opt. | Parfüm |

**Softener fest**

| 21 | |
|---|---|
| 60 | Disunfloweroylethyl Hydroxyethylmonium Methosulfate |
| 12,5 | Guar hydroxypropyltrimonium chlorid |
| 15 | Sunflower methylglucamid |
| 12,5 | Hydroxypropylguar |

**Softener Fest**

| | 22 |
|---|---|
| 6 | Dioleoylethyl Hydroxyethylmonium Methosulfate |
| | Sunflower seed oil glycerides |
| 7,5 | Propylenglykol |
| 60,5 | Sunflower methylglucamid-Saccharose-Agglomerat |
| 24 | Maisstärke |
| 1 | Silikon dioxide |
| 1 | Parfüm |
| optional | Farbstoff |
| Gucamid- Saccharose-Agglomerat:<br>100 g Agglomerat erhalten durch 7,5 g Glucamid und 92,5 g Saccharose.<br>Glucamid -Schmelze bei 60 C | |

Tabelle 13

| | Conditioner | | Conditioner | | Conditioner |
|---|---|---|---|---|---|
| | 23 | | 24 | | 25 |
| 0,8-5 | Dioleoyl hydroxyethylmonium Methosulfate & Sunflower seed oil glycerides | 2 | Dicanola hydroxyethylmonium Methosulfate | 6 | Dicanolaoylethyl hydroxyethylmonium methosulfate |
| 0,5-1 | Sunfloweroyl methylglucamid | 1 | Sunfloweroyl methylglucamid | 35 | Sweet almond oil |
| 5-10 | Cetearyl alkohol | 5 | Oleoyl alkohol | 27 | Cetearyl alkohol |
| 1-2 | Helianthus annus sunflower seed oil | 1,2 | Helianthus annus sunflower seed oil | 2 | Sunfloweroyl methylglucamid |
| 0,2 | Tocopherol | 2 | Mannosylerythritollipide | 30 | Propylenglykol |
| 0-5 | Propylenglykol | 1 | Propylenglykol | | |
| 0,4 | Potassium sorbate & Sodium Benzoate | 0,4 | Potassium sorbate & Sodium Benzoate | | optional: Farbstoff, Konservierungsmittel, Duftstoffe, stabilisator, PH-Stellmittel |
| 0,2 | Fragrance | 0,2 | Fragrance | | |
| opt. | Milchsäure/ Natriumlaktat ad pH= 4 -5 | q.s. | Milchsäure/ Natriumlaktat ad pH= 4 -5 | | |
| ad 100 | Aqua | ad 100 | Aqua | | |
| Conditioner | | Conditioner | | Conditioner | |
| | 26 | | 27 | | 28 |
| 6 | Arachidyl/Behenyl Betainate Esylate (an d) Arachidyl/Behenyl Alkohol | 2,2 | Behenamidopropyl Dimethylamine | 4 | Helianthus Annus Seed oil |
| 2 | Glycerin | 0,5 | Sunfloweroyl methylglucamid | 4 | Isopropylstearate |
| 0,5 | Guar-Verdicker | 4,4 | Cetearyl alkohol | 7,4 | Glyceryl caprylate / Caprate |
| 1,5 | Butyruspermum parkii butter | 0,5 | Caesalpinia Spinosa gum | 23 | Stearyl alkohol |
| 1,5 | Theobroma Cacoa seed butter | 0,2 | Panthenol | 2 | Sunflower seed wax |
| 1 | Propandiol dicaprylate | 1 | Keratin | 3,5 | Butyrospermum Parkii Butter |
| 1,5 | Sunfloweroyl methylglucamid | 0,6 | Lactic acid | 14 | Dioleoylethyl Hydroxyethylmonium Methosulfate/ Sunflower seed glycerides |
| 2 | Apricot kernel oil | opt. | Fragrance, Konservierung | 7 | Sucrose Stearate |
| 0,4 | Potassium sorbate & Sodium Benzoate | 7 | Rhamnolipide | 2 | N-C8/C10-Acyl-N-methyl cyclic glucamide |
| 0,2 | Fragrance | ad 100 | Aqua | 2 | Lactic acid/ Sodium Lactate |

(fortgesetzt)

| Conditioner | | Conditioner | | Conditioner | |
|---|---|---|---|---|---|
| | 26 | | 27 | | 28 |
| 2 | Lactate/ Lactic Buffer (pH =4-5) | | | 0,1 | Fragrance |
| 3 | Biotenside (optional) | | | 31 | Oryza Sativa Starch |
| ad 100 | Aqua | | | | |

Tabelle 14

**Conditioner 29**

| | | |
|---|---|---|
| A | 9 | Argania spinosa kernel oil |
| | 5 | Corylus avellana seed oil |
| | | |
| B | 11 | Quat, z.B. Arachidyl/Behenyl Betainate Esylate |
| | 33 | Brassica alcohol |
| | 5 | Sunflower methylglucamid |
| | 18 | Butyrospremum parkii butter |
| | 5 | Theobroma cacao seed butter |
| | 8 | Mannosylerythritollipid |
| | 4 | Zea Mays Starch |
| | 2 | Sodium Lactate/ Lactic acid |
| | | |

**Conditioner 30**

| | | |
|---|---|---|
| A | 9 | Argania spinosa kernel oil |
| | 5 | Corylus avellana seed oil |
| | | |
| B | 13 | Quat, z.B. Arachidyl/Behenyl Betainate Esylate |
| | 10 | Sunflower methylglucamid |
| | 12 | Butyrospremum parkii butter |
| | 5 | Theobroma cacao seed butter |
| | 40 | Brassica alcohol |
| | 4 | Zea Mays Starch |
| | 2 | Sodium Lactate/ Lactic acid |

Mix comp A to 80 C

Melt comp A and heat to 80 C

Mix A and B under stirring

Add comp C one after another to the formulation

Let the product cool down e.g. in a silicon mold

**Conditioner 31**

| | | |
|---|---|---|
| A | 40 | Brassica alcohol |
| | 1,8 | Glyceryl mono stearate |
| | 2,5 | Sunflower seed oil Sorbitol esters |
| B | 11 | Behenamidopropyl Dimethylamine |
| | 9 | Sunflower methylglucamid |
| | 3,2 | Butyrospremum parkii butter |
| | 3,2 | Theobroma cacao seed butter |
| | 2 | Jojoba oil |
| | | Antioxidant |
| | 25 | Sophorolipid- Zea Mays Starch-Agglomerate |
| | 2 | Sodium Lactate/ Lactic acid |
| | 0,3 | Fragrance |

Sophorolipid- Zea Mays Starch-Agglomerate

8 g Sophorolipid auf 92 g Zea Mais Starch

bei Raumtemperatur vermengen

Trocknen

**Conditioner 33**

| | | |
|---|---|---|
| A | 2,5 | Sunflower seed oil Sorbitol esters |
| | 1,8 | Glyceryl mono stearate |
| | 0,3 | Fragrance |
| | 2 | Olive oil |
| | | |
| B | 11 | Quat z.B. Methyl bis(canola amidoethyl)-2-hydroxyethyl ammonium methyl sulfate |
| | 3,2 | Theobroma cacao seed butter |
| | 40 | Brassica alcohol |
| | 3,2 | Butyrospremum parkii butter |
| | 10 | Sunflower methylglucamid |
| | 5 | Coco methyl isethionate |
| | 19 | Zea Mays Starch |
| | 2 | Sodium Lactate/ Lactic acid |

Mix comp A to 80 C

Melt comp A and heat to 80 C

Mix A and B under stirring

Add comp C one after another to the formulation

Let the product cool down e.g. in a silicon mold

**Conditioner 34**

| | | |
|---|---|---|
| A | 4 | Helianthus Annus Seed oil |
| | 4 | Isopropylstearate |
| | 7,4 | Glyceryl caprylate / Caprate |
| | 23 | Stearyl alkohol |
| | 2 | Sunflower seed wax |
| | 3,5 | Butyrospermum Parkii Butter |
| B | | |
| | 14 | Dioleoylethyl Hydroxyethylmonium Methosulfate/ Sunflower seed glycerides |
| | 7 | Sucrose Stearate |
| | 2 | N-Acyl-N-methyl cyclic glucamide |
| | | |
| | 2 | Lactic acid/ Sodium Lactate |
| | 0,1 | Fragrance |
| | 31 | Rhamnolipid-Oryza Sativa Starch-Agglomerate |

Rhamnolipid-Oryza Sativa Starch-Agglomerate

15 g Sophorolipid auf 85 g Zea Mais Starch

bei Raumtemperatur vermengen

Trocknen

**Conditioner 35**

| | | |
|---|---|---|
| A | 4 | Helianthus Annus Seed oil |
| | 4 | Isopropylstearate |
| | 7,4 | Glyceryl caprylate / Caprate |
| | 23 | Stearyl alkohol |
| | 2 | Sunflower seed wax |
| | 3,5 | Butyrospermum Parkii Butter |
| B | | |
| | 14 | Dioleoylethyl Hydroxyethylmonium Methosulfate/ Sunflower seed glycerides |
| | 7 | Sucrose Stearate |
| | 2 | Sunflower methylglucamid |
| | | |
| | 2 | Lactic acid/ Sodium Lactate |
| | 0,1 | Fragrance |
| | 31 | Secale cereale |

Tabelle 15

| | Shampoo Bar | | Shampoo Bar | | Shampoo Bar |
|---|---|---|---|---|---|
| | **36** | | **37** | | **38** |
| 40 | Sodium Cocoyl Isethionate | 40 | Sodium Cocoyl Isethionate | 40 | Sodium Cocoyl Isethionate |
| 10 | Sodium Lauroyl Methyl Isethionate | 10 | Rhamnolipid oder Sophorolipid | 10 | Sodium Lauroyl Methyl Isethionate |
| | | | Sodium Lauroyl Methyl | | |
| 10 | Sodium Lauroyl Methyl Taurate | 10 | Taurate | 10 | Sophorolipid |
| 30 | Cetearyl Alkohol | 30 | Cetearyl Alkohol | 30 | Cetearyl Alkohol |
| 5 | Rhamnolipid | 5 | Sunfloweroyl Methylglucamide | 5 | Sunfloweroyl Methylglucamide |
| 0,5 | Guar Hydroxypropyltrimonium chloride | 0,5 | Guar Hydroxypropyltrimonium chloride | 0,5 | Guar Hydroxypropyltrimonium chloride |
| 1 | Argan Oil | 1 | Argan Oil | 1 | Argan Oil |
| 1 | Activated Charcoal | 1 | Activated Charcoal | 1 | Activated Charcoal |
| q.s. | Fragrance | q.s. | Fragrance | q.s. | Fragrance |
| | **Shampoo Bar** | | **Body Wash** | | **Body wash** |
| | **39** | | **40** | | **41** |
| ad | | | | | |
| 100 | Glycerin | 5 | Sophorolipid | 5 | Rhamnolipid |
| 25 | Cetearyl alcohol | 0,8 | Sunfloweroyl methylglucamid | 0,8 | Sunfloweroyl methylglucamid |
| 1,5 | Stearic acid | 2 | Cocoyl methylglucamid | 2 | Cocoyl methylglucamid |
| 3 | Hydrolyzed sunflower seed wax | 2 | Sodium Cocoyl isethionate | 2 | Sodium Cocoyl sulfate |
| 2,3 | Hydrogenated castor oil | 2,4 | Coco-betaine | 2,4 | Coco-amidopropylbetain |
| 1 | Glyceryl oleate | q.s. | Citric acid | q.s. | Citric acid |
| 1 | Maltodextrin | q.s. | Konservierungsmittel, Parfum | q.s. | Konservierungsmittel, Parfum |
| 10 | Cocoamidopropylbetain | ad 100 | Wasser | ad 100 | Wasser |
| 5 | Sunfloweroyl Methylglucamide | | pH = 5,5 | | pH = 5,5 |
| 2,8 | Apricot kernel oil | | | | |
| 3 | Olea Europea Fruit Oil | | **Shampoo** | | **Body wash** |
| 25 | Sodium coco Sulfate | | **42** | | **43** |
| 10 | Rhamnolipid oder Sophorolipid | 5 | Sophorolipid | 5 | Rhamnolipid |
| q.s. | Preservative, Parfum | 1,5 | N-C8-10-Acyl-N-methyl cyclic glucamide | 1,5 | N-C8-10-Acyl-N-methyl cyclic glucamide |

(fortgesetzt)

| | Shampoo Bar | | Body Wash | | Body wash |
|---|---|---|---|---|---|
| | 39 | | 40 | | 41 |
| | | 12 | Sodium laureth sulfate | 2,5 | Decylglucosid |
| | | 3 | Cocoamidopropylbetain | 6 | Disodium Cocoamphodiproprionate |
| | | q.s. | Citric acid | q.s. | Citric acid |
| | | q.s. | Konservierungsmittel, Parfum | q.s. | Konservierungsmittel, Parfum |
| | | ad 100 | Wasser | ad 100 | Wasser |
| | | | pH = 5,5 | | pH = 5,5 |

Tabelle 16

| | O/W Sonnenschutzlotion SPF 30 | | | W/O Lotion | | | Deo (Stick) |
|---|---|---|---|---|---|---|---|
| | 44 | | | 45 | | | 46 |
| 2 | Rhamnolipid | | 0,5 | MEL | | 3,2 | Propandiol-1,2, |
| 1 | Sunfloweroyl methylglucamid | | 0,3 | Sunfloweroyl methylglucamid | | 1,8 | Steareth-21 |
| 7 | Phenoxylethylcaprylate | | 2,5 | Polyglyceryl-4-Diisostearate/ Polyhydroxystearate/ Sebacate | | 2,6 | Steareth-21 |
| 1 | Cetearyl alkohol | | 10 | Isopropyl palmitate | | 2,9 | PPG-15 Stearyl Ether |
| 4,8 | Octocrylene | | 8 | Sweet Almond oil | | 0,3 | Rhamnolipid |
| 4,5 | Ethylhexyltriazone | | 7 | Diethylhexyl carbonate | | 0,9 | Phenoxylethanol |
| 2,5 | Butyl methoxydibenzoylmethane | | 3 | Glycerin | | 0,18 | C12-C16 Alkyl dimethyl benzyl ammonium chloride |
| 5,5 | Bis-Ethylhexyloxylphenol Methoxyphenyl triazine | | 1,5 | Magnesium sulfate heptahydrate | | 0,1 | GLDA |
| 0,5 | Tocopheryl acetate | | | | | 0,1 | Citric acid monohydrate |
| 2 | Glycerin | | | | | | |
| 0,1 | Acrylates/C10-30 Alkly Acrylate Crosspolymer | | | | | | |
| 0,3 | Sodium hydroxide | | | | | | |
| q.s. | Konservierungsmittel | | q.s. | Konservierungsmittel | | | |
| q.s. | optional: Parfum | | q.s. | optional: Parfum | | | |
| ad 100 | Wasser | | ad 100 | Wasser | | ad 100 | Wasser |

(fortgesetzt)

| | Deo (Stick) | | | Deo (Stick) | | | Deo |
|---|---|---|---|---|---|---|---|
| | **47** | | | **48** | | | **49** |
| 3,2 | Propandiol-1,2, | | 3,2 | Propandiol-1,2, | | 10 | Propandiol |
| 1,8 | Steareth-21 | | 1,8 | Steareth-21 | | 5 | Coco Caprylate |
| 2,6 | Steareth-21 | | 2,6 | Steareth-21 | | 0,5 | Mannosylerythritollipid |
| 2,9 | Mannosylerythritollipid | | 2,9 | PPG-15 Stearyl Ether | | 1,5 | N-C8-10-Acyl-N-methyl cyclic glucamide |
| 1 | N-C8-10-Acyl-N-methyl cyclic glucamide | | 1 | Sophorolipid | | 8 | Stearic acid |
| 0,9 | Phenoxylethanol | | 0,9 | Phenoxylethanol | | 2,4 | Sodium Hydroxid (50%) |
| 0,18 | C12-C16 Alkyl dimethyl benzyl ammonium chloride | | 0,18 | C12-C16 Alkyl dimethyl benzyl ammonium chloride | | | |
| 0,1 | GLDA | | 0,1 | GLDA | | | |
| 0,1 | Citric acid monohydrate | | 0,1 | Citric acid monohydrate | | q.s. | optional: Parfum |
| ad 100 | Wasser | | ad 100 | Wasser | | ad 100 | Ethanol |

| | Deo |
|---|---|
| | **50** |
| 10 | Propandiol |
| 4 | Coco Caprylate |
| 2 | Sophorolipid |
| 1 | Sunfloweroyl methylglucamid |
| 8 | Stearic acid |
| 2,4 | Sodium Hydroxid (50%) |
| q.s. | optional: Parfum |
| ad 100 | Ethanol |

Tabelle 17

| | Zahncreme | | | Zahncreme (Enzym) | | | Zahncreme (Enzym) |
|---|---|---|---|---|---|---|---|
| | **52** | | | **53** | | | **54** |
| 0,5 | Sunfloweroyl methylglucamid | | 0,5 | Sunfloweroyl methylglucamid | | 0,5 | Sunfloweroyl methylglucamid |
| 1 | Sophorolipid | | 1 | Rhamnolipid | | 1 | Mannosylerithritollipid |
| 14 | Kieselsäure, abrasiv | | 17 | Hydrated silica | | 22 | Hydrated silica |
| 6 | Kieselsäure, verdickend | | 1 | Xanthan gum | | 0,8 | Cellulose gum |
| 0,3 | Xanthan gum | | 40 | Sorbitol (70%) | | 40 | Sorbitol (70%) |
| 35 | Sorbitol (70%) | | 26 | Glycerin | | 26 | Glycerin |
| 13 | Glycerin | | 2 | Propylenglykol | | 2 | Propylenglykol |
| 2 | Propylenglykol | | 0,2 | Natrium benzoat | | 0,2 | Natrium benzoat |
| 0,8 | Aromaöl | | 0,8 | Aromaöl | | 0,8 | Aromaöl |
| 0,1 | Glycyrrhizin | | 0,1 | Stevia | | 0,1 | Stevia |
| 0,3 | Natriumfluorid | | 0,3 | Natriummonofluorphosphat | | 0,3 | Natriummonofluorphosphat |
| 1,2 | optional Dinatriumpyrophosphat | | 0,5 | Zinc gluconate | | 0,5 | Zinc sulfat |
| 4,4 | optional Tetrakaliumpyrophosphat | | 0,1 | Lactoperoxidase | | q.s. | opt. pH- Einstellung, Farbstoff, Konservierung |
| q.s. | opt. pH- Einstellung, Farbstoff, Konservierung | | q.s. | opt. pH- Einstellung, Farbstoff, Konservierung | | | |
| ad 100 | Wasser | | ad 100 | Wasser | | ad 100 | Wasser |

(fortgesetzt)

| | Zahncreme (Mica) | | | Mundwasser | | | Mundwasser (alkoholfrei) |
|---|---|---|---|---|---|---|---|
| | **55** | | | **56** | | | **57** |
| 0,5 | N--Acyl-N-methyl cyclic glucamide | | 55 | Ethanol 96% oder Propylenglykol | | 7 | Propylenglykol |
| 1 | Sophorlipid | | 0,5 | Sunfloweroyl methylglucamid | | 11 | Glycerin |
| 0,35 | Cocoamidopropylbetain | | 1 | Sophorolipid | | | |
| 22 | Hydrated silica | | 3 | Pfefferminzöl und / oder Nelkenöl | | 0,5 | N--Acyl-N-methyl cyclic glucamide |
| 0,8 | Cellulose gum | | 2 | Menthol | | 1 | Rhamnolipid |
| 40 | Sorbitol (70%) | | 0,4 | Natriumsaccharinat | | 0,9 | Aroma |
| 26 | Glycerin | | 0,2 | Kamillenextrakt | | 0,1 | Menthol |
| 0,5 | Propylenglykol | | 0,02 | Sodiumfluorid | | 0,4 | Natriumsaccharinat |
| 0,2 | Natrium benzoat | | 0,2 | Salbeiextrakt | | 0,5 | Zinc PCA |
| 0,8 | Aromaöl | | | opt. pH- Einstellung, Farbstoff, Konservierung | | 0,05 | Zinc hydroxyapatite |
| 0,1 | Xylitol | | ad 100 | Wasser | | | opt. pH- Einstellung, Farbstoff, Konservierung |
| 0,07 | Olaflur | | | | | ad 100 | Wasser |
| 0,5 | Calciumglycerophosphat | | | | | | |
| 0,1 | Natrium fluorid | | | | | | |
| 0,5 | Mica | | | | | | |
| q.s. | opt. pH- Einstellung, Farbstoff, Konservierung | | | | | | |
| ad 100 | Wasser | | | | | | |

Tabelle 18

| | Maschinengeschirrspülmittel (fest) | | | Maschinengeschirrspülmittel (fest) | | | Klarspüler |
|---|---|---|---|---|---|---|---|
| | **58** | | | **59** | | | **60** |
| 30 | Trisodium citrate dihydrate | | 30 | Trisodium citrate dihydrate | | 0,5 | Sunfloweroyl methylglucamid |
| 20 | Sodium carbonat | | 20 | Sodium carbonat | | 3 | MEL |
| 15 | GLDA oder MGDA-Na3 | | 15 | GLDA oder MGDA-Na3 | | 0,5 | Sodium Octyl sulfate |
| 10 | Polycarboxylate | | 10 | Polycarboxylate | | 0,3 | Coco-glucosid |
| 15 | Sodium percarbonate | | 15 | Sodium percarbonate | | 5 | Ethanol |
| 2 | TAED | | 2 | TAED | | q.s. | Citric acid |
| 2 | Sodium silicate | | 2 | Sodium silicate | | ad 100 | Wasser |
| 1,5 | Sunfloweroyl methylglucamid | | 1,5 | N-C8-10-Acyl-N-methyl cyclic glucamide | | | |
| 2 | Rhamnolipid | | 2 | Sophorolipid | | | pH = 4 |
| 0,5 | PROPYLENE GLYCOL | | 0,5 | PROPYLENE GLYCOL | | | |
| 1 | Sodium Sulfate | | 1 | Sodium Sulfate | | | |
| 1 | Enzyme, z.B. Protease, Amylase | | 1 | Enzyme, z.B. Protease, Amylase | | | |
| | optional: BISMUTH CITRATE, HEDP, Glycerin | | | optional: BISMUTH CITRATE, HEDP, Glycerin | | | |
| | **Maschinengeschirrspülmittel** | | | **Maschinengeschirrspülmittel** | | | **Klarspüler** |
| | **61** | | | **62** | | | **63** |
| 0,7 | Sunfloweroyl methylglucamid | | 0,7 | N-Acyl-N-methyl cyclic glucamide | | 0,5 | Glucamid |
| 2,6 | Sophorolipid | | 2,6 | MEL | | | |
| 6,0 | Trisodium citrate dihydrate | | 6,0 | Trisodium citrate dihydrate | | 5 | Ethanol |
| 12,0 | GLDA | | 12,0 | GLDA | | q.s. | Citric acid |
| 3,0 | Glycerin | | 3,0 | Glycerin | | ad 100 | Wasser |
| 1,0 | Propylenglycol | | 1,0 | Propylenglycol | | | |
| 0,5 | Xanthan gum | | 0,5 | Xanthan gum | | | |
| | optional: pH-Stellmittel, z.B. Milchsäure; Enzyme | | | optional: pH-Stellmittel, z.B. Milchsäure; Enzyme | | | |

(fortgesetzt)

| | | | | | | | |
|---|---|---|---|---|---|---|---|
| | **Maschinengeschirrspülmittel** | | | **Maschinengeschirrspülmittel** | | | **Klarspüler** |
| | **61** | | | **62** | | | **63** |
| ad 100 | Wasser | | ad 100 | Wasser | | | |

Tabelle 19

| | 64 | 65 | 66 | 67 | | |
|---|---|---|---|---|---|---|
| Biotensid | 0,2 -20 | 5-20 | 5-20 | 5-20 | | |
| N-Acylglycamid, bev. Sunfloweroyl Methylglucamide | 0,2-10 | 1-10 | 0,5-10 | 1-10 | | |
| Sodium Alkylsulfat oder Sodium Alkylethersulfat oder Lineares Alkylsulfat, bev. C8-C18 | 0-30 | | | 3-15 | | |
| Weitere Nichtionische Tenside | 0-20 | | 0,5-15 | 8-15 | | |
| Seife (aus C12-C22-Fettsäuren) | 0-15 | 0-15 | 0-7 | 1-5 | | |
| Builder, z.B. Natriumcitrat | 1-5 | 1-5 | 1-5 | 1-5 | | |
| Polycarboxylate | 0-5 | 0-5 | 0-5 | 0-5 | | |
| Komplexbildner, z.B. Phosphonate, bevorzugt GLDA, MGDA | 0-2 | 0-2 | 0-2 | 0-2 | | |
| Enzyme, z.B. Proteasen, Amylasen, Lipasen, Cellulasen, Mannasen | 0-1 | 0-1 | 0-1 | 0-1 | | |
| Farbübertragungsinhibitoren, z.B. PVP, PVPNO | 0-2 | 0-2 | 0-2 | 0-2 | | |
| Optische Aufheller, z.B. Stilbene | 0-1 | 0-1 | 0-1 | 0-1 | | |
| Vergrauungsinhibitor, z.B. Cellulosemethylcellulose | 0-1 | 0-1 | 0-1 | 0-1 | | |
| Soil Release Polymere | 0-2 | 0-2 | 0-2 | 0-2 | | |
| Paraffinöl, Silikonöl, oder Pflanzenöl | 0-1 | 0-1 | 0-1 | 0-1 | | |
| Duftstoffe, z.B. ätherische Öle, Pflanzenextrakte | 0-1 | 0-1 | 0-1 | 0-1 | | |
| Lösungsmittel, z.B. Alkohole, Glycole, Glycerin | 0-15 | 0-15 | 1-10 | 1-10 | | |
| optional pH- Einstellung (z.B. Milchsäure) | q.s. | q.s. | q.s. | q.s. | | |
| optional: Konservierungsmittel, Verdicker, Farbstoffe | q.s. | q.s. | q.s. | q.s. | | |
| Wasser | ad 100 | ad 100 | ad 100 | ad 100 | | |
| | | | | | | |
| | **68** | **69** | **70** | **71** | **72** | **73** |
| Biotensid, bev. Mannosylerythritollipid, Rhamnolipid, Sophorolipid | 9 | 6,5 | 7 | 3 | 3 | 3 |
| N-Acylglycamid, bev. Sunfloweroyl Methylglucamide, N-Acyl N-methyl cyclic glucamid | 2,9 | 2,9 | 1,5 | 1 | 1,7 | |
| Sodium Octyl sulfate | | | 2 | | | |
| Sodium Laureth sulfate | | | | | | 6,5 |
| APG | | | | 2,5 | | |
| C-18-Pflanzenölfettsäuren | | 3 | | 2 | 5 | |
| C12-C18-Fettsäuren (z.B. Kokos) | | | 2 | 3 | | 3 |
| Rüböl Monoethanolamid, ethoxyliert | | | | | 1 | |
| C12-18 Fettalkohol mit 7 EO | | | | | | 3 |
| Zitronensäure | 1,5 | 1,5 | 1,5 | 1,5 | 1,5 | 1,5 |
| Ethanol | 3 | 3 | 3 | 3 | 3 | 3 |
| Propylenglycol | 5 | 5 | 5 | 5 | 0,5 | 5 |
| KOH, alternativ NaOH, Ammoniak oder organische Amine | 5 | 5 | 5 | 5 | 5 | 5 |
| optional pH- Einstellung (z.B. Milchsäure) | q.s. | q.s. | q.s. | q.s. | q.s. | q.s. |

(fortgesetzt)

| | 68 | 69 | 70 | 71 | 72 | 73 |
|---|---|---|---|---|---|---|
| optional: Duftstoffe, Konservierungsmittel (z.B. Phenoxyethanol), Hilfsstoffe, e.g. Enzyme, Komplexbildner, Farbübertragungsinhibitoren, Aufheller, Vergrauungsinhibitoren, Verdicker, etc.; | q.s. | q.s. | q.s. | q.s. | q.s. | q.s. |
| Wasser | ad 100 | ad 100 | ad 100 | ad 100 | ad 100 | ad 100 |

Tabelle 20

| | Waschmittel inkl. Tabs | | | | | | |
|---|---|---|---|---|---|---|---|
| | 74 | 75 | 76 | 77 | 78 | 79 | 80 |
| Zeolite | 15-30 | | 15-30 | 15-20 | 15-20 | 15-30 | 15-30 |
| Natrium Carbonat | 5-15 | | 20-25 | 20-25 | 20-25 | 1-5 | 1-10 |
| Natrium Bicarbonat | | 15-30 | 5 | 5 | 5 | | |
| Natrium Percarbonat | | | | 10-15 | | | |
| Natrium Citrat | | | | | | 3-10 | 3-10 |
| Polycarboxylate | | | | 1-5 | 1-5 | 1-5 | 1-5 |
| Biotensid | 10-15 | 10-15 | 10-15 | 10-15 | 10-15 | 5-20 | 5-10 |
| Sunfloweroyl Methylglucamide | 1-5 | 1-5 | 1-5 | 1-5 | | 1-5 | 1-5 |
| N-Acyl-N-methyl glucamid | | | | | 1-5 | | |
| Nichtionisches Tensid, z.B. Fettalkoholethoxylat | | | | | | | 1-10 |
| Natrium laureth sulfat oder lineares Alkylbenzolsulfat | | | | | | | 5-15 |
| Seife, z.B. Natrium Rapsölseife, Natrium Palmitate | 5-10 | 5-15 | 1-2 | 0-2 | 1-2 | 0-2 | 0-2 |
| Ethoxylierte Fettalkohole z.B. Laureth-9, Laureth-3 | | | 1-3 | 1-3 | 1-3 | | |
| Dinatrium Disilicate | 5-15 | 5-15 | 1-5 | 1-5 | 1-5 | 0-5 | 0-5 |
| Tetra acetyl ethylen diamin | | 4-8 | | 1-5 | | | |
| Enzyme: Protease, Lipase, Amylase, Mannase, Cellulase | | | | q.s. | q.s. | q.s. | q.s. |
| Phosphonate | | | | 0,2-1 | 0,2-1 | 0,2-1 | 0,2-1 |
| Carboxymethylcellulose | | | | 0,5-1 | 0,5-1 | 0,5-2 | 0,5-2 |
| Siliconöl/ Paraffinöl, bevorzugt Pflanzenöl | | | | 0-1 | | | 0,5 |
| optische Aufheller | | | | 0,1-1 | | | |
| Farbtransferinhibitoren (z.B. PVP und PVP-Derivate) | | | | | | | 0,5 |

(fortgesetzt)

| | Waschmittel inkl. Tabs | | | | | | |
|---|---|---|---|---|---|---|---|
| | 74 | 75 | 76 | 77 | 78 | 79 | 80 |
| Optional: Duftstoffe, Farbstoffe, Farbtransferinhibitoren, Enzyme, soil release Polymere, Hilfsstoffe, Bindemittel, Scale inhibitoren, Desinfektionsmittel, Fliessmittel | | | | | | | |
| Ggf. Lösungsmtitel z.B. Wasser, Propylenglykol | Spuren | Spuren | Spuren | Spuren | Spuren | Spuren | Spuren |
| Füllstoffe, z.B. Natriumsulfat | ad 100 | ad 100 | ad 100 | ad 100 | ad 100 | ad 100 | ad 100 |

Tabelle 21

| | Glasreiniger | | | Glasreiniger | | | Glasreiniger |
|---|---|---|---|---|---|---|---|
| | 81 | | | 82 | | | 83 |
| 2 | Biotensid, z.B. Rhamnolipid oder Sophorolipid | | 1 | C8-C14 Alkylpolyglycosid | | 1 | Biotensid, z.B. Rhamnolipid oder Sophorolipid |
| 0,5 | Sunfloweroyl methylglucamid | | 0,5 | Sunfloweroyl methylglucamid | | 0,5 | N-Acyl-N-methylglucamid |
| 5 | Alcohol denat. | | 5 | Alcohol denat. | | 5 | Alcohol denat. |
| | | | 0,1 | PEG 40 hydrogenated castor oil | | 0,1 | PEG 40 hydrogenated castor oil |
| | | | 0,1 | Parfum | | 0,1 | Parfum |
| q.s. | optional Parfum, Solubilizer, Konservierungsmittel, pH-Stellmittel, Komplexierungsmittel z.B. GLDA | | q.s. | Konservierungsmittel | | q.s. | Konservierungsmittel |
| ad 100 | Aqua | | ad 100 | Aqua | | ad 100 | Aqua |

(fortgesetzt)

| | saurer Reiniger | | | saurer Reiniger | | | alkal. Reiniger |
|---|---|---|---|---|---|---|---|
| | 84 | | | 85 | | | 86 |
| 3 | Biotensid, z.B: Rhamnolipid oder Sophorolipid | | 3 | Biotensid, z.B: Rhamnolipid oder Sophorolipid | | 2 | Olive oil fatty acids, triethanolamine salt |
| 1 | Sunfloweroyl methylglucamid | | 1 | N-Acyl-N-methylglucamid | | 1,5 | Biotensid, bevorzugt Sophorolipid oder Rhamnolipid |
| 3 | Citric acid | | 3 | Citric acid | | 0,5 | Sunfloweroyl methylglucamid |
| 2 | Sodium citrat | | 2 | Sodium citrat | | 0,5 | Propylenglykol |
| | | | | | | 0,1 | GLDA oder MGDA |
| | | | | | | | opt. pH Stellmittel |
| q.s. | opt. Verdicker, Parfum, Farbstoff | | q.s. | opt. Verdicker, Parfum, Farbstoff | | q.s. | opt. Duftstoffe, Konservierungsmittel, Farbstoffe, Pflanzenextrakte, |
| ad 100 | Aqua | | ad 100 | Aqua | | ad 100 | Aqua |
| | pH = 3-4 | | | pH = 3-4 | | | pH 9-10 |
| | | | | | | | |

(fortgesetzt)

| | **neutraler Reiniger** | | **Reiniger mit Lösungsmitel** | | **Pflege mit Wachs** |
|---|---|---|---|---|---|
| | **87** | | **88** | | **89** |
| 1,9 | Sodium Laureth sulfate | 10 | Phenoxyethanol | 20 | Olive oil fatty acids, potassium salt |
| 1 | Biotensid, bevorzugt Sophorolipid oder Rhamnolipid | 5 | 2-Butoxyethanol | 5 | Cera alba |
| 0,3 | Sunfloweroyl methylglucamid | 5 | Biotensid, bevorzugt Sophorolipid oder Rhamnolipid | 5 | Alcohol denat. |
| 0,5 | Sodium chloride | 3 | Sunfloweroyl methylglucamid | 5 | Biotensid, bevorzugt Sophorolipid oder Rhamnolipid |
| 0,5 | Coco amidopropylbetain | 1 | GLDA oder MGDA | 2 | Sunfloweroyl methylglucamid |
| 0,2 | Glycol distearate | | | 5 | Coco glucoside |
| 0,3 | Propylenglykol | | | 1 | Carnauba wax |
| q.s. | opt. Duftstoffe, Konservierungsmittel, Farbstoffe, Pflanzenextrakte, pH-Stellmittel | | opt. pH- Stellmittel | q.s. | opt. Duftstoffe, Konservierungsmittel, Farbstoffe, Pflanzenextrakte |
| ad 100 | Aqua | ad 100 | Aqua | ad 100 | Aqua |
| | pH= 6,5-8,0 | | pH = 9-10 | | pH = 7-8 |

**Patentansprüche**

1. Zusammensetzung enthaltend mindestens ein N-Acylglycamin der Formel (I)

(I)

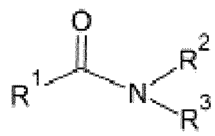

wobei $R^1CO$ einen linearen oder verzweigten, gesättigten oder ungesättigten, gegebenenfalls hydroxysubstituierten oder - $OR^s$ substituierten mit $R^S$= -SOsH oder ein Salz desselben, Acylrest mit 6 bis 24 Kohlenstoffatomen darstellt, bevorzugt einen Fettsäureacylrest, und $R^2$ einen linearen, verzweigten oder heterozyklischen Polyhydroxyalkylrest, bevorzugt ist $R^2 = -CH_2-(CHOH)_n-CH_2OH$ mit n = 3 oder bevorzugter n = 4, oder deren heterozyklischen Anhydride, besonders bevorzugt stammt $R^2$ von Glucose, und $R^3$ stellt ein Wasserstoffatom, einen Hydroxyalkylrest mit 1 bis 4 Kohlenstoffatomen oder bevorzugter einen Alkylrest ausgewählt aus $-(CH_2)_mCH_3$ mit m=0,1, 2 oder 3, $-(CH_2)_oCH(CH_3)_2-$ mit o = 0 oder 1, oder $-C(CH_3)_3-$ dar, besonders bevorzugt ist $R^3 = -CH_3$, wobei die Varianten der unterschiedlichen Reste $R^1CO$, $R^2$ und $R^3$ beliebig und unabhängig voneinander kombiniert werden können.

2. Zusammensetzung gemäss Anspruch 1 **dadurch gekennzeichnet, dass** zusätzlich mindestens ein Glycolipid-Biotensid in der Zusammensetzung enthalten ist umfassend die Gruppen der Rhamnolipide Sophorolipide, Mannosylerythritollipide, Cellobioselipide und Trehaloselipide, sowie deren Kombinationen.

3. Zusammensetzung gemäss einem der vorstehenden Ansprüche **dadurch gekennzeichnet, dass** mindestens ein

Gemisch von zwei, bevorzugt mehr als zwei, der N-Acylglycaminen der Formel (I) enthalten ist, die sich durch Kettenlänge oder Sättigungsgrad der Fettsäureacylreste $R^1CO$ unterscheiden und wobei das Gemisch aus der Umsetzung der entsprechenden Fettsäurederivate eines Pflanzenöls oder der Umsetzung eines Pflanzenöls stammt, bevorzugt stammt das Gemisch von N-Acylglycaminen der Formel (I) von mindestens einem C-18-Pflanzenöl oder den Fettsäurederivaten eines C-18-Pflanzenöls mit einer Verteilung von Fettsäureacylresten mit 18 und mehr Kohlenstoffatomen über 60 Gew.-%, besonders bevorzugt über 72 Gew.-% und ganz besonders bevorzugt über 77 Gew.-% und einem Anteil an ungesättigten Fettsäureacylresten über 55 Gew.-%, vorzugsweise über 65 Gew.-% und besonders bevorzugt über 72 Gew.-%, Gew.-% jeweils bezogen auf die Fettsäureacylreste der entsprechenden Fettsäurederivate oder des C-18-Pflanzenöls .

4. Zusammensetzung gemäss einem der vorstehenden Ansprüche **dadurch gekennzeichnet, dass** der Anteil an ein- oder mehrfach ungesättigten Fettsäureacylresten der N-Acylglycamine der Formel (I) mit mindestens 18 Kohlenstoffatomen > 8 Gew.-%, bevorzugt über 20 Gew.-%, bevorzugter über 60 Gew.-% und äusserst bevorzugt über 72 Gew.-%, jeweils bezogen auf das Gesamtgewicht der Fettsäureacylreste $R^1CO$ der N-Acylglycamine der Formel (I) in der Zusammensetzung.

5. Zusammensetzung gemäss einem der vorstehenden Ansprüche, wobei das mindestens eine N-Acylglycamin der Formel (I) ein N-Alkyl-N-Acylglycamin mit $R^2$ einem linearen Polyhydroxylalkylrest ist, bevorzugt ein N-Alkyl-N-Acylglucamin; besonders bevorzugt ein N-Methyl-N-Acylglucamin der Formel (Ia) mit RaCO = $R^1CO$;

   bevorzugt ist das Gemisch an N-Methyl-N-Acylglucaminen der Formel (Ia) mit unterschiedlichen Fettsäureacylresten RaCO aus mindestens einem C-18-Pflanzenöl in der Zusammensetzung enthalten, wobei die Verteilung der Fettsäureacylresten RaCO wie in Anspruch 3 definiert vorliegt und der Anteil an gesättigten Fettsäureacylresten mit 16 oder 18 Kohlenstoffatomen < 40 Gew.-%, bevorzugter < 25 Gew.-%, besonders bevorzugt ≤ 15 Gew.-%, und ganz besonders bevorzugt zwischen 6 und 15 Gew.-% liegt; Gew.-% bezogen auf die Fettsäureacylreste RaCO des Gemischs an N-Methyl-N-Acylglucaminen der Formel (Ia);
   äussert bevorzugt ist das Gemisch an N-Methyl-N-Acylglucamine mit den Fettsäureacylresten RaCO aus Sonnenblumenöl, Rapsöl, oder Olivenöl in der Zusammensetzung enthalten, insbesondere das Gemisch an N-Methyl-N-Acylglucaminen aus Sonnenblumenöl mit der Bezeichnung Sunfloweroyl methylglucamid.

(Ia)

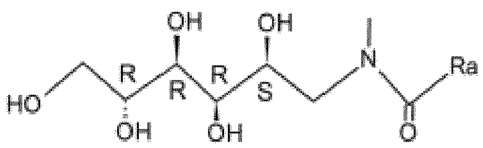

6. Zusammensetzung gemäss Anspruch 1 oder 2, **dadurch gekennzeichnet, dass** der Rest $R^2$ des N-Acylglycamins der Formel (I) das heterocyclisches Anhydrid eines Hexanpentolrests ist, bevorzugt ist das N-Acylglycamin der Formel (I) N-Acyl-N-methyl cyclic glucamide, besonders bevorzugt ist $R^1$ in dieser cyclischen Ausführungsform $-(CH_2)_8CH_3$ oder $-(CH_2)_6CH_3$ oder eine Mischung derselben .

7. Zusammensetzung gemäss einem der vorstehenden Ansprüche, **dadurch gekennzeichnet, dass** das mindestens eine Glycolipid-Biotensid einen Gesamtgehalt von mindestens 0,3 Gew.-%, bevorzugt grösser als 0,3 Gew.-%, besonders bevorzugt > 1 Gew.-%, am bevorzugtesten ≥ 5 Gew.-%, Gew.-% bezogen auf das gesamte Mittel.

8. Zusammensetzung gemäss einem der vorstehenden Ansprüche, **dadurch gekennzeichnet, dass** es in einer fliessförmigen Ausführungsform vorliegt, wobei der Gehalt an anionischen Tensiden unter 30 Gew.-% liegt, und bevorzugt der Gehalt des N-Acylglycamins unter 10 Gew.-% liegt, Gew.-% bezogen auf die gesamte Zusammensetzung.

9. Zusammensetzung gemäss einem der vorstehenden Ansprüche, **dadurch gekennzeichnet, dass** das mindestens eine Glycolipid-Biotensid in einem Mengenverhältnis zu dem N-Acylglycamin von ≥ 1:1, bevorzugt von > 1:1, bevorzugter > 5: 2,9 und besonders bevorzugt > 2: 1 vorliegt.

10. Zusammensetzung gemäss einem der vorstehend Ansprüche, **dadurch gekennzeichnet, dass** weitere nichtionische Tenside mit lipophilen Teilen aus petrochemischem, tierischem oder oleochemischem Ursprung in der Zusammensetzung enthalten sind mit der Vorgabe, dass das Gewichtsverhältnis der N-Acylglycamine zu den optional enthaltenen nichtionischen PEG-Tensiden abgeleitet von C-18-Pflanzenölen > 1 : 2, bevorzugt ≥ 1 : 1 beträgt.

**11.** Zusammensetzung gemäss einem der vorstehend Ansprüche, **dadurch gekennzeichnet, dass** weitere anionische Tenside mit lipophilen Teilen aus petrochemischem, tierischem oder oleochemischem Ursprung in der Zusammensetzung enthalten sind mit der Vorgabe, dass das Gewichtsverhältnis der N-Acylglycamine zu den optional enthaltenen anionischen PEG-Tensiden abgeleitet von C-18-Pflanzenölen > 1 : 3,4, besonders bevorzugt ≥ 1 : 2 beträgt.

**12.** Zusammensetzung gemäss einem der vorstehend Ansprüche, **dadurch gekennzeichnet, dass** die weiteren anionische Tenside ausgewählt sind aus den Gruppen der Seifen, Alkylsulfate, Alkylethersulfaten, Alkenylsulfate, Alkenylethersulfate, Sulfonate, Fettalkoholcarboxylate, Alkylethercarboxylate, Alkenylethercarboxylate, Amidethercarboxylate, anionische N-Acylamide, N-Acylaminosäuretenside, acylierte Polypeptide, Alkylisethionate, Alkenylisethionate Acylisethionate, Acyl methyl isethionate, Acyltaurine, Acyl Methyl taurate, Acyl sulfate, Sulfatierte Amide, sulfatierte Fettsäuren oder sulfatierte Öle, Carbonsäureamidethersulfate, Acyllactylate und Lactyl Lactate, anionische Glycolipide derivatisiert mit anionischen Gruppen, Sulfosuccinate, Sulfoacetate; Alkyletherphosphate, Alkylenetherphosphate, Phosphor- und Polyphosphorsäureester, Alkyl- und Alkenyltartrate.

**13.** Zusammensetzung gemäss einem der vorstehend Ansprüche, **dadurch gekennzeichnet, dass** das mindestens eine N-Acylglycamin ausgewählt ist aus der Gruppe der Sunfloweroyl methylglucamide und N-Acyl-N-methyl cyclic glucamide und das mindestens eine Glykolipid-Biotensid ausgewählt ist aus der Gruppe der Sophorolipide, Rhamnolipide und Mannosylerytrhitollipide, sowie jeweils deren Kombinationen; bevorzugt enthält die Zusammensetzung mindestens eine der folgenden Tensidkombinationen: mindestens ein N-Acyl-N-methyl cyclic glucamid und mindestens ein Sophorolipid, mindestens ein N-Acyl-N-methyl cyclic glucamid und mindestens ein Rhamnolipid, mindestens ein N-Acyl-N-methyl cyclic glucamid und mindestens ein Mannosylerytrhitollipid, Sunfloweroyl methylglucamid und mindestens ein Sophorolipid, Sunfloweroyl methylglucamid und mindestens ein Rhamnolipid, Sunfloweroyl methylglucamid und mindestens ein Mannosylerytrhitollipid, Sunfloweroyl methylglucamide und N-Acyl-N-methyl cyclic glucamid sowie jeweils deren Kombinationen; besonders bevorzugt die Kombinationen Sunfloweroyl methylglucamid und mindestens ein Sophorolipid, Sunfloweroyl methylglucamid und mindestens ein Rhamnolipid, Sunfloweroyl methylglucamid und mindestens ein Mannosylerythritollipid, sowie deren Kombinationen.

Europäisches Patentamt
European Patent Office
Office européen des brevets

# EUROPÄISCHER RECHERCHENBERICHT

Nummer der Anmeldung
EP 23 02 0436

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | CN 114 621 823 A (NANJING ARMY METAMATERIAL TECH CO LTD) 14. Juni 2022 (2022-06-14) * Beispiel 2 * ----- | 1,2,5, 7-10,13 | INV. C11D1/66 A61K8/60 |
| X | EP 3 744 819 A1 (FAMA HOLDING AG [CH]) 2. Dezember 2020 (2020-12-02) * Tabelle 13 * * Beispiel E55 * ----- | 1,3-5,8, 11,12 | |
| X | WO 2013/178697 A2 (CLARIANT INT LTD [CH]) 5. Dezember 2013 (2013-12-05) * Beispiel 3 * * Herstellbeispiel 6 * ----- | 1,3-5,8, 11,12 | |
| X | WO 2018/002100 A1 (CLARIANT INT LTD [CH]) 4. Januar 2018 (2018-01-04) * Tabellen 1, 3 * * Beispiele 6, 26 * ----- | 1,3,6,8, 10-12 | |
| X | WO 2013/178700 A2 (CLARIANT INT LTD [CH]) 5. Dezember 2013 (2013-12-05) * Tabellen 1, 2 * * Beispiel 2 * * Herstellbeispiel H2 * ----- | 1,3-5,8, 11,12 | RECHERCHIERTE SACHGEBIETE (IPC) C11D A61Q A61K |

Der vorliegende Recherchenbericht wurde für alle Patentansprüche erstellt

1

| Recherchenort | Abschlußdatum der Recherche | Prüfer |
|---|---|---|
| Den Haag | 19. Februar 2024 | Placke, Daniel |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 23 02 0436

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-02-2024

| Im Recherchenbericht angeführtes Patentdokument | Datum der Veröffentlichung | Mitglied(er) der Patentfamilie | Datum der Veröffentlichung |
|---|---|---|---|
| CN 114621823 A | 14-06-2022 | KEINE | |
| EP 3744819 A1 | 02-12-2020 | CH 716229 A2 | 30-11-2020 |
| | | EP 3744819 A1 | 02-12-2020 |
| WO 2013178697 A2 | 05-12-2013 | BR 112014029774 A2 | 27-06-2017 |
| | | CN 104582678 A | 29-04-2015 |
| | | EP 2858622 A2 | 15-04-2015 |
| | | ES 2603387 T3 | 27-02-2017 |
| | | JP 6442400 B2 | 19-12-2018 |
| | | JP 2015518028 A | 25-06-2015 |
| | | US 2015126616 A1 | 07-05-2015 |
| | | US 2019076344 A1 | 14-03-2019 |
| | | WO 2013178697 A2 | 05-12-2013 |
| WO 2018002100 A1 | 04-01-2018 | BR 112018077158 A2 | 02-04-2019 |
| | | CN 109640659 A | 16-04-2019 |
| | | EP 3478067 A1 | 08-05-2019 |
| | | EP 3735827 A1 | 11-11-2020 |
| | | ES 2814379 T3 | 26-03-2021 |
| | | JP 6826134 B2 | 03-02-2021 |
| | | JP 2019519567 A | 11-07-2019 |
| | | US 2019224099 A1 | 25-07-2019 |
| | | US 2020261342 A1 | 20-08-2020 |
| | | WO 2018002100 A1 | 04-01-2018 |
| WO 2013178700 A2 | 05-12-2013 | BR 112014029762 A2 | 27-06-2017 |
| | | CN 104520417 A | 15-04-2015 |
| | | EP 2855649 A2 | 08-04-2015 |
| | | JP 2015523341 A | 13-08-2015 |
| | | US 2015133560 A1 | 14-05-2015 |
| | | WO 2013178700 A2 | 05-12-2013 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2014206555 A
- US 20160136072 A
- WO 2013178697 A
- WO 2018002100 A
- EP 0499434 A1
- EP 1445302 A1
- WO 2016050439 A
- EP 3071546 A1
- EP 2735605 A, Evonik
- US 20130130319 A, Evonik
- US 20160272667 A, Logos
- US 20190309248 A, Logos
- EP 0499434 A
- US 7985722 B
- WO 03006146 A
- JP 60183032 A
- DE 19648439
- DE 19600743
- JP 1304034 A
- CN 1337439
- JP 2006 A
- JP 274233 A
- KR 2004033376
- JP 2006083238 A
- JP 2006070231 A
- WO 03002700 A
- FR 2740779
- DE 2939519
- US 7556654 B
- FR 2855752
- EP 1445302 A
- JP 2008062179 A
- JP 2007181789 A
- WO 2004020647 A
- JP 20042544595 B
- WO 2020006194 A
- WO 2021127339 A
- WO 2017220957 A
- EP 0282942 A
- KR 20100022289
- ES 2018637
- CN 1431312
- CN 102250790
- US 2008032383 A
- DE 4319540
- FR 2692593
- JP 2004254595 B
- JP 2007252279 B
- CN 101845468
- CN 101948786
- EP 19835193 A


### In der Beschreibung aufgeführte Nicht-Patentliteratur


- **C. MULLIGAN.** Biosurfactants: Research Trends and Application. CRC Press, 2014, 112
- **NITSCH, CH. ; HÜTTMANN, G.** Empfehlungen zur Qualitätsbewertung der Reinigungsleistung von Handgeschirrspülmitteln. *SÖFW Journal,* 2002, vol. 128 (5
- **A. FITZNER ; U. AßMUS.** Empfehlungen zur Qualitätsbewertung der Produktleistung von Allzweckreinigern. *SÖFW Journal,* 2004, vol. 130 (10